# EUROPEAN PATENT SPECIFICATION

(11) **EP 4 477 757 B1**
(45) Date of publication and mention of the grant of the patent: **23.09.2026**
(21) Application number: 24192701.1
(22) Date of filing: 24.05.2017
(51) Int. Cl.: C12Q 1/68

(54) **METHODS FOR VARIANT DETECTION**
VERFAHREN ZUR VARIANTENERKENNUNG
PROCÉDÉS DE DÉTECTION DE VARIANTS

(30) Priority: 13.04.2017 US 201715487401
(43) Date of publication of application: 18.12.2024
(62) Document number(s) of the earlier application(s) in accordance with Art. 76 EPC: 17728370.2 / 3 610 030
(73) Proprietor: Integrated DNA Technologies, Inc., Coralville, IA 52241 (US)
(72) Inventor: DOBOSY, Joseph, Coralville, IA 52241 (US); CHEN, Caifu, Palo Alto, CA 94303 (US); BEHLKE, Mark, Coralville, IA 52241 (US); RETTIG, Garrett Richard, Coralville, IA 52241 (US); OWCZARZY, Richard, Coralville, IA 52241 (US)
(74) Representative: D Young & Co LLP

(56) References cited:
- WO-A1-2013/142364
- WO-A1-2014/110528
- WO-A1-2015/073931
- WO-A2-2012/135053
- CA-A1- 2 949 315
- JOSEPH R DOBOSY ET AL: "RNase H-dependent PCR (rhPCR): improved specificity and single nucleotide polymorphism detection using blocked cleavable primers", BMC BIOTECHNOLOGY, BIOMED CENTRAL LTD. LONDON, GB, vol. 11, no. 1, 10 August 2011 (2011-08-10), pages 80, XP021108976, ISSN: 1472-6750, DOI: 10.1186/1472-6750-11-80
- SAMUEL H. STERNBERG ET AL: "DNA interrogation by the CRISPR RNA-guided endonuclease Cas9", NATURE, vol. 507, no. 7490, 29 January 2014 (2014-01-29), pages 62 - 67, XP055161285, ISSN: 0028-0836, DOI: 10.1038/nature13011

## Description

### FIELD OF THE INVENTION

The invention can be used to provide a more efficient and less error-prone method of detecting variants in DNA, such as single nucleotide polymorphisms (SNPs), multi-nucleotide polymorphisms (MNPs), and indels. The invention also provides a method for performing inexpensive multi-color assays, and provides methods for visualizing multiple allele results in a two-dimensional plot. The invention also provides methods for detection of DNA sequences altered after cleavage by a targetable endonuclease, such as the CRISPR Cas9 protein from the bacterium *Streptococcus pyogenes.*

### BACKGROUND OF THE INVENTION

RNase H2-dependent PCR (rhPCR) (see U.S. Patent Application Publication No. US 2009/0325169 A1) and standard allele-specific PCR (ASPCR) can both be utilized for mutation detection. In ASPCR, the DNA polymerase performs the mismatch discrimination by detection of a mismatch at or near the 3' end of the primer. While ASPCR is sometimes successful in mismatch detection, the discrimination can be limited, due to the low mismatch detection ability of wild-type DNA polymerases.

In contrast with ASPCR, the mismatch sensitivity of the RNase H2 enzyme in rhPCR allows for both sensitive detection of DNA mutations, and elimination of primer-dimer artifacts from the reaction. When attempting to detect DNA mutations with rhPCR, however, placement of the mismatch within the primer is important. The nearer to the cleavable RNA the mismatch is located, the more discrimination is observed from the RNase H2 enzyme, and the greater the discrimination of the resulting rhPCR assay. Given the fact that most common wild-type DNA polymerases such as Taq often display low levels of mismatch detection, the polymerase cannot be solely relied upon to perform this discrimination after RNase H2 cleavage. Coupled with the repeated interrogation desired from every cycle of standard rhPCR, placing the mismatch anywhere other than immediately opposite the RNA is undesirable when utilizing these polymerases.

There is thus a need for assays with improved mismatch sensitivity.

In addition, there is a need for improved methods for detection of mutations altered after cleavage by targetable endonucleases, such as the CRISPR Cas9 protein. A commonly used method to detect mutations introduced into genomic DNA following repair of dsDNA cleavage events is the enzymatic mismatch cleavage assay (EMCA). EMCA assays cleave at sites where base mismatches are present in dsDNA. For EMCA detection of the mutations introduced into DNA following Cas9 cleavage and repair, genomic DNA from cells is harvested and the regions around the dsDNA cut site is amplified by PCR using primers that flank the cut site. Typically 100-1000 base amplicons are used for this purpose. Following completion of amplification, heteroduplexes are formed by heating the reaction products and allowing them to re-anneal, which leads to formation of homoduplex WT/WT, Mut/Mut or heteroduplex WT/Mut or Mut1/Mut2 variants. The dsDNAs are then subjected to cleavage by a mismatch endonuclease (such as T7EI, Surveyor, etc.). Heteroduplexes are cleaved and the presence of shorter fragments is detected by gel electrophoresis, capillary electrophoresis, or any of a number of methods known to those of skill in the art. Although such an assay is fast and inexpensive, it often does not accurately reflect the changes that are actually generated from the CRISPR mutagenesis process. If the same mutation is introduced a large number of times, Mut/Mut homodimers form, which are not detected. Further, the mismatch endonuclease enzymes often fail to cleave single-base events, leading to yet another class of mutations that are undetected. Thus an EMCA assay will almost always underestimate the extent of genome editing that occurred after Cas9 dsDNA cleavage and repair.

An alternative method of analysis involves large scale DNA sequencing using "Next-Gen" sequencing (NGS) methods of the modified DNA, which is highly accurate, but is slow and costly. Other methods are available to assess the mutation outcome following CRISPR/Cas9 cleavage and repair. For example, Sanger sequencing results can be analyzed using sequence trace decomposition ("TIDE" analysis); fluorescent-labeled primer-extension on an amplicon spanning the Cas9 cut site can be used to map indels using Indel Detection by Amplicon Analysis (IDAA); or high resolution melt analysis (HRM) can be applied to PCR amplicons that span the Cas9 cut site. However, none of these methods approaches the accuracy of NGS analysis, while all are more costly and slower to perform than EMCA methods. Thus, improved methods are needed to assess the frequency of mutations that arise from genome editing experiments that are rapid and low cost.

### BRIEF SUMMARY OF THE INVENTION

The disclosure provides assays making use of high discrimination polymerase mutants or other high mismatch discrimination polymerases to create a new assay design that can utilize mismatches located 5' of the RNA, 3' of the RNA, overlapping with the RNA, and/or in the same position as the RNA.

The invention can be used to provide a more efficient and less error-prone method of detecting mutations in DNA, such as SNPs and indels. The invention also provides a method for performing inexpensive multi-color assays. The invention also provides methods for detection of DNA sequences altered after cleavage by a targetable endonuclease, such as the CRISPR Cas9 protein from the bacterium *Streptococcus pyogenes.*

These and other advantages of the invention, as well as additional inventive features, will be apparent from the description of the invention provided herein.

### BRIEF DESCRIPTION OF THE DRAWINGS

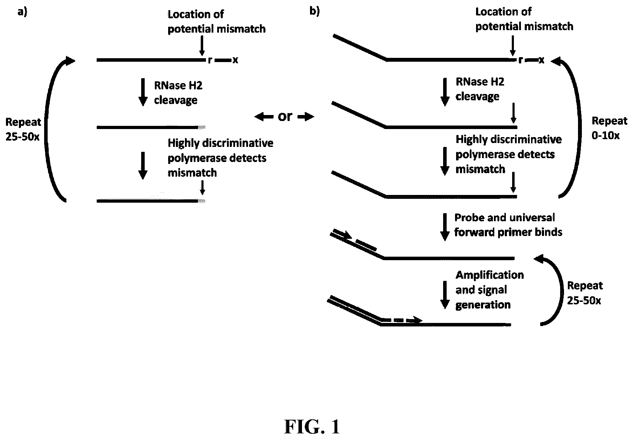

Figure 1 is a diagram showing two primer designs utilized in this invention. Part a) is a blocked-cleavable primer designed so that the SNP of interest is 5' of the RNA base when hybridized to a template. The RNase H2 cleaves, leaving a 3' interrogating base, which is determined to be either a match or a mismatch by the highly discriminative DNA polymerase. Thermal cycling allows for this process to continue. Part b) illustrates the RNase H2 cleavage and SNP detection are identical to a), but the primer also includes a 5' "tail" domain that includes a binding site for a probe and a universal forward primer. After 1-10 cycles of discrimination with the RNase H2 and the polymerase, the highly concentrated universal forward primer comes to dominate the amplification, degrading the probe when it amplifies. This cycle is repeated 25-50x, generating the output signal. This primer design may be multiplexed, allowing for one-tube multi-color assay designs.

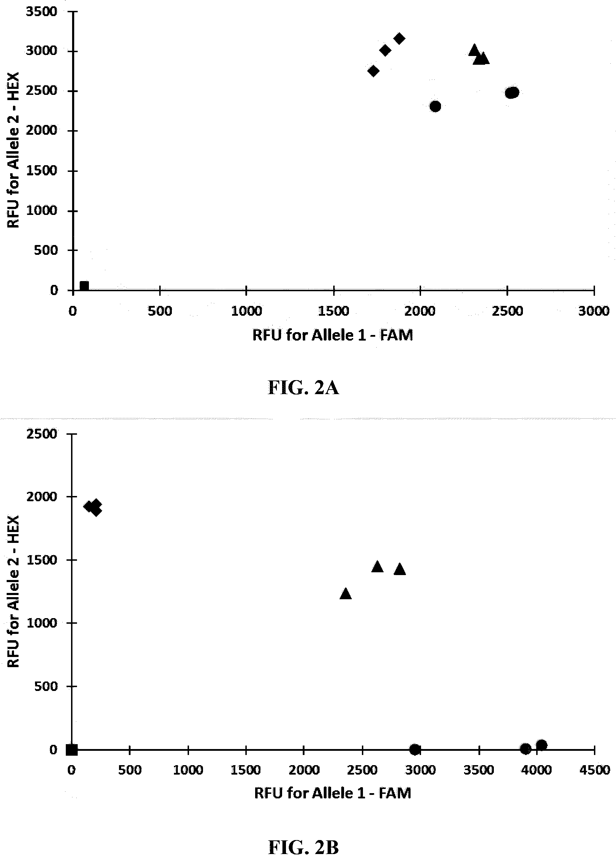

Figures 2A and 2B are end-point fluorescence plots from the assay described in Example 1. FAM and HEX fluorescence values are plotted onto the X and Y axis. Figure 2A is a "Universal" SNP assay for rs351855 performed with WT Taq polymerase. Figure 2B is a "Universal" SNP assay for rs351855 performed with mutant H784Q Taq polymerase, demonstrating greatly enhanced discrimination between each of the allelic variants as observed by the greater separation of the clusters in the mutant Taq case. In both cases, the no template controls (NTCs) (squares) are near the (0,0) coordinates, as desired. Allele 1 samples are shown as circles, allele 2 samples as diamonds, and heterozygotes as triangles. Each reaction was performed in triplicate.

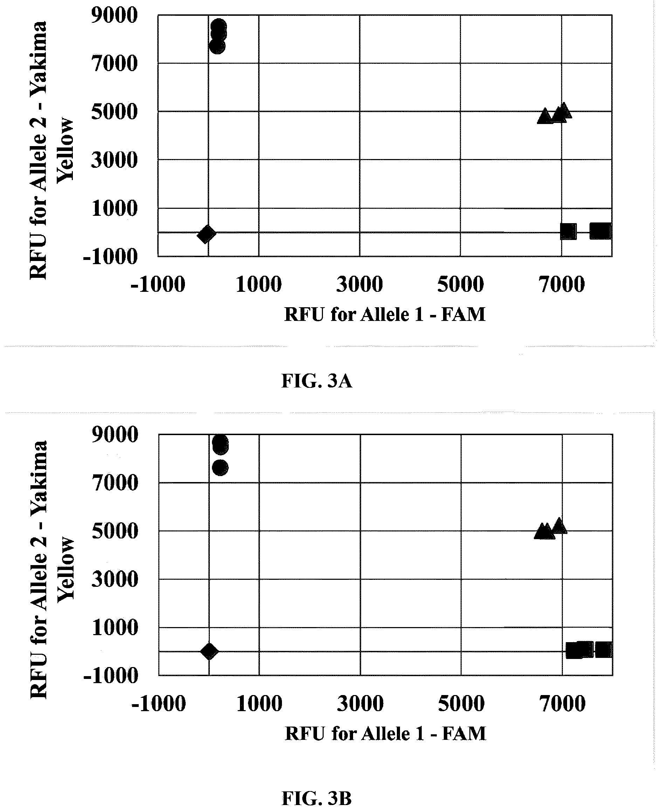

Figures 3A and 3B are allelic discrimination plots with genotyping calls for rs4655751. The reaction plate was cycled immediately after reaction setup (A) or held at room temperature on the benchtop for 48 hours prior to cycling (B). Diamonds: no template controls (NTCs); squares: allele 1 samples; circles: allele 2 samples; triangles: heterozygotes. Genotypes are tightly clustered and have good angle separation, indicating excellent allelic specificity. Each sample was assigned the correct genotyping call, and no change in performance was observed over the 48 hour hold period.

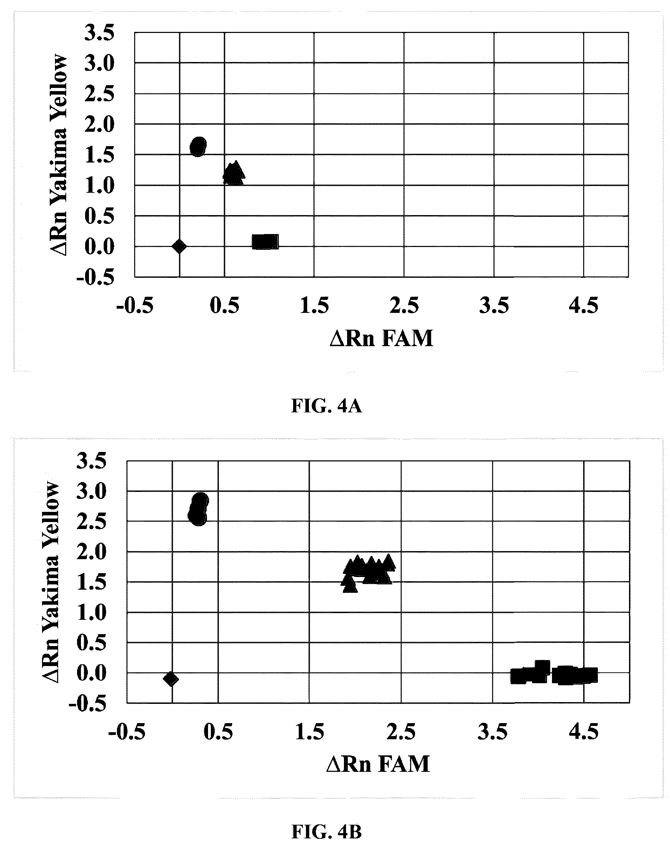

Figures 4A and 4B illustrate a side-by-side comparison of Allelic Discrimination Plots of gene CCR2, rs1799865 from a TaqMan based assay versus rhPCR. Diamonds: no template controls (NTCs); squares: allele 1 samples; circles: allele 2 samples; triangles: heterozygotes. The rhPCR Genotyping Assay (FIG. 4B) achieved higher fluorescence signal compared to a traditional 5'-nuclease genotyping assay (FIG. 4A) while showing concordant results.

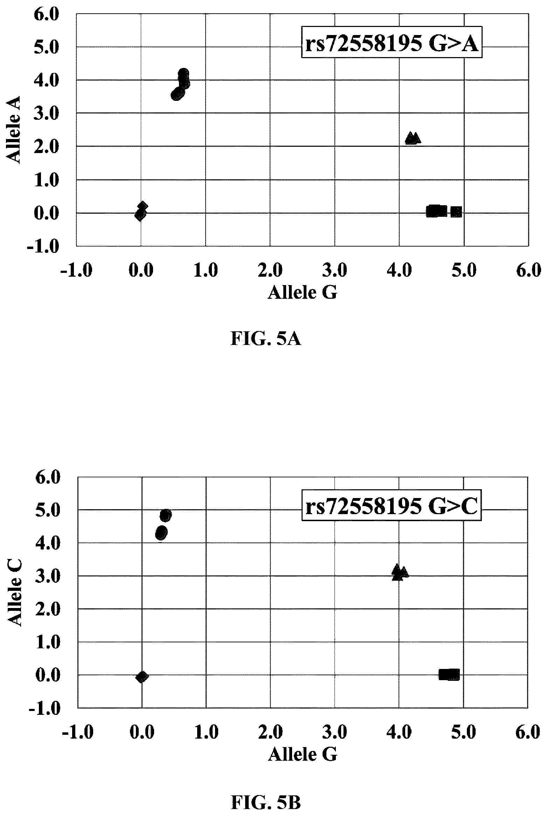

Figures 5A and 5B are Allelic Discrimination plots of tri-allelic SNP, CYP2C8 (rs72558195), using an rhPCR genotyping single tube multiplex assay on the QuantStudio^{™} 7 Flex platform (Thermo Fisher). In Figure 5A, diamonds: no template controls (NTCs); squares: allele G (allele 1) samples; circles: allele A (allele 2) samples; triangles: heterozygotes. In Figure 5B, diamonds: no template controls (NTCs); squares: allele G (allele 1) samples; circles: allele C (allele 3) samples; triangles: heterozygotes.

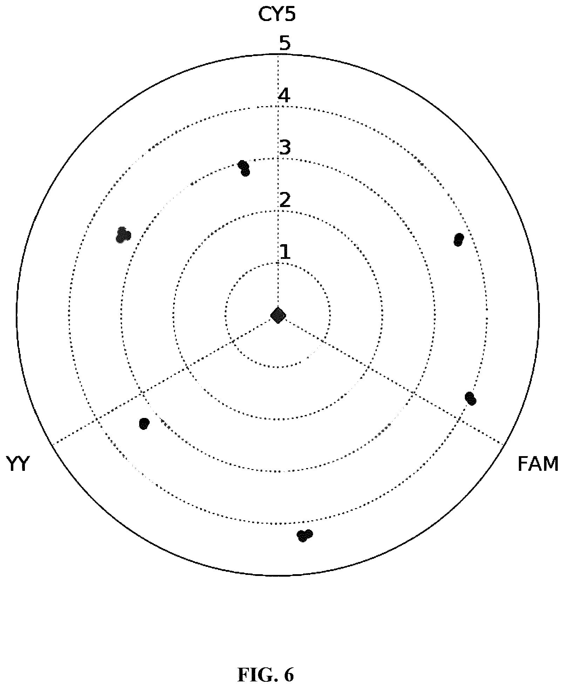

Figure 6 shows the Tri-allelic Allelic Discrimination 360plot of CYP2C8 rs72558195, using rhPCR genotyping assay with 3 allele-specific primers multiplexed in a single reaction.

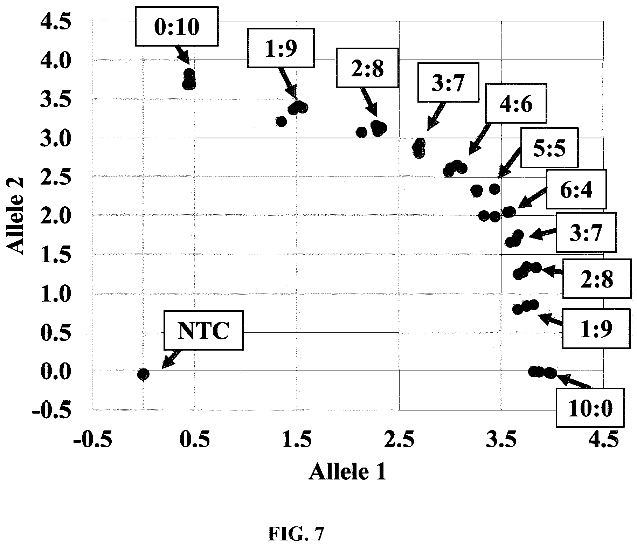

Figure 7 is an allelic discrimination plot illustrating the ability of the rhPCR assay to perform quantitative genotyping.

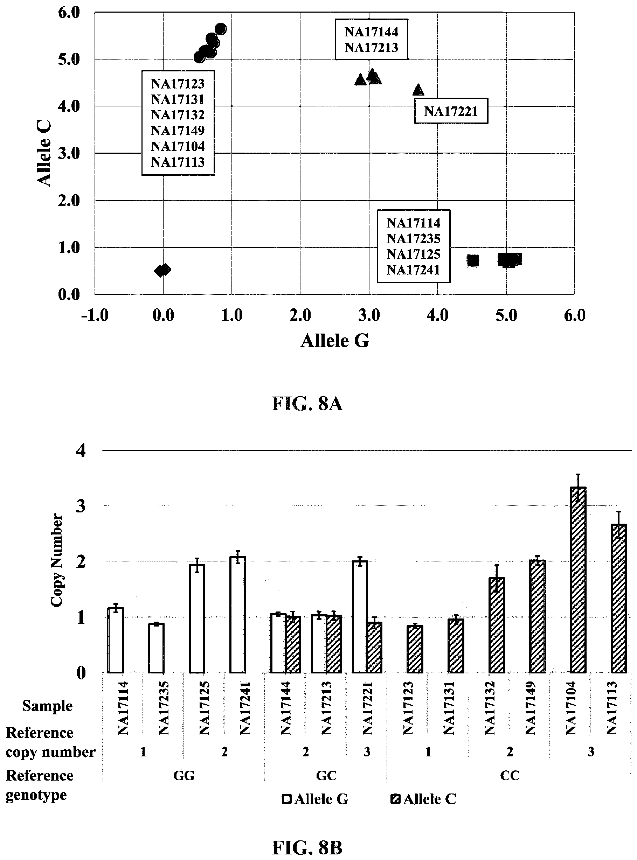

Figures 8A and 8B illustrate genotyping results and detection of allelic copy number variation that is possible with the present invention. gDNA samples were tested using varying copy numbers and varying reference genotypes. In Figure 8A, diamonds: no template controls (NTCs); squares: allele G samples; circles: allele C samples; and triangles: heterozygotes. The resulting data correlates with the test input.

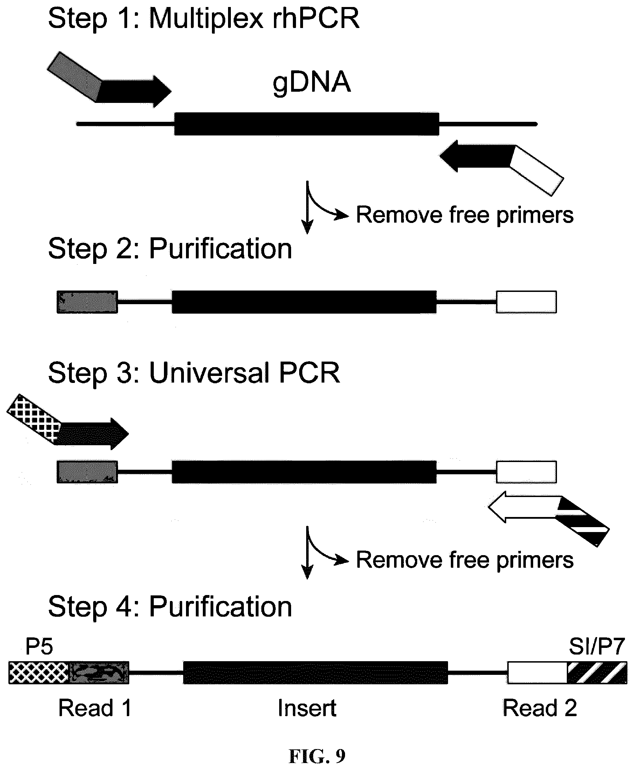

Figure 9 is a schematic representation of multiplex rhPCR.

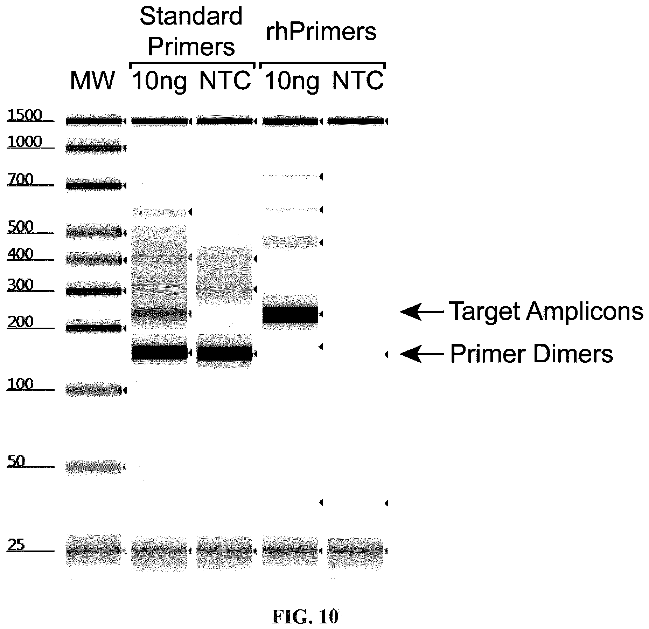

Figure 10 is the resulting tape station image indicating the effectiveness of the multiplex rhPCR methods in reducing primer dimers and increasing desired amplicon yield.

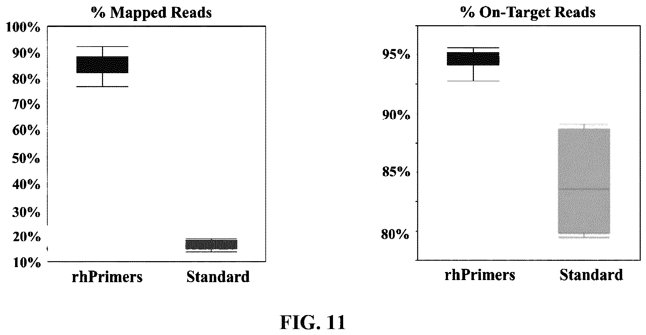

Figure 11 graphically represents the effectiveness of the rhPrimers in the percent of mapped reads and on-target reads.

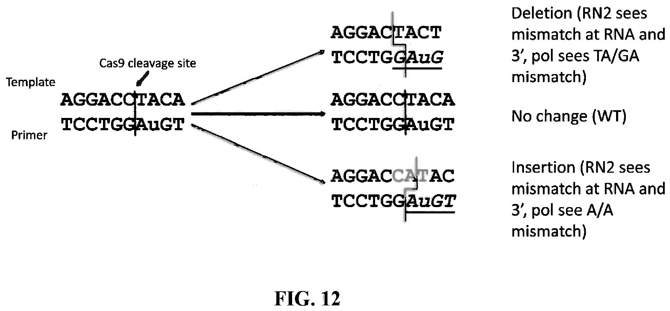

Figure 12 shows placement of the RNA residue relative to the most common cleavage site for the methods of the disclosure (such as in Example 10). The RNA is shown in lower case, while DNA residues are shown in upper case. Cas9 cleavage site is shown with a line through both strands of DNA. RN2 = RNase H2 enzyme; pol = polymerase.

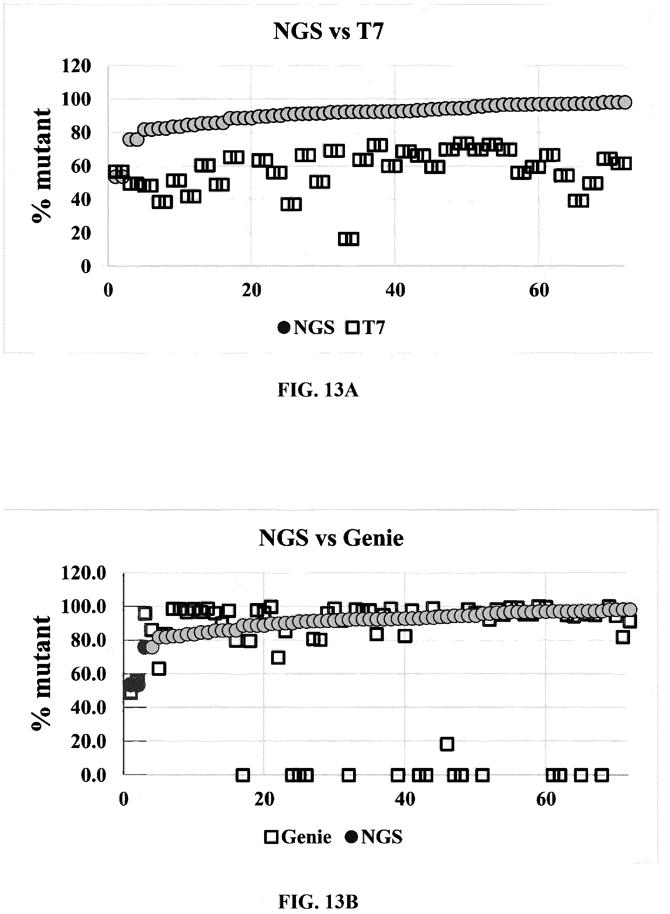

Figures 13A-13B show analysis of CRISPR mutations demonstrating that the results obtained using the qPCR methods of the disclosure are more accurate than using the T7EI EMCA method. Figure 13A: the percentage of mutated templates is consistently underestimated by T7EI EMCA (empty squares), when compared with NGS results (grey circles) on the same samples. Figure 13B: using the same samples from Figure 13A, the percentage of mutations detected is seen to be much more accurately estimated by the qPCR methods of the disclosure (empty squares) when compared with NGS results.

### DETAILED DESCRIPTION OF THE INVENTION

The invention pertains to a methods of single-nucleotide polymorphism (SNP) discrimination utilizing blocked-cleavable rhPCR primers (see U.S. Patent Application Publication No. US 2009/0325169 A1) and a DNA polymerase with high levels of mismatch discrimination. In one embodiment, the mismatch is placed at a location other than opposite the RNA base. In these situations, the majority of the discrimination comes not from the RNase H2, but from the high discrimination polymerase. The use of blocked-cleavable primers with RNase H2 acts to reduce or eliminate primer-dimers and provide some increased amount of SNP or indel (insertion/deletion) discrimination (Figure 1a).

For the purposes of this invention, high discrimination is defined as any amount of discrimination over the average discrimination of WT Thermus aquaticus (Taq) polymerase. Examples include KlenTaq^{®} DNA polymerase (Wayne Barnes), and mutant polymerases described in U.S. Patent Application Publication No. US 2015/0191707 such as H784M, H784S, H784A and H784Q mutants.

In a further embodiment a universal detection sequence(s) is added to the 5'-end of the blocked-cleavable primers. The detection sequence includes a binding site for a probe, and a binding site for a universal amplification primer. The primer binding site is positioned at or near the 5'-end of the final oligonucleotide and the probe binding site is positioned internally between the universal primer site and the SNP-detection primer domain. Use of more than one such chimeric probe in a detection reaction wherein distinct probe binding sites are employed allows primers to be multiplexed and further allows for multiple color detection of SNPs or other genomic features. Blocked-cleavable rhPCR primers reduce or eliminate primer-dimers. Primer-dimers are a major problem for use of "universal" primer designs in SNP detection assays, and that limits their utility (Figure 1b). Combining a universal amplification/detection domain with a SNP primer domain in blocked-cleavable primer format overcomes this difficulty.

Previously, the best preferred embodiment for rhPCR SNP discrimination employed blocked-cleavable primers having the mismatch (SNP site) positioned opposite the single RNA base (cleavage site). While this works for many SNP targets, there are base match/mismatch pairings where sufficient discrimination is not obtained for robust base calling. Moreover, due to the high level of differential SNP discrimination observed with rhPCR, end-point detection can be difficult, especially with heterozygous target DNAs. In the proposed method, the RNA base is identical in both discriminating primers, eliminating this issue.

Placing the mismatch at either a position 5' or 3' of the RNA is also useful in some embodiments. For example, a mutation located 5' of the RNA allows for the use of a highly discriminatory DNA polymerase as a secondary selection step during PCR amplification following primer cleavage, although this configuration restricts the mismatch discrimination potential of the RNase H2 to the first cleavage event, and subsequent priming events on daughter amplicons will bind to whatever sequence was present in the primer. Placement of the mismatch 3' of the RNA reduces or eliminates the potential for primer conversion to the opposite allele.

Thus, in some aspects, the disclosure provides methods and compositions comprising mutant RNase H2 enzymes that show enhanced mismatch discrimination during rhPCR when the mutations are positioned 5'- or 3'- relative to the RNA residue. For example, and without limiting the scope of the disclosed aspects and embodiments of the disclosure, mutations located at the twelfth, thirteenth, and 169th amino acid positions in the RNase H2 enzyme from *Pyrococcus abyssi (P.a.)* have been identified that have this unexpected and useful property. Similar mutations in RNase H2 enzymes from other species that support rhPCR may likewise show similar improved properties relative to their WT forms.

In other embodiments, the methods of the disclosure comprise the use of blocked-cleavable primers wherein the mismatch is placed 1-2 bases 5' of the RNA. In a further embodiment, the method involves the use of blocked-cleavable primers with three or more DNA bases 3' of an RNA residue, and the primers are designed such that the mismatch is placed immediately 5' of the RNA.

Following cleavage by RNase H2, the remaining primer has a DNA residue positioned at the 3'-end exactly at the SNP site, effectively creating an ASPCR primer. In this configuration, a high-specificity DNA polymerase can discriminate between match and mismatch with the template strand (Figure 1a and b). Native DNA polymerases, such as Taq DNA polymerase, will show some level of discrimination in this primer configuration, and if the level of discrimination achieved is not sufficient for robust SNP calling in a high throughput assay format then the use of polymerases with improved template discrimination can be used. In one embodiment, mutant DNA polymerases, such as those disclosed in U.S. Patent Application Publication No. US 2015/0191707 or any other polymerase designed or optimized to improve template discrimination can be used. When using polymerases with increased mismatch discrimination, the final level of match/mismatch discrimination achieved will be additive with contributions from both the ASPCR primer polymerase interaction and from the rhPCR primer/RNaseH2 interaction. Further, the use of blocked-cleavable primers reduces risk of primer-dimer formation, which produces false-positive signals, making the overall reaction more robust and having higher sensitivity and higher specificity. The relative contributions of each component of the assay may vary with use of different polymerases, different blocking groups on the 3'-end of the primer and different RNase H2 enzymes.

In another embodiment, the invention may utilize a "tail" domain added to the 5' end of the primer, containing a universal forward primer binding site sequence and optionally a universal probe sequence. This tail would not be complementary to the template of interest, and when a probe is used, the tail would allow for inexpensive fluorescent signal detection, which could be multiplexed to allow for multiple color signal detection in qPCR (Figure 1b). In one embodiment, 1-10 cycles of initial cycling and discrimination occurs from both the RNase H2 and the DNA polymerase. After this initial pre-cycling, a highly concentrated and non-discriminatory universal forward primer comes to dominate the amplification, degrading the probe and generating the fluorescent signal when the DNA amplifies. This cycle is repeated 25-50x, allowing for robust detection. This assay design is prone to issues with primer-dimers, and the presence of the blocked-cleavable domain in the primers will suppress or eliminate these issues.

In another embodiment, a forward primer is optionally used with a reverse primer, and a tail domain is added to the 5' end of one or both of a forward and reverse primer set. The tail domain comprises a universal forward primer binding site. The primers can be used to hybridize and amplify a target such as a genomic sample of interest. The primers would add universal priming sites to the target, and further cycles of amplification can be performed using universal primers that contain adapter sequences that enable further processing of the sample, such as the addition of P5/P7 flow cell binding sites and associated index or barcoding sequences useful in adapters for next-generation sequencing (see Fig. 9). In a further embodiment a high fidelity polymerase is used, which will further lower the rate of base misincorporation into the extended product and increase the accuracy of the methods of the invention.

As noted in U.S. Patent Application Publication No. US 2009/0325169, RNase H2 can cleave at positions containing one or more RNA bases, at 2'-modified nucleosides such as 2'-fluoronucleosides. The primers can also contain nuclease resistant linkages such as phosphorothioate, phosphorodithioate, or methylphosphonate.

Further aspects of the disclosure pertain to detection of DNA sequences altered after cleavage by a targetable endonuclease, such as the CRISPR Cas9 protein from the bacterium *Streptococcus pyogenes.* This protein and similar ones have successfully been used for targeted genomic modification in the well documented Clustered Regularly Interspaced Short Palindromic Repeats (CRISPR) system.

In a further embodiment, the tailed primers detailed above could be used to detect editing events for genome editing technology. For example, CRISPR/Cas9 is a revolutionary strategy in genome editing that enables generation of targeted, double-stranded breaks (DSBs) in genomic DNA. Methods to achieve DSBs by CRISPR/Cas9-a bacterial immune defense system comprised of an endonuclease that is targeted to double-stranded DNA by a guide RNA-are widely used in gene disruption, gene knockout, gene insertion, etc. In mammalian cells, the endonuclease activity is followed by an endogenous repair process that leads to some frequency of insertions/deletions/substitutions in wild-type DNA at the target locus which gives the resultant genome editing.

RNase H-cleavable primers have been designed to flank edited loci in order to 1) generate locus-specific amplicons with universal tails, and 2) be subsequently amplified with indexed P5/P7 universal primers for next-generation sequencing. In pilot experiments, this strategy resulted in reliable, locus-specific amplification which captures CRISPR/Cas9 editing events in a high-throughput and reproducible manner. The key finding is that the overall targeted editing by this NGS-based method was determined to be 95%; whereas, previous enzymatic strategies suggested overall editing from the same samples was approximately 55% at the intended target site. Further, primers were designed to amplify off-target locations of genomic editing based on in silico predictions by internal bioinformatics tools.

These assays would be pooled for amplification of a single genomic DNA sample in order to capture the on-target as well as > 100 potential sites for off-target genome editing mediated by sequence homology to the guide RNA. The results from this experiment would allow for 1) identification of CRISPR/Cas9 off-target sites and provide an assay for comparing strategies to reduce those effects, 2) improved design of the CRISPR/Cas9 off-target prediction algorithm, and 3) improved design of primer sets.

Thus, in further aspects, the disclosure provides methods that employ the above-described universal rhPCR assay system to detect mutations generated by a targetable endonuclease such as Cas9 or Cpfl. The rhPCR assays according to these aspects of the disclosure utilizes a thermostable RNase H2 enzyme, and optionally a DNA polymerase with enhanced mismatch discrimination. The RNase H2 cleaves at the single RNA residue only when the primer oligonucleotide is duplexed with a target nucleic acid, which removes a 3'-blocking group and activates the primer. The DNA polymerase uses the primer to initiate DNA synthesis and, in multiple cycles, supports PCR. Discrimination of mutations is achieved by the action of the RNase H2 or the combined action of both the RNase H2 and the DNA polymerase, wherein the RNase H2 has reduced de-blocking activity when a mismatch is present and the DNA polymerase has reduced priming/DNA synthesis activity when a mismatch is present. In one embodiment, the primers comprise multiple functional domains including (from the 5'-end): a universal primer domain, a universal probe binding domain, a target-specific primer domain, a single RNA residue (cleavable linkage), a short 3'-extension domain, and a 3'-blocking group that prevents the oligonucleotide from priming DNA synthesis. Cleavage by RNase H2 removes the RNA residue, 3'-extension domain, and 3'-blocking group.

In some embodiments, a second assay is present in the reaction and runs as a 2-color multiplex, targeting the RNase P gene or some other control gene. This second assay allows for normalization to an internal control gene that was not targeted by the CRISPR genome editing reaction. This control assay may be performed as either a standard three-oligonucleotide 5' nuclease assay, or as a second rhPCR-based universal assay.

In another embodiment, the primers lack the universal 5' domain, but still retain the 3' removable blocking group. In this alternative embodiment, a standard 5' nuclease fluorescence-quenched probe is placed between the forward and reverse primers. The probe is positioned within the amplicon such that it lies outside of any region that may be altered by the genome editing event.

In each experiment, relative position of the discriminatory (*i.e.,* mutation interrogating) primer on the sequence is important. RNase H2 cleaves 5' of an RNA residue. Placement of the primer so that the RNA residue binds two nucleotides after the most common cleavage site is important for recognition of the mutagenized samples. A diagram of this principle is shown in Figure 12. In the Wild-Type (WT) samples, amplification occurs normally, as neither the RNase H2 nor the DNA polymerase are hindered in their functions. If an insertion is introduced to the sequence, a mismatch for both the RNase H2 and the DNA polymerase are produced, allowing two independent chances to distinguish mutant from WT. The same interrogation of the samples is achieved if a deletion is present-both the RNase H2 and the DNA polymerase detect the mismatches generated (Figure 12). This double level of interrogation allows for very precise quantification of the presence of mutated DNA in a heterogeneous sample.

Thus, in one aspect, the disclosure provides blocked-cleavable primers for rhPCR, the primers comprising: 5'-A-B-C-D-E-3', wherein A is optional and is a tail extension that is not complementary to a target; B is a sequence domain that is complementary to a target; C is a discrimination domain; D is a cleavable domain that, when hybridized to the target, is cleavable by RNase H2; and E is a blocking domain that prevents extension of the primer.

In some embodiments, D is comprised of 1-3 RNA bases. In some embodiments, D is comprised of 1 RNA base. In some embodiments, the cleavage domain comprises one or more of the following moieties: a DNA residue, an abasic residue, a modified nucleoside, or a modified phosphate internucleotide linkage. In some embodiments, a sequence flanking the cleavage site contains one or more internucleoside linkages resistant to nuclease cleavage. In some embodiments, the nuclease resistant linkage is a phosphorothioate. In some embodiments, the 3' oxygen atom of at least one of the RNA residues is substituted with an amino group, thiol group, or a methylene group. In some embodiments, the blocking group is attached to the 3'-terminal nucleotide of the primer. In some embodiments, A is comprised of a region that is identical to a universal forward primer and optionally a probe binding domain.

In another aspect, the disclosure provides methods of detecting a variation in a target DNA sequence, the method comprising: (a) providing a reaction mixture comprising (i) an oligonucleotide primer having a cleavage domain positioned 5' of a blocking group and 3' of a position of variation, the blocking group linked at or near the end of the 3'-end of the oligonucleotide primer wherein the blocking group prevents primer extension and/or inhibits the primer from serving as a template for DNA synthesis, (ii) a sample nucleic acid that may or may not have the target sequence, and where the target sequence may or may not have the variation (iii) a cleaving enzyme and (iv) a polymerase; (b) hybridizing the primer to the target DNA sequence to form a double-stranded substrate; (c) cleaving the hybridized primer, if the primer is complementary at the variation, with the cleaving enzyme at a point within or adjacent to the cleavage domain to remove the blocking group from the primer; and (d) extending the primer with the polymerase.

In some embodiments, the cleaving enzyme is a hot start cleaving enzyme which is thermostable and has reduced activity at lower temperatures. In some embodiments, the cleaving enzyme is a chemically modified hot start cleaving enzyme which is thermostable and has reduced activity at lower temperatures. In some embodiments, the hot start cleaving enzyme is a chemically modified *Pyrococcus abyssi* RNase H2. In some embodiments, the cleaving enzyme is a hot start cleaving enzyme that is reversibly inactivated through interaction with an antibody at lower temperatures which is thermostable and has reduced activity at lower temperatures. In some embodiments, the cleaving domain is comprised of at least one RNA base, and the cleaving enzyme cleaves between the position complementary to the variation and the RNA base. In some embodiments, the cleaving domain is comprised of one or more 2'-modified nucleosides, and the cleaving enzyme cleaves between the position complementary to the variation and the one or more modified nucleosides. In some embodiments, the one or more modified nucleosides are 2'-fluoronucleosides. In some embodiments, the polymerase is a high-discrimination polymerase. In some embodiments, the polymerase is a mutant H784Q Taq polymerase. In some embodiments, the mutant H784Q Taq polymerase is reversibly inactivated via chemical, aptamer or antibody modification. In some embodiments, the primer contains a 5' tail sequence that comprises a universal primer sequence and optionally a universal probe sequence, wherein the tail is non-complementary to the target DNA sequence. In some embodiments, the target DNA sequence is a sample that has been treated with a gene editing enzyme. In some embodiments, the target DNA sequence is a sample that has been treated with a CRISPR enzyme. In some embodiments, the target DNA sequence is a sample that has been treated with a Cas9 or Cpfl enzyme.

In another aspect, the disclosure provides oligonucleotide compositions for genotyping a target sample, wherein the composition comprises, from 5' to 3': (a) a tailing portion that is non-complementary to the target sample but contains (5' to 3') a first sequence that is identical to a universal forward primer and a second sequence that is identical to a reporter probe sequence that corresponds to an allele; (b) a region complementary to a target; (c) an allele-specific domain; and (d) a 3' terminal region containing a blocking domain. In some embodiments, the allele-specific domain is capable of being cleaved by an RNase H enzyme when hybridized to the target sample. In some embodiments, the allele-specific domain is 5'-D-R-3', wherein D is a DNA base that aligns with a SNP position of interest and R is an RNA base. In some embodiments, the oligonucleotide is about 15-30 bases long. In some embodiments, the blocking domain is comprised of 0-5 DNA bases. In some embodiments, the blocking domain is comprised of at least two of the flowing: DNA, RNA, 1,3-propanediol, mismatched DNA or RNA, and a labeling moiety. In some embodiments, the allele-specific domain is comprised of 2'-fluoro analogues.

In another aspect, the disclosure provides kits for genotyping assays, the kits comprising: (a) a first allele oligonucleotide comprised as in the previous aspect; (b) a second allele oligonucleotide comprised as in the previous aspect; (c) a locus-specific reverse primer; (d) a universal forward primer; (e) a polymerase; (f) a first probe corresponding to a first allele; (g) a second probe corresponding to a second allele; and (h) an RNase H enzyme.

In another aspect, the disclosure provides methods of visualization of multiple different fluorescent signals from allelic amplification plots, the methods comprising: (a) using three fluorescent signals from multiple fluorescent dye signals in a single reaction well, subtracting a lowest fluorescence Dye₃ from fluorescence signals from Dye₁ and Dye₂; (b) calculating the distance of data from an origin and an angle from one of the axis with an equation $Distance from origin = \sqrt{{\left(Δ{Rn}_{Dye 1}\right)}^{2} + {\left(Δ{Rn}_{Dye 2}\right)}^{2}}$ $Angle = {tan}^{- 1} \left(Δ{Rn}_{Dye 1}\div Δ{Rn}_{Dye 2}\right) \times \frac{120}{90} ;$ and (c) plotting on a circle plot with three axis, one for each dye or allele, the resulting distance.

In another aspect, the disclosure provides methods of target enrichment comprising: (a) providing a reaction mixture comprising (i) a first oligonucleotide primer having a tail domain that is not complementary to a target sequence, the tail domain comprising a first universal primer sequence; a cleavage domain positioned 5' of a blocking group and 3' of a position of variation, the blocking group linked at or near the end of the 3'-end of the first oligonucleotide primer wherein the blocking group prevents primer extension and/or inhibits the first primer from serving as a template for DNA synthesis, (ii) a sample nucleic acid that may or may not have the target sequence, (iii) a cleaving enzyme and (iv) a polymerase; (b) hybridizing the first primer to the target DNA sequence to form a double-stranded substrate; (c) cleaving the hybridized first primer, if the first primer is complementary to the target, with the cleaving enzyme at a point within or adjacent to the cleavage domain to remove the blocking group from the first primer; and (d) extending the first primer with the polymerase.

In some embodiments, the methods further comprise a second primer in reverse orientation to support priming and extension of the first primer extension product. In some embodiments, the second primer further comprises a tail domain comprising a second universal primer sequence. In some embodiments, steps b-d are performed 1-10 times. In some embodiments, the methods further comprise removing unextended primers from the reaction and hybridizing universal primers to the extension product to form a second extension product. In some embodiments, the universal primers further comprise tailed sequences for addition of adapter sequences to the second extension product. In some embodiments, sequencing is performed on the second extension product to determine the sequence of the target. In some embodiments, the target DNA sequence is a sample that has been treated with a gene editing enzyme. In some embodiments, the target DNA sequence is a sample that has been treated with a CRISPR enzyme. In some embodiments, the target DNA sequence is a sample that has been treated with a Cas9 or Cpfl enzyme. In some embodiments, the cleaving enzyme is a hot start cleaving enzyme which is thermostable and has reduced activity at lower temperatures. In some embodiments, the cleaving enzyme is a chemically modified hot start cleaving enzyme which is thermostable and has reduced activity at lower temperatures. In some embodiments, the hot start cleaving enzyme is a chemically modified *Pyrococcus abyssi* RNase H2. In some embodiments, the cleaving enzyme is a hot start cleaving enzyme that is reversibly inactivated through interaction with an antibody at lower temperatures which is thermostable and has reduced activity at lower temperatures. In some embodiments, the cleaving domain is comprised of at least one RNA base, and the cleaving enzyme cleaves between the position complementary to the variation and the RNA base. In some embodiments, the cleaving domain is comprised of one or more 2'-modified nucleosides, and the cleaving enzyme cleaves between the position complementary to the variation and the one or more modified nucleosides. In some embodiments, the one or more modified nucleosides are 2'-fluoronucleosides. In some embodiments, the polymerase is a high-discrimination polymerase. In some embodiments, the polymerase is a mutant H784Q Taq polymerase. In some embodiment the mutant H784Q Taq polymerase is reversibly inactivated via chemical, aptamer or antibody modification.

In another aspect, the disclosure provides methods of detecting variations in target DNA sequences that have been altered with a gene editing enzyme, the methods comprising: (a) providing a reaction mixture comprising: (i) an oligonucleotide primer having a cleavage domain positioned 5' of a blocking group and 3' of a position of variation, the blocking group linked at or near the end of the 3'-end of the oligonucleotide primer wherein the blocking group prevents primer extension and/or inhibits the primer from serving as a template for DNA synthesis; (ii) a sample nucleic acid that may or may not have the target sequence, and where the target sequence may or may not have the variation; (iii) a cleaving enzyme; and (iv) a polymerase; (b) hybridizing the primer to the target DNA sequence to form a double-stranded substrate; (c) cleaving the hybridized primer, if the primer is complementary at the variation, with the cleaving enzyme at a point within or adjacent to the cleavage domain to remove the blocking group from the primer; and (d) extending the primer with the polymerase.

In some embodiments, the target DNA sequence has been treated with a CRISPR enzyme. In some embodiments, the target DNA sequence has been treated with a Cas9 or Cpf1 enzyme. In some embodiments, the cleaving enzyme is a hot start cleaving enzyme which is thermostable and has reduced activity at lower temperatures. In some embodiments, the cleaving enzyme is an RNase H2 enzyme. In some embodiments, the cleaving enzyme is *Pyrococcus abyssi* RNase H2 enzyme. In some embodiments, the cleaving enzyme is a chemically modified hot start cleaving enzyme which is thermostable and has reduced activity at lower temperatures. In some embodiments, the hot start cleaving enzyme is a chemically modified *Pyrococcus abyssi* RNase H2. In some embodiments, the cleaving enzyme is a hot start cleaving enzyme that is reversibly inactivated through interaction with an antibody at lower temperatures.

In some embodiments, the cleavage domain comprises at least one RNA base, and the cleaving enzyme cleaves between the position complementary to the variation and the RNA base. In some embodiments, the cleavage domain comprises at least one RNA base located 3' of the position of variation, and comprises one DNA base between the position of variation and the RNA base. In some embodiments, there are no DNA bases between the position of variation and the RNA base. In other embodiments, the RNA base is located within the position of variation. In some embodiments, the cleavage domain comprises one or more 2'-modified nucleosides, and the cleaving enzyme cleaves between the position complementary to the variation and the one or more modified nucleosides. In some embodiments, the one or more modified nucleosides are 2' -fluoronucleosides.

In some embodiments, the polymerase is a high-discrimination polymerase. In some embodiments, the polymerase is a mutant H784Q Taq polymerase. In some embodiments, the mutant H784Q Taq polymerase is reversibly inactivated via chemical, aptamer, or antibody modification. In some embodiments, the primer contains a 5' tail sequence that comprises a universal primer sequence and optionally a universal probe sequence, wherein the tail is non-complementary to the target DNA sequence.

In some embodiments, the methods of this aspect of the disclosure further comprise (e) detection of an internal control gene not targeted by the gene editing enzyme; and (f) normalization of the results of steps (a)-(d) to the results of step (e). In some embodiments, the internal control gene not targeted by the gene editing enzyme is the RNase P gene. In some embodiments, the reaction mixture further comprises a control oligonucleotide primer specific for the internal control gene not targeted by the gene editing enzyme, wherein the control oligonucleotide primer comprises a cleavage domain positioned 5' of a blocking group and 3' of a position of variation, the blocking group linked at or near the end of the 3'-end of the oligonucleotide primer wherein the blocking group prevents primer extension and/or inhibits the primer from serving as a template for DNA synthesis. In some embodiments, the internal control gene not targeted by the gene editing enzyme is detected using a three-oligonucleotide 5' nuclease assay.

In another aspect, the disclosure provides methods of target enrichment comprising: (a) providing a reaction mixture comprising: (i) a first oligonucleotide primer having a tail domain that is not complementary to a target sequence, the tail domain comprising a first universal primer sequence; a cleavage domain positioned 5' of a blocking group and 3' of a position of variation, the blocking group linked at or near the end of the 3'-end of the first oligonucleotide primer wherein the blocking group prevents primer extension and/or inhibits the first primer from serving as a template for DNA synthesis; (ii) a sample nucleic acid that has been treated with a gene editing enzyme, which may or may not have the target sequence; (iii) a cleaving enzyme; and (iv) a polymerase; (b) hybridizing the first primer to the target DNA sequence to form a double-stranded substrate; (c) cleaving the hybridized first primer, if the first primer is complementary to the target, with the cleaving enzyme at a point within or adjacent to the cleavage domain to remove the blocking group from the first primer; and (d) extending the first primer with the polymerase.

In some embodiments, the target DNA sequence is a sample that has been treated with a CRISPR enzyme. In some embodiments, the target DNA sequence is a sample that has been treated with a Cas9 or Cpfl enzyme. In some embodiments, the methods further comprise a second primer in reverse orientation to support priming and extension of the first primer extension product. In some embodiments, the second primer further comprises a tail domain comprising a second universal primer sequence. In some embodiments, steps (b)-(d) are performed 1-10 times. In some embodiments, the methods further comprise removing unextended primers from the reaction and hybridizing universal primers to the extension product to form a second extension product. In some embodiments, the universal primers further comprise tailed sequences for addition of adapter sequences to the second extension product. In some embodiments, sequencing is performed on the second extension product to determine the sequence of the target.

In some embodiments, the cleaving enzyme is a hot start cleaving enzyme which is thermostable and has reduced activity at lower temperatures. In some embodiments, the cleaving enzyme is an RNase H2 enzyme. In some embodiments, the cleaving enzyme is *Pyrococcus abyssi* RNase H2 enzyme. In some embodiments, the cleaving enzyme is a chemically modified hot start cleaving enzyme which is thermostable and has reduced activity at lower temperatures. In some embodiments, the hot start cleaving enzyme is a chemically modified *Pyrococcus abyssi* RNase H2. In some embodiments, the cleaving enzyme is a hot start cleaving enzyme that is reversibly inactivated through interaction with an antibody at lower temperatures which is thermostable and has reduced activity at lower temperatures. In some embodiments, the cleavage domain comprises at least one RNA base, and the cleaving enzyme cleaves between the position complementary to the variation and the RNA base.

In some embodiments, the cleavage domain comprises at least one RNA base located 3' of the position of variation, and comprises one DNA base between the position of variation and the RNA base. In some embodiments, there are no DNA bases between the position of variation and the RNA base. In other embodiments, the RNA base is located within the position of variation. In some embodiments, the cleavage domain comprises one or more 2'-modified nucleosides, and the cleaving enzyme cleaves between the position complementary to the variation and the one or more modified nucleosides. In some embodiments, the one or more modified nucleosides are 2'-fluoronucleosides. In some embodiments, the polymerase is a high-discrimination polymerase. In some embodiments, the polymerase is a mutant H784Q Taq polymerase. In some embodiments, the mutant H784Q Taq polymerase is reversibly inactivated via chemical, aptamer or antibody modification.

In another aspect, the disclosure provides blocked-cleavable primers for rhPCR, comprising: 5'-A-B-C-D-E-3', wherein A is optional and is a tail extension that is not complementary to a target; B is a sequence domain that is complementary to a target; C is a discrimination domain; D is a cleavage domain that, when hybridized to the target, is cleavable by RNase H2, and which comprises an RNA base; and E is a blocking domain that prevents extension of the primer.

In some embodiments, D is a cleavage domain that, when hybridized to the target, is cleavable by RNase H2, and which comprises an RNA base that is: separated from the discrimination domain by one base position, within the discrimination domain, or adjacent to the discrimination domain. In some embodiments, the RNA base is separated from the discrimination domain by one base position. In some embodiments, the RNA base is within the discrimination domain. In some embodiments, the RNA base is adjacent to the discrimination domain. For example, when the RNA base is adjacent to the discrimination domain, no intervening DNA residue is present between the RNA base and the discrimination domain.

In some embodiments, D comprises 1-3 RNA bases. In some embodiments, the cleavage domain comprises one or more of the following moieties: a DNA residue, an abasic residue, a modified nucleoside, or a modified phosphate internucleotide linkage. In some embodiments, a sequence flanking the cleavage site contains one or more internucleoside linkages resistant to nuclease cleavage. In some embodiments, the nuclease resistant linkage is a phosphorothioate. In some embodiments, the 3' oxygen atom of at least one of the RNA residues is substituted with an amino group, thiol group, or a methylene group. In some embodiments, the blocking group is attached to the 3'-terminal nucleotide of the primer. In some embodiments, A is comprised of a region that is identical to a universal forward primer and optionally a probe binding domain.

In some embodiments, the discrimination domain C does not comprise or overlap with the cleavage domain D. In some other embodiments, the discrimination domain C comprises the cleavage domain D. In some other embodiments, the discrimination domain C overlaps with the cleavage domain D.

In another aspect, the disclosure provides blocked-cleavable primers for rhPCR, the primers comprising:

5'-A-B-Z-E-3'

wherein A is optional and is a tail extension that is not complementary to a target; B is a sequence domain that is complementary to a target; Z comprises: C, a discrimination domain, and D, a cleavage domain that, when hybridized to the target, is cleavable by RNase H2; and E is a blocking domain that prevents extension of the primer.

In some embodiments, the discrimination domain C is located 5' of the cleavage domain D. In some embodiments, the discrimination domain C is located 3' of the cleavage domain D. In some embodiments, the discrimination domain C overlaps with the cleavage domain D. In some embodiments, C comprises 1-3 RNA bases. In some embodiments, C comprises 1 RNA base. In some embodiments, the discrimination domain C consists of the 1 RNA base. In some embodiments, the cleavage domain comprises one or more of the following moieties: a DNA residue, an abasic residue, a modified nucleoside, or a modified phosphate internucleotide linkage. In some embodiments, a sequence flanking the cleavage site contains one or more internucleoside linkages resistant to nuclease cleavage. In some embodiments, the nuclease resistant linkage is a phosphorothioate. In some embodiments, the 3' oxygen atom of at least one of the RNA residues is substituted with an amino group, thiol group, or a methylene group. In some embodiments, the blocking group is attached to the 3'-terminal nucleotide of the primer. In some embodiments, A comprises a region that is identical to a universal forward primer and optionally a probe binding domain.

In another aspect, the disclosure provides methods of detecting a variation in a target DNA sequence, the methods comprising: (a) providing a reaction mixture comprising (i) an oligonucleotide primer having a cleavage domain positioned 5' of a blocking group, the blocking group linked at or near the end of the 3'-end of the oligonucleotide primer wherein the blocking group prevents primer extension and/or inhibits the primer from serving as a template for DNA synthesis, (ii) a sample nucleic acid that may or may not have the target sequence, and where the target sequence may or may not have the variation, (iii) a cleaving enzyme, and (iv) a polymerase; (b) hybridizing the primer to the target DNA sequence to form a double-stranded substrate; (c) cleaving the hybridized primer, if the primer is complementary at the variation, with the cleaving enzyme at a point within or adjacent to the cleavage domain to remove the blocking group from the primer; and (d) extending the primer with the polymerase.

In some embodiments, the cleaving enzyme is a hot start cleaving enzyme comprising at least one amino acid substitution as compared to its wild type, which is thermostable and has reduced activity at lower temperatures, and which exhibits enhanced mismatch discrimination during rhPCR as compared to its wild type. In some embodiments, the hot start cleaving enzyme is *Pyrococcus abyssi* RNase H2 comprising (a) a G12A amino acid substitution; (b) a P13T amino acid substitution; (c) a G169A amino acid substitution; or (d) a combination thereof. In some embodiments, the cleaving enzyme is chemically modified. In some embodiments, the cleaving enzyme is reversibly inactivated through interaction with an antibody at lower temperatures. In some embodiments, the cleavage domain comprises at least one RNA base. In some embodiments, the cleavage domain is located 3' of the position of variation. In some embodiments, the cleavage domain is located 5' of the position of variation. In some embodiments, the cleavage domain overlaps with the position of variation. In some embodiments, the cleavage domain comprises one or more 2'-modified nucleosides, and the cleaving enzyme cleaves between the position complementary to the variation and the one or more modified nucleosides. In some embodiments, the one or more modified nucleosides are 2'-fluoronucleosides. In some embodiments, the polymerase is a high-discrimination polymerase. In some embodiments, the polymerase is a mutant H784Q Taq polymerase. In some embodiments, the mutant H784Q Taq polymerase is reversibly inactivated via chemical, aptamer or antibody modification. In some embodiments, the primer contains a 5' tail sequence that comprises a universal primer sequence and optionally a universal probe sequence, wherein the tail is non-complementary to the target DNA sequence. In some embodiments, the target DNA sequence is a sample that has been treated with a gene editing enzyme. In some embodiments, the target DNA sequence is a sample that has been treated with a CRISPR enzyme. In some embodiments, the target DNA sequence is a sample that has been treated with a Cas9 or Cpfl enzyme.

In another aspect, the disclosure provides methods of target enrichment comprising: (a) providing a reaction mixture comprising (i) a first oligonucleotide primer having a tail domain that is not complementary to a target sequence, the tail domain comprising a first universal primer sequence; a cleavage domain positioned 5' of a blocking group, the blocking group linked at or near the end of the 3'-end of the first oligonucleotide primer wherein the blocking group prevents primer extension and/or inhibits the first primer from serving as a template for DNA synthesis, (ii) a sample nucleic acid that may or may not have the target sequence, (iii) a cleaving enzyme, and (iv) a polymerase; (b) hybridizing the first primer to the target DNA sequence to form a double-stranded substrate; (c) cleaving the hybridized first primer, if the first primer is complementary to the target, with the cleaving enzyme at a point within or adjacent to the cleavage domain to remove the blocking group from the first primer; and (d)extending the first primer with the polymerase.

In some embodiments, the methods further comprise a second primer in reverse orientation to support priming and extension of the first primer extension product. In some embodiments, the second primer further comprises a tail domain comprising a second universal primer sequence. In some embodiments, steps (b) - (d) are performed 1-10 times. In some embodiments, the methods further comprise removing unextended primers from the reaction and hybridizing universal primers to the extension product to form a second extension product. In some embodiments, the universal primers further comprise tailed sequences for addition of adapter sequences to the second extension product. In some embodiments, sequencing is performed on the second extension product to determine the sequence of the target. In some embodiments, the target DNA sequence is a sample that has been treated with a gene editing enzyme. In some embodiments, the target DNA sequence is a sample that has been treated with a CRISPR enzyme. In some embodiments, the target DNA sequence is a sample that has been treated with a Cas9 or Cpfl enzyme. In some embodiments, the cleaving enzyme is a hot start cleaving enzyme comprising at least one amino acid substitution as compared to its wild type, which is thermostable and has reduced activity at lower temperatures, and which exhibits enhanced mismatch discrimination during rhPCR as compared to its wild type. In some embodiments, the hot start cleaving enzyme is *Pyrococcus abyssi* RNase H2 comprising (a) a G12A amino acid substitution; (b) a P13T amino acid substitution; or (c) a G169A amino acid substitution; or (d) a combination thereof. **In** some embodiments, the cleaving enzyme is chemically modified. **In** some embodiments, the cleaving enzyme is a hot start cleaving enzyme that is reversibly inactivated through interaction with an antibody at lower temperatures. **In** some embodiments, the cleaving domain comprises at least one RNA base. **In** some embodiments, the cleavage domain is located 3' of the position of variation. **In** some embodiments, the cleavage domain is located 5' of the position of variation. **In** some embodiments, the cleavage domain overlaps with the position of variation. **In** some embodiments, the cleaving domain comprises one or more 2'-modified nucleosides, and the cleaving enzyme cleaves between the position complementary to the variation and the one or more modified nucleosides. **In** some embodiments, the one or more modified nucleosides are 2'-fluoronucleosides. In some embodiments, the polymerase is a high-discrimination polymerase. In some embodiments, the polymerase is a mutant H784Q Taq polymerase. In some embodiments, the mutant H784Q Taq polymerase is reversibly inactivated via chemical, aptamer or antibody modification.

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention.

"Complement" or "complementary" as used herein means a nucleic acid, and can mean Watson-Crick (e.g., A-T/U and C-G) or Hoogsteen base pairing between nucleotides or nucleotide analogs of nucleic acid molecules.

"Fluorophore" or "fluorescent label" refers to compounds with a fluorescent emission maximum between about 350 and 900 nm.

"Hybridization" as used herein, refers to the formation of a duplex structure by two single-stranded nucleic acids due to complementary base pairing. Hybridization can occur between fully complementary nucleic acid strands or between "substantially complementary" nucleic acid strands that contain minor regions of mismatch. "Identical" sequences refers to sequences of the exact same sequence or sequences similar enough to act in the same manner for the purpose of signal generation or hybridizing to complementary nucleic acid sequences. "Primer dimers" refers to the hybridization of two oligonucleotide primers. "Stringent hybridization conditions" as used herein means conditions under which hybridization of fully complementary nucleic acid strands is strongly preferred. Under stringent hybridization conditions, a first nucleic acid sequence (for example, a primer) will hybridize to a second nucleic acid sequence (for example, a target sequence), such as in a complex mixture of nucleic acids. Stringent conditions are sequence-dependent and will be different in different circumstances. Stringent conditions can be selected to be about 5-10°C lower than the thermal melting point (Tm) for the specific sequence at a defined ionic strength pH. The Tm can be the temperature (under defined ionic strength, pH, and nucleic concentration) at which 50% of an oligonucleotide complementary to a target hybridize to the target sequence at equilibrium (as the target sequences are present in excess, at Tm, 50% of the probes are occupied at equilibrium). Stringent conditions can be those in which the salt concentration is less than about 1.0 M sodium ion, such as about 0.01-1.0 M sodium ion concentration (or other salts) at pH 7.0 to 8.3 and the temperature is at least about 30°C for short probes (e.g., about 10-50 nucleotides) and at least about 60°C for long probes (e.g., greater than about 50 nucleotides). Stringent conditions can also be achieved with the addition of destabilizing agents such as formamide. For selective or specific hybridization, a positive signal can be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C.

The terms "nucleic acid," "oligonucleotide," or "polynucleotide," as used herein, refer to at least two nucleotides covalently linked together. The depiction of a single strand also defines the sequence of the complementary strand. Thus, a nucleic acid also encompasses the complementary strand of a depicted single strand. Many variants of a nucleic acid can be used for the same purpose as a given nucleic acid. Thus, a nucleic acid also encompasses substantially identical nucleic acids and complements thereof. A single strand provides a probe that can hybridize to a target sequence under stringent hybridization conditions. Thus, a nucleic acid also encompasses a probe that hybridizes under stringent hybridization conditions.

Nucleic acids can be single stranded or double stranded, or can contain portions of both double stranded and single stranded sequences. The nucleic acid can be DNA, both genomic and cDNA, RNA, or a hybrid, where the nucleic acid can contain combinations of deoxyribo- and ribonucleotides, and combinations of bases including uracil, adenine, thymine, cytosine, guanine, inosine, xanthine hypoxanthine, isocytosine and isoguanine. Nucleic acids can be obtained by chemical synthesis methods or by recombinant methods. A particular nucleic acid sequence can encompass conservatively modified variants thereof (e.g., codon substitutions), alleles, orthologs, single nucleotide polymorphisms (SNPs), and complementary sequences as well as the sequence explicitly indicated.

"Polymerase Chain Reaction (PCR)" refers to the enzymatic reaction in which DNA fragments are synthesized and amplified from a substrate DNA in vitro. The reaction typically involves the use of two synthetic oligonucleotide primers, which are complementary to nucleotide sequences in the substrate DNA which are separated by a short distance of a few hundred to a few thousand base pairs, and the use of a thermostable DNA polymerase. The chain reaction consists of a series of 10 to 40 cycles. In each cycle, the substrate DNA is first denatured at high temperature. After cooling down, synthetic primers which are present in vast excess, hybridize to the substrate DNA to form double-stranded structures along complementary nucleotide sequences. The primer-substrate DNA complexes will then serve as initiation sites for a DNA synthesis reaction catalyzed by a DNA polymerase, resulting in the synthesis of a new DNA strand complementary to the substrate DNA strand. The synthesis process is repeated with each additional cycle, creating an amplified product of the substrate DNA.

"Primer," as used herein, refers to an oligonucleotide capable of acting as a point of initiation for DNA synthesis under suitable conditions. Suitable conditions include those in which hybridization of the oligonucleotide to a template nucleic acid occurs, and synthesis or amplification of the target sequence occurs, in the presence of four different nucleoside triphosphates and an agent for extension (e.g., a DNA polymerase) in an appropriate buffer and at a suitable temperature.

"Probe" and "fluorescent generation probe" are synonymous and refer to either a) a sequence-specific oligonucleotide having an attached fluorophore and/or a quencher, and optionally a minor groove binder or b) a DNA binding reagent, such as, but not limited to, SYBR^{®} Green dye.

"Quencher" refers to a molecule or part of a compound, which is capable of reducing the emission from a fluorescent donor when attached to or in proximity to the donor. Quenching may occur by any of several mechanisms including fluorescence resonance energy transfer, photo-induced electron transfer, paramagnetic enhancement of intersystem crossing, Dexter exchange coupling, and exciton coupling such as the formation of dark complexes.

The term "RNase H PCR (rhPCR)" refers to a PCR reaction which utilizes "blocked" oligonucleotide primers and an RNase H enzyme. "Blocked" primers contain at least one chemical moiety (such as, but not limited to, a ribonucleic acid residue) bound to the primer or other oligonucleotide, such that hybridization of the blocked primer to the template nucleic acid occurs, without amplification of the nucleic acid by the DNA polymerase. Once the blocked primer hybridizes to the template or target nucleic acid, the chemical moiety is removed by cleavage by an RNase H enzyme, which is activated at a high temperature (e.g., 50°C or greater). Following RNase H cleavage, amplification of the target DNA can occur.

The term "discrimination domain" can be the same or different as the cleavage domain. The discrimination domain is the position of the potential mutation site, and the enzyme will only cleave at the cleavage site if the criteria at the discrimination domain are met. For example, in one embodiment RNase H2 will cleave or not cleave a double-stranded target at the RNA residue (cleavage domain), depending on whether a mutation exists at the discrimination domain.

In one embodiment, the 3' end of a blocked primer can comprise the moiety rDDDDMx, wherein relative to the target nucleic acid sequence, "r" is an RNA residue, "D" is a complementary DNA residue, "M" is a mismatched DNA residue, and "x" is a C3 spacer. A C3 spacer is a short 3-carbon chain attached to the terminal 3' hydroxyl group of the oligonucleotide, which further inhibits the DNA polymerase from binding before cleavage of the RNA residue.

The methods described herein can be performed using any suitable RNase H enzyme that is derived or obtained from any organism. Typically, RNase H-dependent PCR reactions are performed using an RNase H enzyme obtained or derived from the hyperthermophilic archaeon *Pyrococcus abyssi* (*P*.*a*.), such as RNase H2. Thus, in one embodiment, the RNase H enzyme employed in the methods described herein desirably is obtained or derived from *Pyrococcus abyssi,* preferably an RNase H2 obtained or derived from *Pyrococcus abyssi.* In other embodiments, the RNase H enzyme employed in the methods described herein can be obtained or derived from other species, for example, *Pyrococcus furiosis, Pyrococcus horikoshii, Thermococcus kodakarensis,* or *Thermococcus litoralis.*

The following examples further illustrate the invention but should not be construed as in any way limiting its scope.

### EXAMPLE 1

This example demonstrates an enhanced rhPCR assay that utilizes a highly discriminatory DNA polymerase and RNase H2 for discrimination

To demonstrate the utility of these new assay designs, rhPrimers and standard allele-specific primers were designed against rs113488022, the V600E mutation in the human BRAF gene. These primers were tested in PCR and rhPCR with WT or H784Q mutant Taq polymerase. Primers utilized in these assays were as shown in Table 1 (SEQ ID NOs: 1-7).

**Table 1: Sequence of oligonucleotides employed in SNP discrimination assay described in Example 1.**

| Name | Sequence | SEQ ID NO. |
|---|---|---|
| Forward non-discriminating primer | GCTGTGATTTTGGTCTAGCTACAG | SEQ ID NO. 1 |
| Forward Allele 1 ASP1 ASPCR primer | GCTGTGATTTTGGTCTAGCTACAGT | SEQ ID NO. 2 |
| Forward Allele 2 ASP2 ASPCR primer | GCTGTGATTTTGGTCTAGCTACAGA | SEQ ID NO. 3 |
| Probe | FAM-TCCCATCAG-ZEN- TTTGAACAGTTGTCTGGA-IBFQ | SEQ ID NO. 4 |
| rs113488022 Allele 1 Forward ASP1 rhPrimer | GCTGTGATTTTGGTCTAGCTACAGTgAA ATG-x | SEQ ID NO. 5 |
| rs113488022 Allele 2 Forward ASP2 rhPrimer | GCTGTGATTTTGGTCTAGCTACAGAgAA ATG-x | SEQ ID NO. 6 |
| Reverse rhPrimer | GCCCTCAATTCTTACCATCCACAAAaTG GAA-x | SEQ ID NO. 7 |

| | | |
|---|---|---|
| Nucleic acid sequences are shown 5'-3'. DNA is uppercase, RNA is lowercase. Location of potential mismatch is underlined. ZEN = internal ZEN^{™} quencher (IDT, Coralville, IA), FAM = 6-carboxyfluorescein, IBFQ = Iowa Black^{®} FQ (fluorescence quencher, IDT, Coralville, IA), and x = C3 propanediol spacer block | | |

10 µL reaction volumes were used in these assays. To perform the reaction, 5 µL of 2x Integrated DNA Technologies (IDT) (Coralville, IA) rhPCR genotyping master mix (containing dNTPs, H784Q mutant or WT Taq DNA polymerase, stabilizers, and MgCl₂) was combined with 200 nM (2 pmol) of either of the allelic primers. 200 nM (2 pmol) of the probe, as well as 200 nM (5 pmol) of the reverse primer were also added. Additionally, 2.5 mU (5.25 fmol/0.53 nM) of P.a. RNase H2 and 1000 copies of synthetic gBlock^{™} (Integrated DNA Technologies, Coralville, IA) template (1000 copies Allele 1, 500 copies allele 1+500 copies allele 2 (heterozygote), or 1000 copies Allele 2 (for gBlock^{™} sequences, see Table 2, SEQ ID NOs: 8-9) were added to the reaction mix. The reaction was thermocycled on a Bio-Rad^{™} CFX384^{™} Real-time system. Cycling conditions were as follows: 953:00 - (950:10-650:30) x 65 cycles. Each reaction was performed in triplicate.

**Table 2: Synthetic gBlock templates for Example 1 assay**

| Name | Sequence | SEQ ID NO. |
|---|---|---|
| rs113488022 gBlock Template 1 | AAAAAATAAGAACACTGATTTTTGTGAAT ACTGGGAACTATGAAAATACTATAGTTGA GACCTTCAATGACTTTCTAGTAACTCAGCA GCATCTCAGGGCCAAAAATTTAATCAGTG GAAAAATAGCCTCAATTCTTACCATCCAC AAAATGGATCCAGACAACTGTTCAAACTG ATGGGACCCACTCCATCGAGATTTCACTGT AGCTAGACCAAAATCACCTATTTTTACTGT GAGGTCTTCATGAAGAAATATATCTGAGG TGTAGTAAGTAAAGGAAAACAGTAGATCT CATTTTCCTATCAGAGCAAGCATTATGAAG AGTTTAGGTAAGAGATCTAATTTCTATAAT TCTGTAATATAATATTCTTTAAAACATAGT ACTTCATCTTTCCTCTTA | SEQ ID NO. 8 |
| rs113488022 gBlock Template 2 | AAAAAATAAGAACACTGATTTTTGTGAAT ACTGGGAACTATGAAAATACTATAGTTGA GACCTTCAATGACTTTCTAGTAACTCAGCA GCATCTCAGGGCCAAAAATTTAATCAGTG GAAAAATAGCCTCAATTCTTACCATCCAC AAAATGGATCCAGACAACTGTTCAAACTG ATGGGACCCACTCCATCGAGATTTCTCTGT AGCTAGACCAAAATCACCTATTTTTACTGT GAGGTCTTCATGAAGAAATATATCTGAGG TGTAGTAAGTAAAGGAAAACAGTAGATCT | SEQ **ID** NO. 9 |
| | CATTTTCCTATCAGAGCAAGCATTATGAAG AGTTTAGGTAAGAGATCTAATTTCTATAAT TCTGTAATATAATATTCTTTAAAACATAGT ACTTCATCTTTCCTCTTA | |

| | | |
|---|---|---|
| Nucleic acid sequences are shown 5'-3'. Location of SNPs are shown bold and underlined. | | |

Cq Results of the experiment are shown in Table 3. This data shows that the mismatch discrimination of the assay system increases with rhPCR over ASPCR with WT Taq polymerase, and that the discrimination is enhanced by the use of the H784Q Taq polymerase.

**Table 3: Resulting Cq values**

| | WT Taq | | | | H784Q | | | |
|---|---|---|---|---|---|---|---|---|
| | Allele 1 | Het | Allele 2 | NTC | Allele 1 | Het | Allele 2 | NTC |
| Non discrmin | 29.3 | 29.3 | 29.4 | >65 | 30.6 | 30.6 | 30.8 | >65 |
| ASP1 ASPCR | 30.2 | 30.2 | 31.4 | >65 | 29.2 | 32.5 | 40.3 | >65 |
| ASP2 ASPCR | 36.7 | 30.5 | 29.4 | >65 | 44.2 | 31.7 | 30.8 | >65 |
| ASP1 rhPCR | 30.9 | 32.1 | 38.2 | >65 | 31.9 | 31.4 | 49.2 | >65 |
| ASP2 rhPCR | 39.3 | 31.0 | 30.8 | >65 | 43.4 | 33.9 | 32.5 | >65 |

| | | | | | | | | |
|---|---|---|---|---|---|---|---|---|
| All numbers in this table represent Cq values obtained from the CFX384^{™} instrument (Bio-Rad^{™}, Hercules, CA). | | | | | | | | |

### EXAMPLE 2

The following example demonstrates an enhanced rhPCR assay that utilizes a highly discriminatory DNA polymerase and RNase H2 for discrimination.

In order to demonstrate that this new assay design could function, rhPrimers and standard allele-specific primers were designed against rs113488022, the V600E mutation in the human BRAF gene. These primers were tested in PCR and rhPCR with H784Q mutant Taq polymerase. Primers utilized in these assays were as shown in Table 4 (SEQ ID NOs: 1, 4 and 10-12).

**Table 4: Sequence of oligonucleotides employed in SNP discrimination assay described in Example 2**

| Name | Sequence | SEQ ID NO. |
|---|---|---|
| Forward non-discrimin primer | GCTGTGATTTTGGTCTAGCTACAG | SEQ ID NO. 1 |
| Probe | FAM-TCCCATCAG-ZEN- TTTGAACAGTTGTCTGGA-IBFQ | SEQ ID NO. 4 |
| rs113488022 Allele 1 Forward dxxd rhPrimer | GCTGTGATTTTGGTCTAGCTACAGTg AxxTG | SEQ ID NO. 10 |
| rs113488022 Allele 2 Forward dxxd rhPrimer | GCTGTGATTTTGGTCTAGCTACAGAg AxxTG | SEQ ID NO. 11 |
| Reverse rhPrimer | GCCCTCAATTCTTACCATCCACAAAa TGGAA-x | SEQ ID NO. 12 |

| | | |
|---|---|---|
| Nucleic acid sequences are shown 5'-3'. DNA is uppercase, RNA is lowercase. Location of potential mismatch is underlined. ZEN = internal Zen^{™} fluorescent quencher (IDT, Coralville, IA). FAM = 6-carboxyfluorescein, IBFQ = Iowa Black FQ (fluorescence quencher), and x = C3 propanediol spacer. | | |

10 µL reaction volumes were used in these assays. To perform the reaction, 5 µL of 2x Integrated DNA Technologies (IDT) (Coralville, IA) rhPCR genotyping master mix (containing dNTPs, H784Q mutant DNA polymerase, stabilizers, and MgCl₂) was combined with 200 nM (2 pmol) of either of the allelic primers. 200 nM (2 pmol) of the probe, as well as 200 nM (5 pmol) of the reverse primer were also added. Additionally, 7.5 mU (15.75 fmol/1.58 nM), 50 mU (105 fmol/10.5 nM) or 200 mU (420 fmol/42 nM) of *P.a.* RNase H2 and 5e4 copies of synthetic gBlock^{™} (Integrated DNA Technologies, Coralville, IA) template (1e5 copies Allele 1, 5e4 copies allele 1+5e4 copies allele 2 (heterozygote), or 1e5 copies Allele 2 (for gBlock^{™} sequences, see Table 2, SEQ ID NOs: 8-9) were added to the reaction mix. The reaction was thermocycled on a Bio-Rad^{™} CFX384^{™} Real-time system. Cycling conditions were as follows: 95^{3:00} - (95^{0:10}-65^{0:30}) x 65 cycles. Each reaction was performed in triplicate.

Cq Results of the experiment are shown in Table 5. This data shows that the mismatch discrimination of the assay system increases with rhPCR over ASPCR with WT Taq polymerase, and that the discrimination is enhanced by the use of the H784Q Taq polymerase.

**Table 5: Resulting Cq values**

| Averages | | Allele 1 | Het | Allele 2 | NTC | ΔCq |
|---|---|---|---|---|---|---|
| Unblocked | 7.5 mU | 21.9 | 22.3 | 22.1 | >75 | |
| | 50 mU | 22.7 | 22.5 | 22.7 | >75 | |
| | 200 mU | 21.8 | 21.8 | 21.9 | >75 | |
| AgAxxTG | 7.5 mU | 43.7 | 25.6 | 24.6 | >75 | 19.1 |
| | 50 mU | 50.3 | 24.5 | 23.5 | >75 | 26.8 |
| | 200 mU | 48.5 | 25.2 | 24.1 | >75 | 24.4 |
| TgAxxTG | 7.5 mU | 25.1 | 26.3 | 42.5 | >75 | 17.4 |
| | 50 mU | 24.2 | 25.4 | 41.0 | >75 | 16.9 |
| | 200 mU | 22.9 | 23.8 | 37.2 | >75 | 14.3 |

| | | | | | | |
|---|---|---|---|---|---|---|
| All numbers in this table represent Cq values obtained from the CFX384^{™} instrument (Bio-Rad^{™}, Hercules, CA). | | | | | | |

The delta Cq values were significantly higher than the ones obtained with the Gen 1 versions of these primers, indicating that there is an advantage to this primer design, as seen before in rhPCR.

### EXAMPLE 3

The following example illustrates the heightened reliability of universal assays using a DNA polymerase with a high mismatch discrimination.

To demonstrate that the disclosed assays can function in a universal format and that they are significantly improved with a polymerase with high mismatch discrimination, "universal" assay primers were designed against rs351855, the G338R mutation in the human FGFR4 gene. This "universal" assay design has numerous advantages, including the ability to multiplex the allele-specific rhPrimers and obtain multiple-color readouts. Primers utilized in this assay were as shown in Table 6 (SEQ ID NOs: 13-18).

**Table 6: Sequences of oligonucleotides employed in "universal" SNP discrimination assay**

| Name | Sequence | SEQ ID NO. |
|---|---|---|
| Universal Forward primer | CGCCGCGTATAGTCCCGCGTAAA | SEQ ID NO. 13 |
| Probe 1 (FAM) | FAM-C+CATC+A+C+CGTG+CT-IBFQ | SEQ ID NO. 14 |
| Probe 2 (HEX) | HEX-CAATC+C+C+CGAG+CT-IBFQ | SEQ ID NO. 15 |
| rs351855 Allele 1 Forward primer | GCCCATGTCCCAGCGAACCATCACCGTGCTA GCCCTCGATACAGCCCgGCCAC-x | SEQ ID NO. 16 |
| rs351855 Allele 2 Forward primer | GCCCATGTCCCAGCGAACAATCCCCGAGCTG CCCTCGATACAGCCTgGCCAC-x | SEQ ID NO. 17 |
| Reverse primer | GCGGCCAGGTATACGGACATcATCCA-x | SEQ ID NO. 18 |

| | | |
|---|---|---|
| Nucleic acid sequences are shown 5'-3'. DNA is uppercase, RNA is lowercase. Location of potential mismatch is underlined. LNA residues are designated with a +. FAM = 6-carboxyfluorescein, HEX = *6-carboxy-2',4,4',5', 7, 7'-hexachlorofluorescein,* IBFQ = Iowa Black FQ (fluorescence quencher), and x = C3 propanediol spacer block. | | |

10 µL reaction volumes were used in these assays. To perform the reaction, 5 µL of 2x Integrated DNA Technologies (IDT) (Coralville, IA) rhPCR genotyping master mix (containing dNTPs, mutant or WT Taq DNA polymerase, stabilizers, and MgCl₂) was combined with 50 nM (500 fmol) of each of the two allelic primers. 250 nM (2.5 pmol) of each of the two probes, as well as 500 nM (5 pmol) of the Universal Forward primer and 500 nM (5 pmol) of the reverse primer were also added. Additionally, 2.5 mU (5.25 fmol/0.53 nM) of *P.a.* RNase H2 and 1000 copies of synthetic gBlock^{™} (Integrated DNA Technologies, Coralville, IA) template (1000 copies Allele 1, 500 copies allele 1+500 copies allele 2 (heterozygote), or 1000 copies Allele 2 (for gBlock^{™} sequences, see Table 7, SEQ ID NOs: 19-20) were added to the reaction mix. The reaction was thermocycled on a Bio-Rad^{™} CFX384^{™} Real-time system. Cycling conditions were as follows: 95^{3:00} - (95^{0:10}-60^{0:30}) x 3 cycles - (95^{0:10}-65^{0:30}) x 65 cycles. Each reaction was performed in triplicate. Fluorescence reads were taken after a total of 50 cycles were completed. Fluorescence values were plotted on the FAM and HEX axis, and results are shown in Figure 2a and 2b.

**Table 7: Synthetic gBlock templates for Example 3**

| Name | Sequence | SEQ ID NO. |
|---|---|---|
| rs351855 gBlock Template 1 | GTTGGGAGCTGGGAGGGACTGAGTTAGGG TGCACGGGGCGGCCAGTCTCACCACTGAC CAGTTTGTCTGTCTGTGTGTGTCCATGTGC GAGGGCAGAGGAGGACCCCACATGGACC | SEQ ID NO. 19 |
| | GCAGCAGCGCCCGAGGCCAGGTATACGGA CATCATCCTGTACGCGTCGGGCTCCCTGGC CTTGGCTGTGCTCCTGCTGCTGGCCGGGCT GTATCGAGGGCAGGCGCTCCACGGCCGGC ACCCCCGCCCGCCCGCCACTGTGCAGAAG CTCTCCCGCTTCCCTCTGGCCCGACAGGTA CTGGGCGCATCCCCCACCTCACATGTGAC AGCCTGACTCCAGCAGGCAGAACCAAGTC TCCCACTTTGCAGTTCTCCCTGGAGTCAGG CTCTTCCGGCAAGTCAAGCT | |
| rs351855 gBlock Template 2 | GTTGGGAGCTGGGAGGGACTGAGTTAGGG TGCACGGGGCGGCCAGTCTCACCACTGAC CAGTTTGTCTGTCTGTGTGTGTCCATGTGC GAGGGCAGAGGAGGACCCCACATGGACC GCAGCAGCGCCCGAGGCCAGGTATACGGA CATCATCCTGTACGCGTCGGGCTCCCTGGC CTTGGCTGTGCTCCTGCTGCTGGCCAGGCT GTATCGAGGGCAGGCGCTCCACGGCCGGC ACCCCCGCCCGCCCGCCACTGTGCAGAAG CTCTCCCGCTTCCCTCTGGCCCGACAGGTA CTGGGCGCATCCCCCACCTCACATGTGAC AGCCTGACTCCAGCAGGCAGAACCAAGTC TCCCACTTTGCAGTTCTCCCTGGAGTCAGG CTCTTCCGGCAAGTCAAGCT | SEQ ID NO. 20 |

| | | |
|---|---|---|
| Nucleic acid sequences are shown 5'-3'. Location of SNPs are shown bold and underlined. | | |

The results illustrate that the mismatch discrimination between homozygotes is sufficient with both polymerases, although the resulting data using the WT Taq demonstrate that it is more difficult to make an allelic call. Importantly, however, the WT Taq polymerase cannot efficiently discriminate heterozygotes from homozygotes, and places them too close to the allele 1 and 2 signals (Figure 2a). In contrast, the signal from the heterozygotes in the assays utilizing the mutant Taq polymerase are easily distinguishable from the homozygotes (Figure 2b).

The importance of the mutant Taq can be further understood when examining the Cq values from this example (Table 8). The data show that not only does the H784Q Taq mutant increase mismatch discrimination dramatically, but the Cqs of the NTCs decrease from the low-to-mid 50s, to greater than the number tested in the assay (>65). From this experiment, it is shown that allele identity can be determined from Cq values as well as end-point fluorescence.

**Table 8: Cq and delta Cq data for the experiment described in Example 3**

| Template | WT Taq | | | H784Q | | |
|---|---|---|---|---|---|---|
| | FAM | HEX | Delta Cq | FAM | HEX | Delta Cq |
| Allele 1 | 32.9 | 31.3 | -1.6 | 37.5 | 56.8 | 19.3 |
| Allele 1 | 31.9 | 31.1 | -0.8 | 36.2 | 51.4 | 15.2 |
| Allele 1 | 31.8 | 31.0 | -0.9 | 36.6 | 54.3 | 17.8 |
| Heterozygote | 33.0 | 29.4 | -3.5 | 38.7 | 37.8 | -0.9 |
| Heterozygote | 32.8 | 29.7 | -3.1 | 38.7 | 38.2 | -0.5 |
| Heterozygote | 33.2 | 30.0 | -3.3 | 39.9 | 39.1 | -0.8 |
| Allele 2 | 35.1 | 29.1 | 6.0 | 50.6 | 36.5 | 14.1 |
| Allele 2 | 34.7 | 29.3 | 5.4 | 52.1 | 36.6 | 15.5 |
| Allele 2 | 34.5 | 29.0 | 5.5 | 50.7 | 36.1 | 14.6 |
| NTC | 51.8 | 56.1 | --- | >65 | >65 | --- |
| NTC | 52.8 | 56.1 | --- | >65 | >65 | --- |
| NTC | 52.1 | 50.7 | --- | >65 | >65 | --- |

| | | | | | | |
|---|---|---|---|---|---|---|
| All numbers in this table represent Cq and delta Cq values values obtained from the CFX384 instrument (Bio-Rad^{™}, Hercules, CA). | | | | | | |

### EXAMPLE 4

The following example illustrates the detection of rare allelic variants with the assay designs of the present invention. To demonstrate the utility of these new assay designs to detect rare allelic variants, previously described second generation rhPrimers (rdxxdm) were utilized against rs113488022, the V600E mutation in the human BRAF gene (see Table 4; SEQ ID NOs: 1,4 and 10-12).

10 µL reaction volumes were used in these assays. To perform the reaction, 5 µL of 2x Integrated DNA Technologies (IDT) (Coralville, IA) rhPCR genotyping master mix (containing dNTPs, H784Q mutant or WT Taq DNA polymerase, stabilizers, and MgCl₂) was combined with 200 nM (2 pmol) of either of the allelic primers, or the non-discriminatory forward primer. 200 nM (2 pmol) of the probe, as well as 200 nM (5 pmol) of the reverse primer were also added. Additionally, 50 mU (105 fmol/10.5 nM) of *P.a.* RNase H2 and 50,000 copies of synthetic gBlock^{™} (Integrated DNA Technologies, Coralville, IA) match template, was combined with either 0, 50, or 500 copies of the opposite allele (for gBlock^{™} sequences, see Table 6, SEQ ID NOs: 16-17) were added to the reaction mix. The reaction was thermocycled on a Bio-Rad^{™} CFX384^{™} Real-time system. Cycling conditions were as follows: 95^{3:00} - (95^{0:10}-60^{0:30}) x 65 cycles. Each reaction was performed in triplicate.

Data for the WT polymerase is shown in Table 9, and for the H784Q mutant Taq polymerase in Table 10. One of the advantages of this system for rare allele detection over "conventional" rhPCR is the ability to utilize a single amount of RNase H2 for both alleles. This advantage halves the potential requirement for determining the enzyme amount required for cleavage.

**Table 9: Average Cq and delta Cq values for the rare allele experiment with the WT Taq polymerase described in Example 4.**

| | Back-ground | 50,000 | 50,000 | 50,000 | 0 | 0 | 0 |
|---|---|---|---|---|---|---|---|
| | Target | 500 | 50 | 0 | 500 | 50 | 0 |
| SEQ ID No. 1 | Non-discrimin | 22.9 | 23.1 | 22.8 | 30.4 | 34.2 | >65 |
| SEQ ID NO. 10 | ...TgAxxTG | 31.6 | 34.5 | 36.1 | 31.6 | 36.0 | >65 |
| SEQ ID NO. 11 | ... AgAxxTG | 29.1 | 29.1 | 29.9 | 30.8 | 34.4 | >65 |

| | | | | | | | |
|---|---|---|---|---|---|---|---|
| All numbers in this table represent Cq and delta Cq values values obtained from the CFX384 instrument (Bio-Rad^{™}, Hercules, CA). DNA is uppercase, RNA is lowercase. Location of potential mismatch is underlined. x = internal C3 propanediol spacer block. | | | | | | | |

**Table 10: Average Cq and delta Cq values for the rare allele experiment with the H784Q mutant Taq polymerase described in Example 4.**

| | Back-ground | 50,000 | 50,000 | 50,000 | 0 | 0 | 0 |
|---|---|---|---|---|---|---|---|
| | Target | 500 | 50 | 0 | 500 | 50 | 0 |
| SEQ ID No. 1 | Non-discrimin | 22.7 | 23.2 | 23.2 | 31.5 | 34.2 | >65 |
| SEQ ID NO. 10 | ... TgAxxTG | 33.8 | 36.6 | 47.3 | 33.1 | 36.4 | >65 |
| SEQ ID NO. 11 | ... AgAxxTG | 32.1 | 35.2 | 38.8 | 32.0 | 35.5 | >65 |

| | | | | | | | |
|---|---|---|---|---|---|---|---|
| All numbers in this table represent Cq and delta Cq values obtained from the CFX384 instrument (Bio-Rad^{™}, Hercules, CA). DNA is uppercase, RNA is lowercase. Location of potential mismatch is underlined. x = internal C3 propanediol spacer block. | | | | | | | |

The data clearly shows that the H784Q DNA polymerase allows for detection of 50 copies of target in a 50,000 copies of background DNA (a 1:1000 discrimination level) for the mutant A allele of rs113488022, with a delta Cq of over 11 cycles. While only slightly more than 3 cycles was observed for the T allele in this assay, this was a significant improvement over the WT Taq polymerase, which did not show any discrimination for the T allele, and only a delta of 3 cycles for the A allele.

### EXAMPLE 5

This example demonstrates successful allelic discrimination with the use of a universal rhPCR genotyping assay and Integrated DNA Technologies (IDT) (Coralville, IA) rhPCR genotyping master mix, and the robust stability of the reaction components. To demonstrate the robust nature of the assay design and mixture components, universal primers were designed against rs4657751, a SNP located on the human Chromosome 1 (See Table 11, SEQ ID NOs: 14, 21-25).

Identical universal rhPCR genotyping reactions were set up in two white Hard-Shell^{®} 384-well skirted PCR plates (Bio-Rad, Hercules, CA) on the Bio-Rad CFX384 Touch^{™} Real-Time PCR Detection System with 10 µL final volume. Each well contained the rhPCR assay primers (150 nM of rs4657751 Allele Specific Primer 1 (SEQ ID NO: 23), 150 nM of rs4657751 Allele Specific Primer 2 (SEQ ID NO: 24), and 500 nM rs4657751 Locus Specific Primer (SEQ ID NO: 25). Reactions contained universal reporter oligos at the following concentrations: 250 nM of universal FAM probe (SEQ ID NO: 14), 450 nM of universal Yakima Yellow^{®} (SEQ ID NO: 22) probe, and 1000 nM of universal forward primer (SEQ ID NO: 21), and 5 µL of 2x Integrated DNA Technologies (IDT) (Coralville, IA) rhPCR genotyping master mix (containing dNTPs, a mutant H784Q *Taq* polymerase (see Behlke, et al. U.S. 2015/0191707), chemically modified *Pyrococcus abyssi* RNase H2 (See Walder et al. UA20130288245A1), stabilizers, and MgCl₂).

gBlocks^{®} Gene Fragments (Integrated DNA Technologies, Inc., Coralville, IA) containing either allele of the rs4657751 SNP were utilized as the source of template DNA (See Table 12, SEQ ID NOs: 26 and 27). Each well contained template representing one of three possible genotypes: allele 1 homozygote (1000 copies rs4657751 Allele 1 gBlock^{®} template (SEQ ID NO: 26)), allele 2 homozygote (1000 copies rs4657751 Allele 2 gBlock^{®} template (SEQ ID NO: 27)), or heterozygote (mix of 500 copies of rs4657751 Allele 1 gBlock^{®} template (SEQ ID NO: 26) and 500 copies of rs4657751 Allele 2 gBlock^{®} template (SEQ ID NO: 27)). Template or water for the no template control (NTC) reactions were added into three replicate wells of two individual plates. The reactions underwent the following cycling protocol: 95°C for 10 minutes, then 45 cycles of 95°C for 10 seconds and 60°C for 45 seconds.

**Table 11: Sequences of oligonucleotides used in Example 5**

| Name | Sequence | SEQ ID NO. |
|---|---|---|
| Universal Forward primer | CGGCCCATGTCCCAGCGAA | SEQ ID NO. 21 |
| Probe 1 (FAM) | FAM-C+CATC+A+C+CGTG+CT-IBFQ | SEQ ID NO. 14 |
| Probe 2 (Yakima Yellow) | Yak-CAATC+C+C+CGAG+CT-IBFQ | SEQ ID NO. 22 |
| rs4657751 Allele 1 Forward primer | GCCCATGTCCCAGCGAACCATCACCGTGC TACTTCCCACACCCTCATATCuTGTTA-x | SEQ ID NO. 23 |
| rs4657751 Allele 2 Forward primer | GCCCATGTCCCAGCGAACAATCCCCGAGC TCTTACTTCCCACACCCTCATATAuTGTTA- x | SEQ ID NO. 24 |
| rs4657751 Reverse primer | GCGCTAAGTAAACATTCCTGATTGCAaCTT AT-x | SEQ ID NO. 25 |

| | | |
|---|---|---|
| Nucleic acid sequences are shown 5'-3'. DNA is uppercase, RNA is lowercase. Location of potential mismatch is underlined. LNA residues are designated with a +. FAM = 6-*carboxyfluorescein,* Yak = Yakima Yellow (3-(5,6,4',7'-tetrachloro-5'-methyl-3',6'-dipivaloylfluorescein-2-yl)), IBFQ = Iowa Black FQ (fluorescence quencher), and x = C3 propanediol spacer block. | | |

**Table 12: Synthetic gBlock^{®} templates used in Example 5.**

| Name | Sequence | SEQ ID NO. |
|---|---|---|
| rs4657751 Allele 1 gBlock template | GATTTTTTTTTTTGGCATTTCTTCTTAGAT TTCTATCTCCTAACATAGGATCACTTATTT GTGAAATTATTTGTATACCTTTTTATGGA GTGATGATGTGATACAAATTCTATCCTTAA GGATATAAGAACATCTTTTCTTTATATTAG GATTTTTCTGGACCCATGAGTTACATGCTT ACTTCCCACACCCTCATATCTTGTTTAAAT TTGTAGAATTAAATTCATAGGTAATTATTT CTGAAACTTCTTCCCTGTGTGAGCAATCTA AATAATTATTACAATGCCTTAAGTTGCAAT CAGGAATGTTTACTTAGCACAGACTTTTTT CCCCACTACTGCACTCAAAGGATAACAGA TATATGGCAAATCTAACCATATTCTTTGTC CTTTGTCCATGTTGCGGAGGGAAGCTCATC AGTGGGGCCACGAGCTGAGTGCGTCCTGT CACTCCACTCCCATGTCCCTTGGGAAGGTC TGAGACTAGGG | SEQ ID NO. 26 |
| rs4657751 Allele 2 gBlock template | GATTTTTTTTTTTTGGCATTTCTTCTTAGAT TTCTATCTCCTAACATAGGATCACTTATTT GTGAAATTATTTGTATACCTTTTTTATGGA GTGATGATGTGATACAAATTCTATCCTTAA GGATATAAGAACATCTTTTCTTTATATTAG GATTTTTCTGGACCCATGAGTTACATGCTT ACTTCCCACACCCTCATATATTGTTTAAAT | SEQ ID NO. 27 |
| | TTGTAGAATTAAATTCATAGGTAATTATTT CTGAAACTTCTTCCCTGTGTGAGCAATCTA AATAATTATTACAATGCCTTAAGTTGCAAT CAGGAATGTTTACTTAGCACAGACTTTTTT CCCCACTACTGCACTCAAAGGATAACAGA TATATGGCAAATCTAACCATATTCTTTGTC CTTTGTCCATGTTGCGGAGGGAAGCTCATC AGTGGGGCCACGAGCTGAGTGCGTCCTGT CACTCCACTCCCATGTCCCTTGGGAAGGTC TGAGACTAGGG | |

| | | |
|---|---|---|
| Nucleic acid sequences are shown 5'-3'. DNA is uppercase. The location of the SNP is underlined. | | |

One reaction plate was cycled immediately (0 hr benchtop hold) and one reaction plate was held at room temperature for 2 days (48 hr benchtop hold) to demonstrate reaction stability over time. Allelic discrimination analysis was performed using Bio-Rad CFX Manager 3.1 software (Bio-Rad, Hercules, CA). FAM and Yakima Yellow fluorophores were detected in each well. For both fluorophores the baseline cycles were set to begin at cycle 10 and end at cycle 25. Fluorescence signal (RFU) in each well at the end of 45 cycles was used to generate an allelic discrimination plot and genotypes were determined with auto-call features of the analysis software. Identical performance was obtained with the immediate run (Figure 3A) and 48 hour hold plate (Figure 3B), demonstrating robust stability of the reaction components. Each sample is assigned the correct genotyping call and samples of the same genotype are tightly clustered together. The heterozygote cluster is separated from both of the homozygous clusters by an approximate 45 degree angle, indicating excellent allelic specificity of the universal rhPCR genotyping assays and master mix.

### EXAMPLE 6

The following example compares the performance of the genotyping methods of the present invention versus traditional 5' nuclease genotyping assay methods (Taqman^{™}).

The rs1799865 SNP in the CCR2 gene was selected, and rhPCR genotyping primers as well as an rs1799865 5' nuclease assay (Thermo-Fisher (Waltham, MA)), were designed and obtained. Sequences for the rs1799865 rhPCR genomic SNP assay are shown in Table 14 (SEQ ID NOs: 14, 21, 22, and 28-30). Thermo-Fisher 5' nuclease primer/probe (Taqman^{™}) sequences are not published, and therefore are not included in this document.

Reactions were performed in 10 µL volumes, containing 10 ng Coriell genomic DNA (Camden, NJ), 250 nM of universal FAM probe (SEQ ID NO: 14), 450 nM of universal Yakima Yellow^{®} (SEQ ID NO: 22) probe, 1000 nM of universal forward primer (SEQ ID NO: 21), 150 nM of the two allele-specific forward primers (SEQ ID NOs: 28 and 29), 500 nM of the reverse primer (SEQ ID NO: 30), and 5 µL of 2x Integrated DNA Technologies (IDT) (Coralville, IA) rhPCR genotyping master mix (containing dNTPs, a mutant H784Q *Taq* polymerase (see Behlke, et al. U.S. 2015/0191707), chemically modified *Pyrococcus abyssi* RNase H2 (See Walder et al. UA20130288245A1), stabilizers, and MgCl₂).

PCR was performed on Life Technologies (Carlsbad, CA) QuantStudio^{™} 7 Flex real-time PCR instrument using the following cycling conditions: 10 mins at 95°C followed by 50 cycles of 95°C for 10 seconds and 60°C for 45 seconds. End-point analysis of each of the plates was performed after 45 cycles with the QuantStudio^{™} Real-Time PCR Software v1.3 (Carlsbad, CA).

**Table 14. Sequences of oligonucleotides used for the rs1799865 genotyping assay in Example 6.**

| Name | Sequence | SEQ ID NO. |
|---|---|---|
| Universal Forward primer | CGGCCCATGTCCCAGCGAA | SEQ ID NO. 21 |
| Probe 1 (FAM) | FAM-C+CATC+A+C+CGTG+CT-IBFQ | SEQ ID NO. 14 |
| Probe 2 (Yakima Yellow) | Yak-CAATC+C+C+CGAG+CT-IBFQ | SEQ ID NO. 22 |
| rs1799865 Allele 1 Forward primer | GCCCATGTCCCAGCGAACCATCACCGTG CTTTCTCTTCTGGACTCCCTATAATaTTGT G-x | SEQ ID NO. 28 |
| rs1799865 Allele 2 Forward primer | GCCCATGTCCCAGCGAACAATCCCCGAG CTTTCTCTTCTGGACTCCCTATAACaTTGT G-x | SEQ ID NO. 29 |
| rs1799865 Reverse primer | GCGGATTGATGCAGCAGTGAgTCATG-x | SEQ ID NO. 30 |

| | | |
|---|---|---|
| Nucleic acid sequences are shown 5'-3'. DNA is uppercase, RNA is lowercase. LNA residues are designated with a +. Location of potential mismatch is underlined. FAM = 6-carboxyfluorescein, Yak = Yakima Yellow (3-(5,6,4',7'-tetrachloro-5'-methyl-3',6'-dipivaloylfluorescein-2-yl)), IBFQ = Iowa Black FQ (fluorescence quencher), and x = C3 propanediol spacer block. | | |

Figures 4A and 4B show a side-by-side comparison of the resulting allelic discrimination plots. The rhPCR Genotyping Assay (FIG. 4B) achieved higher fluorescence signal compared to a traditional 5'-nuclease genotyping assay (FIG. 4A) while showing concordant results. The higher signal and minimal non-specific amplification from NTC in the rhPCR assay allow better cluster separation and accurate genotype calls.

### EXAMPLE 7

The following example illustrates the present methods allowing for detection and analysis of tri-allelic SNP. The rs72558195 SNP is present in the CYP2C8 gene, and has three potential genotypes. This SNP was selected for analysis with the rhPCR genotyping system.

Conventional workflow of interrogating tri-allelic SNP, as illustrated in Figures 5A and 5B, involves running a pair of assays using the same samples, manual calling, and comparing the paired assay result to obtain the true genotype of samples.

To demonstrate that such a system can function with the universal rhPCR genotyping system, reactions were set up in a white Hard-Shell^{®} 384-well skirted PCR plates (Bio-Rad, Hercules, CA) on the Life Technologies (Carlsbad, CA) QuantStudio^{™} 7 Flex real-time PCR with 10 µL final volume. Each well contained the rhPCR assay primers (See Table 16, SEQ ID NOs: 14, 21, 22, 31-33). Specifically, 150 nM of rs72558195 G:A Allele Specific Primer 1 (SEQ ID NO: 31) and 150 nM of rs72558195 G:A Allele Specific Primer 2 (SEQ ID NO: 32), or 150 nM of rs72558195 G:A Allele Specific Primer 1 (SEQ ID NO: 31) and 150 nM of rs72558195 G:C Allele Specific Primer 3 (SEQ ID NO: 33) as well as 500 nM rs72558195 Locus Specific Primer (SEQ ID NO: 34) were included in the reactions.

Reactions contained universal reporter oligos at the following concentrations: 250 nM of universal FAM probe (SEQ ID NO: 14), 450 nM of universal Yakima Yellow^{®} (SEQ ID NO: 22) probe, and 1000 nM of universal forward primer (SEQ ID NO: 21), 50 nM ROX internal standard, and 5 µL of 2x Integrated DNA Technologies (IDT) (Coralville, IA) rhPCR genotyping master mix (containing dNTPs, a mutant H784Q *Taq* polymerase (see Behlke, et al. U.S. 2015/0191707), chemically modified *Pyrococcus abyssi* RNase H2 (See Walder et al. UA20130288245A1), stabilizers, and MgCl₂).

**Table 16. Sequences of oligonucleotides used for the rs72558195 genotyping assay in Example 7.**

| Name | Sequence | SEQ ID NO. |
|---|---|---|
| Universal Forward primer | CGGCCCATGTCCCAGCGAA | SEQ ID NO. 21 |
| Probe 1 (FAM) | FAM-C+CATC+A+C+CGTG+CT-IBFQ | SEQ ID NO. 14 |
| Probe 2 (Yakima Yellow) | Yak-CAATC+C+C+CGAG+CT-IBFQ | SEQ ID NO. 22 |
| rs72558195 :G:A Allele 1 Forward primer | GCCCATGTCCCAGCGAACCATCACCGTGCTC TCCGTTGTTTTCCAGAAACgATTTC-x | SEQ ID NO. 31 |
| rs72558195 :G:A Allele 2 Forward primer | GCCCATGTCCCAGCGAACAATCCCCGAGCTC TCCGTTGTTTTCCAGAAATgATTTC-x | SEQ ID NO. 32 |
| rs72558195 :G:C Allele 3 Forward primer | GCCCATGTCCCAGCGAACAATCCCCGAGCTC TCCGTTGTTTTCCAGAAAGgATTTC-x | SEQ ID NO. 33 |
| rs1135840 Reverse primer | GCAACCAAGTCTTCCCTACAACcTTGAT-x | SEQ ID NO. 34 |

| | | |
|---|---|---|
| Nucleic acid sequences are shown 5'-3'. DNA is uppercase, RNA is lowercase. LNA residues are designated with a +. Location of potential mismatch is underlined. FAM = 6-carboxyfluorescein, Yak = Yakima Yellow (3-(5,6,4',7'-tetrachloro-5'-methyl-3',6'-dipivaloylfluorescein-2-yl)), IBFQ = Iowa Black FQ (fluorescence quencher), and x = C3 propanediol spacer block. | | |

gBlocks^{®} Gene Fragments (Integrated DNA Technologies, Inc., Coralville, IA) containing alleles of the rs72558195 SNP were utilized as the source of template DNA (See Table 17, SEQ ID NOs: 35, 36 and 37). Each well contained template representing one of six possible genotypes: allele 1 homozygote (1000 copies rs72558195 Allele 1 gBlock^{®} template (SEQ ID NO: 35)), allele 2 homozygote (1000 copies rs72558195 Allele 2 gBlock^{®} template (SEQ ID NO: 36)), allele 3 homozygote (1000 copies rs72558195 Allele 2 gBlock^{®} template (SEQ ID NO: 37)), heterozygote (mix of 500 copies of rs72558195 Allele 1 gBlock^{®} template (SEQ ID NO: 35) and 500 copies of rs72558195 Allele 2 gBlock^{®} template (SEQ ID NO: 36). heterozygote (mix of 500 copies of rs72558195 Allele 1 gBlock^{®} template (SEQ ID NO: 35) and 500 copies of rs72558195 Allele 3 gBlock^{®} template (SEQ ID NO: 37)). Template or water for the no template control (NTC) reactions were added into three replicate wells of two individual plates. The reactions underwent the following cycling protocol: 95°C for 10 minutes, then 45 cycles of 95°C for 10 seconds and 60°C for 45 seconds.

**Table 17. gBlock^{®} sequences used in Example 7**

| Name | Sequence | SEQ ID NO. |
|---|---|---|
| rs72558195 Allele 1 gBlock template | ACATCATTTTTATTGTATAAAAGCATTTTA GTATCAATTTTCTCATTTTTAAACCAAGTC TTCCCTACAACCTTGAATAAATGGTTTCCA AGGAAAATAAAATCTTGGCCTTACCTGGA TCCATGGGGAGTTCAGAATCCTGAAGTTT TCATTGAATCTTTTCATCAGGGTGAGAAA ATTCTGATCTTTATAATCAAATCGTTTCTG GAAAACAACGGAGCAGATCACATTGCAG GGAGCACAGCCCAGGATGAAAGTGGGAT CACAGGGTGAAGCTAAAGATTTAAAAATT TTTAAAAAAATTATTAAAAAATAAATATT TAAAAGATTTGCATTTGTTAAGACATAAA | SEQ ID NO. 35 |
| | GGAAATTTAGAAATTTTAAACAATATCTT ACAAATTCCCCATGTGTCCAAA | |
| rs72558195 Allele 2 gBlock template | ACATCATTTTTATTGTATAAAAGCATTTTA GTATCAATTTTCTCATTTTTAAACCAAGTC TTCCCTACAACCTTGAATAAATGGTTTCCA AGGAAAATAAAATCTTGGCCTTACCTGGA TCCATGGGGAGTTCAGAATCCTGAAGTTT TCATTGAATCTTTTCATCAGGGTGAGAAA ATTCTGATCTTTATAATCAAATCATTTCTG GAAAACAACGGAGCAGATCACATTGCAG GGAGCACAGCCCAGGATGAAAGTGGGAT CACAGGGTGAAGCTAAAGATTTAAAAATT TTTAAAAAAATTATTAAAAAATAAATATT TAAAAGATTTGCATTTGTTAAGACATAAA GGAAATTTAGAAATTTTAAACAATATCTT ACAAATTCCCATGTGTCCAAA | SEQ ID NO. 36 |
| rs72558195 Allele 3 gBlock template | ACATCATTTTATTGTATAAAAGCATTTTA GTATCAATTTTCTCATTTTTAAACCAAGTC TTCCCTACAACCTTGAATAAATGGTTTCCA AGGAAAATAAAATCTTGGCCTTACCTGGA TCCATGGGGAGTTCAGAATCCTGAAGTTT TCATTGAATCTTTTCATCAGGGTGAGAAA ATTCTGATCTTTATAATCAAATCCTTTCTG GAAAACAACGGAGCAGATCACATTGCAG GGAGCACAGCCCAGGATGAAAGTGGGAT CACAGGGTGAAGCTAAAGATTTAAAAATT TTTAAAAAAATTATTAAAAAATAAATATT TAAAAGATTTGCATTTGTTAAGACATAAA GGAAATTTAGAAATTTTAAACAATATCTT ACAAATTCCCCATGTGTCCAAA | SEQ ID NO. 37 |

| | | |
|---|---|---|
| Nucleic acid sequences are shown 5'-3'. DNA is uppercase. The location of the SNP is underlined. | | |

The results are shown in Figures 5A and 5B. From this, it is clear that the universal rhPCR genotyping system can be used to characterize multi-allelic genotypes.

A Tri-allelic AD 360plot was designed for illustrating allelic discrimination. Fluorescence signal (ΔRn) from the last PCR cycle of each dye was normalized across the three dyes from the same well. Angle and distance of data point from the origin is calculated using formula below: $Angle = {tan}^{- 1} \left(Δ{RnDye}_{1}\div Δ{RnDye}_{2}\right) \times \frac{120}{90}$ $Distance from origin = \sqrt{{\left(Δ{RnDye}_{1}\right)}^{2} + {\left(Δ{RnDye}_{2}\right)}^{2}}$

Figure 5B shows the Tri-allelic Allelic Discrimination 360plot of rs72558195, using rhPCR genotyping assay with 3 allele-specific primers multiplexed in a single reaction. By collecting fluorescence signal from all assays, six genotypes could be detected in a single reaction. The distance of data points from origin indicated the signal strength of dyes and the wide angle separation between data clusters indicated specificity of multiplex assay. NTC in the center of the plot indicated no primer dimers or non-specific amplification. The specificity of multiplex assay is achieved by the selectivity of RNase H2 and the mutant *Taq* DNA polymerase as used in the previous examples. This AD 360plot will also enable auto-calling capability by genotyping software.

A 360plot could be implemented for tetra-allelic, penta-allelic or hexa-allelic visualization. Therefore, visualization is possible for positions that could have multiple bases as well as potential deletions. The distance from origin remains unchanged for each calculation, and the angle formulas would be: $Angle = {tan}^{- 1} \left(Δ{RnDye}_{1}\div Δ{RnDye}_{2}\right)$ $Angle = {tan}^{- 1} \left(Δ{RnDye}_{1}\div Δ{RnDye}_{2}\right) \times \frac{72}{90}$ $Angle = {tan}^{- 1} \left(Δ{RnDye}_{1}\div Δ{RnDye}_{2}\right) \times \frac{60}{90}$

### EXAMPLE 8

The following example illustrates the capability of the methods of the present invention to provide quantitative SNP genotyping, allowing for determination of the copy numbers of different alleles. To demonstrate this, an assay was designed against rs1135840, a SNP in the human CYP2D6 gene. This gene can be present in multiple copies, and the number of copies with the rs1135840 SNP appears to affect drug metabolism (rapid metabolism of the drug Debrisoquine).

To demonstrate that the assay system can detect small differences in allele rations, a standard curve for analysis was created. Two gBlock^{™} (IDT, Coralville, IA) gene fragments were synthesized (Allele 1 and Allele 2, representing the two allelic variants (G>C) of the rs1135840 SNP) and then mixed at different ratios. Reactions were performed in 10 µL volumes, containing a total of 1500 copies of template at the ratios shown (10:0, 9:1, 8:2, 7:3, 6:4, 5:5, 4:6, 3:7, 2:8, 1:9, and 0:10), 250 nM of universal FAM probe (SEQ ID NO: 14), 450 nM of universal Yakima Yellow^{®} (SEQ ID NO: 22) probe, 1000 nM of universal forward primer (SEQ ID NO: 21), 150 nM of the two allele-specific forward primers, 500 nM of the reverse primer, and 5 µL of 2x Integrated DNA Technologies (IDT) (Coralville, IA) rhPCR genotyping master mix (containing dNTPs, a mutant H784Q *Taq* polymerase (see Behlke, et al. U.S. 2015/0191707), chemically modified *Pyrococcus abyssi* RNase H2 (See Walder et al. UA20130288245A1), stabilizers, and MgCl₂).

PCR was performed on Life Technologies (Carlsbad, CA) QuantStudio^{™} 7 Flex real-time PCR instrument using the following cycling conditions: 10 mins at 95°C followed by 45 cycles of 95°C for 10 seconds and 60°C for 45 seconds. End-point analysis of each of the plates was performed after 45 cycles with software provided by the respective companies (Bio-Rad CFX Manager 3.1 software (Bio-Rad, Hercules, CA) and QuantStudio^{™} Real-Time PCR Software v1.3 (Carlsbad, CA)).

**Table 16. Oligonucleotide sequences used in Example 8.**

| Name | Sequence | SEQ ID NO. |
|---|---|---|
| Universal Forward primer | CGGCCCATGTCCCAGCGAA | SEQ ID NO. 21 |
| Probe 1 (FAM) | FAM-C+CATC+A+C+CGTG+CT-IBFQ | SEQ ID NO. 14 |
| Probe 2 (Yakima Yellow) | Yak-CAATC+C+C+CGAG+CT-IBFQ | SEQ ID NO. 22 |
| rs1135840 Allele 1 Forward primer | GCCCATGTCCCAGCGAACCATCACCGTGC TGTCTTTGCTTTCCTGGTGAGcCCATG-x | SEQ ID NO. 38 |
| rs1135840 Allele 2 Forward primer | GCCCATGTCCCAGCGAACAATCCCCGAGC TGTCTTTGCTTTCCTGGTGAcCCATG-x | SEQ ID NO. 39 |
| rs1135840 Reverse primer | GCGTTGGAACTACCACATTGCTTTATuGTA CT-x | SEQ ID NO. 40 |

| | | |
|---|---|---|
| Nucleic acid sequences are shown 5'-3'. DNA is uppercase, RNA is lowercase. LNA residues are designated with a +. Location of potential mismatch is underlined. FAM = 6-*carboxyfluorescein,* Yak = Yakima Yellow (3-(5,6,4',7'-tetrachloro-5'-methyl-3',6'-dipivaloylfluorescein-2-yl)), IBFQ = Iowa Black FQ (fluorescence quencher), and x = C3 propanediol spacer block. | | |

The resulting data is illustrated in Figure 7. The spread of each of the sample mixes is sufficient for the determination of the number of copies of each template.

After demonstration of the required amount of separation of allelic quantities, it is possible to determine the number of copies present of each allele in an experimental sample. To test this, the previously described assay designed against rs1135840, was utilized to test thirteen Coriell genomic DNA (Camden, NJ) samples with varying CYP2D6 copy numbers with varying rs1135840 genotypes. These samples have known defined copy numbers and rs1135840 genotypes which could be verified after testing with the universal rhPCR genotyping mix. From this, these samples can also be categorized as being homozygotes for either allele, or heterozygotes.

To calculate the copy number from the data, two duplex reactions were run for each sample. Reactions were performed in 10 µL volumes, containing 3 ng of one of the following genomic DNAs: NA17123, NA17131, NA17132, NA17149, NA17104, NA17113, NA17144, NA17213, NA17221, NA17114, NA17235, or NA17241. Each individual assay also contained 50 nM ROX normalizer oligo, 250 nM of universal FAM probe (SEQ ID NO: 14), 450 nM of universal Yakima Yellow^{®} (SEQ ID NO: 22) probe, 1000 nM of universal forward primer (SEQ ID NO: 21), 150 nM of the two allele-specific forward primers (SEQ ID NO: 38 and 39), 500 nM of the reverse primer (SEQ ID NO: 40), and 5 µL of 2x Integrated DNA Technologies (IDT) (Coralville, IA) rhPCR genotyping master mix (containing dNTPs, a mutant H784Q *Taq* polymerase (see Behlke, et al. U.S. 2015/0191707), chemically modified *Pyrococcus abyssi* RNase H2 (See Walder et al. UA20130288245A1), stabilizers, and MgCl₂). Assays also contained a separate RNase P assay (See table 17, SEQ ID NOs: 41 - 43)) for normalization of the template concentration.

**Table 17. RNase P assay sequences used in Example 8.**

| Name | Sequence | SEQ ID NO. |
|---|---|---|
| RNase P Forward primer | GCGGAGGGAAGCTCATCAG | SEQ ID NO. 41 |
| RNase P Reverse primer | CCCTAGTCTCAGACCTTCCCAA | SEQ ID NO. 42 |
| Probe 2 (Yakima Yellow) | Yak-CCACGAGCTGAGTGCGTCCTGTCA-IBFQ | SEQ ID NO. 43 |

| | | |
|---|---|---|
| Nucleic acid sequences are shown 5'-3'. DNA is uppercase. FAM = *6-carboxyfluorescein,* Yak = Yakima Yellow (3-(5,6,4',7'-tetrachloro-5'-methyl-3',6'-dipivaloylfluorescein-2-yl)), IBFQ = Iowa Black FQ (fluorescence quencher). | | |

Quantitative PCR was performed on Life Technologies (Carlsbad, CA) QuantStudio^{™} 7 Flex real-time PCR instrument using the following cycling conditions: 10 mins at 95°C followed by 45 cycles of 95°C for 10 seconds and 60°C for 45 seconds. End-point analysis of each of the plates was performed after 45 cycles with the QuantStudio^{™} Real-Time PCR Software v1.3 (Carlsbad, CA) software provided by the company.

Copy number was determined by the following method. For each sample shown to be a homozygote, ΔCq (RNase P Cq - rs1135840 assay Cq) was calculated for each sample. For samples shown to be heterozygotes, ΔCq was calculated for both alleles (RNase P Cq - rs1135840 assay 1 Cq and RNase P Cq - rs1135840 assay 2 Cq). Next, ΔΔCq (ΔCq - mean ΔCq for known 2 copy control DNA samples) was calculated for each allele. This correction allowed for normalization against amplification differences between the SNP assay and the RNase P assay. Finally, the following equation was used to calculate copy number for each allele: $Copy number of allele = 2 ∗ \left({2}^{∧}\left(ΔΔCq\right)\right)$

The resulting end-point data is shown in Figure 8A and calculated copy numbers are shown in Figure 8B. The genotypes determined in Figure 8A (homozygotes allele 1, Homozygotes allele 2, or heterozygote) all matched the known genotypes, and allowed correct calculation of the copy number. The established reference copy number of the individual samples is shown under each result. In each case, the copy number determined by the assay correctly determined the genotype and copy number of the input DNA.

### EXAMPLE 9

The following example demonstrates that a variation of an rhPCR probe can be used for multiplexed rhPCR.

The assay schematic is provided in Figure 9. In the first round of PCR, 5' tailed target-specific rhPrimers are used. The 5' tails upon incorporation into the amplicon contain binding sites for a second round of PCR with different primers (blocked or unblocked) to add application specific sequences. For example, as depicted in Figure 9, this system can be used for amplification enrichment for next generation sequencing. In this case, 5' tailed rhPCR primers contain read 1/read 2 primer sequences. The second round of PCR adds adapter sequences such as the P5/P7 series for Illumina^{®} based sequencing platforms or other adaptors, including ones containing barcodes/unique molecular identifiers. This approach allows for adding any additional sequences onto the amplicon necessary for input into any NGS platform type.

As illustrated in Figure 10, two primers sets, including one containing a 96-plex set of 5' tailed rhPrimers, and one containing 96 DNA "standard" 5' tailed PCR primers were designed using an IDT algorithm. The two primer sets differed only in that the rhPrimers contained an internal cleavable RNA base and a blocking group on the 3' end. Once the blocking group was removed by RNase H2 cleavage, the primer sequences become identical.

The first round of PCR reactions contained the 96 plex at 10 nM of each blocked target specific primer, 10 ng of NA12878 human genomic DNA (Coriell Institute for Medical Research, Camden,NJ), 200 mU of chemically modified *Pyrococcus abyssi* RNase H2 (See Walder et al. UA20130288245A1) (IDT, Coralville, IA) and 1X KAPA 2G HotStart Fast Ready Mix^{™} (Kapa Biosystems, Wilmington, MA). The thermal cycling profile was 10 mins at 95°C followed by 8 cycles of 95°C for 15 seconds and 60°C for 4 minutes, and a final 99°C finishing step for 15 minutes. Reactions were cleaned up with a 2X AMPure^{™} XP beads (Beckman Coulter, Brea, CA). Briefly, 100 µL AMPure^{™} SPRI beads were added to each PCR well, incubated for 5 minutes at room temperature and collected for 5 minutes at room temperature on plate magnet (DynaMag^{™} (Thermo-Fisher, (Watherham, MA) 96-well plate side-magnet). Beads were washed twice with 80% ethanol, and allowed to dry for 3 minutes at room temperature. Samples were eluted in 22 µL of TE at pH 8.0.

The second round of PCR was set up using 20 µL of the cleaned up first round PCR products, universal PCR-50F and PCR-47R primers (See table 18, SEQ ID NOs: 44 and 45) at 2 uM and 1X KAPA 2G HotStart Fast Ready Mix^{™} (KAPA Biosystems, Wilmington, MA). Reactions were cycled for 45 seconds at 98°C followed by 20 cycles of 98°C for 15 seconds, 60°C for 30 seconds, and 72°C for 30 seconds. A final 1 minute 72°C polishing step finished the reaction. Samples were cleaned up again with 0.8X AMPure^{™} beads. Briefly, 40 µL AMPure^{™} SPRI beads were added the second PCR wells, incubated for 5 minutes at room temperature and collected for 5 minutes at room temperature on plate magnet (DynaMag^{™} (Thermo-Fisher, (Watherham, MA) 96-well plate side-magnet). Beads were washed twice with 80% ethanol, and allowed to dry for 3 minutes at room temperature. Samples were eluted in 22 µL of TE at pH 8.0, and 20 µL was transferred to a new tube.

2 µL of the samples were analyzed using the Agilent^{®} High Sensitivity D1000^{™} Screen Tape^{™} on the Agilent^{®} 2200 Tape Station^{™} (Agilent Technologies^{®}, Santa Clara, CA). Quantification was performed using the KAPA Library Quantification Kit (KAPA Biosystems, Wilmington, MA) for Illumina^{®} Platforms, according to the manufacturer's protocol. Replicate samples were pooled to a final concentration of 10 pM, and 1% PhiX bacteriophage sequencing control was added. Samples were run with a V2 300 cycle MiSeq^{™} kit on an Illumina^{®} (San Diego, CA) MiSeq^{™} platform, using standard protocols from the manufacturer.

**Table 18. Universal assay sequences used in Example 9.**

| Name | Sequence | SEQ ID NO. |
|---|---|---|
| Universal PCR-50F | AATGATACGGCGACCACCGAGATCTACAC TCTTTCCCTACACGACGCTCT | SEQ ID NO. 44 |
| Universal PCR-47R | CAAGCAGAAGACGGCATACGAGATGGACC TATGTGACTGGAGTTCAGACGTGTGC | SEQ ID NO. 45 |

| | | |
|---|---|---|
| Nucleic acid sequences are shown 5'-3'. DNA is uppercase. | | |

Figure 10 shows the results from the Agilent^{®} Tape Station. The primer dimer product was the most significant product produced using standard DNA primers in the presence of DNA template, with only a small amount of full length expected product. In the absence of template, the primer dimer product was the major component of the reaction. In the case of the blocked rhPCR primers, the vast majority of the material was the desired PCR products, with little primer dimer observed. In the absence of template, there is no primer dimer present, contrasting with the overwhelming abundance of primer dimer observed in the no template lane of the unblocked DNA primers. Quantitation of the product versus primer dimer bands show that mass ratio of product to primer dimer for the unblocked DNA primers was 0.6. The mass ratio for the rhPCR primers was 6.3.

Figure 11 summarizes two key sequencing metrics. The first is the percent of mapped reads from the sequencing data. The rhPCR reactions gave a percentage of reads mapped to the human genome at 85%, whereas the non-blocked DNA primers on give a mapped read percentage of less than 20. A second metric, the percentage of on-target reads, is almost 95% when using rhPCR primers, but less than 85% when the non-blocked primers are used in the multiplex. These results clearly demonstrate the utility of using rhPCR in multiplexing, where a large increase of the desired material is seen, and a vast reduction in undesired side products is observed. The differences mean less unwanted sequencing reads , and the depth of coverage of desired sequences is higher.

### EXAMPLE 10

This example demonstrates enhanced sensitivity and accuracy of assay systems of the disclosure as compared to standard T7 endonuclease cleavage assays.

A total of 36 sites modified with the CRISPR/Cas9 protein system were chosen to be comparatively analyzed by T7 endonuclease, next-generation sequencing (NGS), or a qPCR "Genie" assay system of the disclosure.

To mutate the chosen genomic sites, a HEK293 cell line was generated with stable expression of *Spyogenes* Cas9. AltR^{™} guide RNAs were reverse transfected into the cells in 96-well plates (40,000 cells/well) using 0.75 µL RNAiMAX (Thermo) per well. Briefly, AltR^{™} crRNAs were designed from the IDT CRISPR2.0 design engine to target exon 1 or 2 of selected human genes. Prior to transfection, guide RNAs (crRNA/tracrRNA with AltR^{™} chemistry) were duplexed in an equimolar ratio at 3 µM final concentration of the complex in IDT duplex buffer (Integrated DNA Technologies, Coralville, IA). Complexes were heated to 95 °C for 5 min and cooled to room temperature. Genomic DNA was isolated after 48 hrs with 50 µL Quick Extract buffer (Epicentre), using standard techniques described by the manufacturer. DNA solutions were further diluted with 100 µL water before further analysis was performed.

Analysis by T7 endonuclease digestion was done as follows. CRISPR-Cas9-treated cells were washed with 100 µL of PBS. Cells were lysed by adding 50 µL of QuickExtract^{™} DNA Extraction Solution (Integrated DNA Technologies). Cell lysates were then transferred to appropriate PCR tubes or plate, then vortexed and heated in a thermal cycler at 65 °C for 10 min, followed by 98°C for 5 min, after which 100 µL of Nuclease-Free Water was added to dilute the genomic DNA. The samples were then vortexed and spun down. PCR was set up using template, primers, and components of the Alt-R Genome Editing Detection Kit and KAPA HiFi HotStart PCR Kit as follows. Sample: 4 µL (~ 40 ng) genomic DNA, 300 nM forward primer, 300 nM reverse primer, 5 µL (1X) of KAPA HiFi Fidelity Buffer (5X), 1.2 mM (0.3 mM each) dNTPs, 0.5 U KAPA HiFi Hotstart DNA Polymerase (1 U/µL), for a total volume of 25 µL. Alt-R^{™} Control A: 1 µL Alt-R^{™} Control A template/primer mix, 5 µL (1X) of KAPA HiFi Fidelity Buffer (5X), 1.2 mM (0.3 mM each) dNTPs, 0.5 U KAPA HiFi Hotstart DNA Polymerase (1 U/µL), for a total volume of 25 µL. Alt-R^{™} Control B: 1 µL Alt-R^{™} Control B template/primer mix, 5 µL (1X) of KAPA HiFi Fidelity Buffer (5X), 1.2 mM (0.3 mM each) dNTPs, 0.5 U KAPA HiFi Hotstart DNA Polymerase (1 U/µL), for a total volume of 25 µL. PCR was run using the following conditions: denature at 95 °C for 5 min; 30 cycles of: denature at 98 °C for 20 sec, anneal between 64-67 °C (depending on polymerase) for 15 sec, extend at 72 °C for 30 sec; then extend at 72 °C for 2 minutes. Heteroduplexes for T7EI digestion were formed as follows. 2 µL T7EI Reaction Buffer (10X) and 6 µL Nuclease-Free Water was combined with 10 µL experimental target or Alt-R^{™} HPRT control from the PCR, 10 µL Control A PCR component (homoduplex control), or 5 µL Control A and 5 µL Control B (heteroduplex control). The PCR products were then placed in a thermal cycler with 95 °C denaturation for 10 min, ramp from 95-85 °C at a ramp rate of -2 °C/sec, then ramp from 85-25 °C at a ramp rate of -0.3°C/sec. 18 µL of PCR heteroduplexes from the previous step were combined with 2 µL T7 endonuclease I (1 U/µL), then the T7EI reaction was incubated at 37 °C for 60 min. T7EI mismatch detection result were visualized on a Fragment Analyzer^{™} system with Mutation Discovery Kit according to the manufacturer's instructions (Integrated DNA Technologies). After amplification and cleavage, amplicons were sized on the Fragment Analyzer^{™} (Advanced Analytical, Inc, Ames, IA) capillary electrophoresis system.

NGS sequencing analysis of the mutated samples employed locus-specific primers positioned approximately 75-bp flanking the Cas9 cleavage site. Primers contained universal 5'-tails that allowed for secondary amplification that added Illumina^{™} TruSeq^{™} i5 and i7 adapters with sample-specific barcodes to the amplicons. The locus-specific primers were designed as RNase H2-cleavable primers with the 4DMX blocking modification at the 3'-end (where the 4DMX nomenclature indicates 4 DNA bases, a mismatched DNA base and a propanediol C3-spacer 3' of the RNA base). Using a master-mix containing a hot-start Taq polymerase and hot-start RNaseH2, the genomic DNAs were amplified using the following cycling conditions: 95 °C^{5:00} + (95 °C^{0:15} + 60 °C^{1:00}) x 8 cycles + 99 °C^{15:00}. Samples were purified using SPRI beads (1.5x Agencourt^{™} Ampure^{®} XP beads, Beckman Coulter) per the manufacturer's protocol. The second PCR incorporated the Illumina adapters and was run under the following conditions: 95 °C^{5:00} + (95 °C^{0:15} + 60 °C^{0:30} + 72 °C^{0:30}) x 18 cycles + 99 °C^{15:00}.

The resultant amplicons underwent 1x SPRI^{™} clean-up and were quantified via the KAPA^{™} library qPCR quantitation (KAPA Biosystems, Wilmington, MA) kit per the manufacturer's recommended protocol. In addition, amplicons were sized on the Fragment Analyzer^{™} (Advanced Analytical, Inc., Ames, IA) capillary electrophoresis system.

DNA sequencing was carried out on an Illumina^{™} MiSeq^{®} using a MiSeq^{®} Nano cartridge (v2, 300 cycles). Data was de-multiplexed via an in-house bioinformatics processing pipeline. Analysis for specific editing events relative to a reference amplicon was performed with CRISPResso^{™} using methods described.

Quantitative PCR assay primers for analysis according to methods of the disclosure were designed so that the RNA nucleotide was located two bases after the primary Cas9 cleavage site, allowing for maximal discrimination from both the RNase H2 enzyme and the DNA polymerase (Figure 12). Primers were designed to include a proprietary universal 5' domain (UniFor-UniPro-), which has sequence identity with both a universal forward primer, and a universal 5' nuclease degradable probe (Table 19, Seq ID No. 1-72 and Table 20, Seq ID No. 76-147). A Taqman-based RNase P assay (Seq ID No. 73-75) was utilized as a universal control for template concentration normalization in all cases. All primers were synthesized at Integrated DNA Technologies (IDT, Coralville, IA). Amplification was performed with 2.5 µL of the same QuickExtract^{™} genomic DNA utilized in T7 and NGS analyses. A wild-type (WT) control that was grown and extracted by the same method was also analyzed for normalization purposes. Reaction volumes were 10 µL in all cases, and included the universal forward primer, the universal probe, the interrogating primer, and the non-interrogating reverse primer. 1x of a rhPCR-genotyping master mix, containing hot-start RNase H2, a thermophilic DNA polymerase, buffer, and dNTPs was also included. The universal forward primer was present in all reactions at 1000 nM (10 pmol), and the universal assay probe was present at 300 nM (3 pmol). The assay specific primers were present at 200 nM (2 pmol) for the forward (mutation interrogating) primer, while the non-interrogating locus-specific reverse primer was present at 500 nM (5 pmol). RNase P control reactions were run with 500 nM of forward and reverse RNase P primers, and 250 nM probe (Seq ID No. 73-75). Reactions were run on a CFX384^{™} Real-Time qPCR machine (Bio-Rad^{®}, Hercules, CA). The following cycling conditions were utilized: 95°C^{10:00} + (95°C^{0:15} + 59°C^{0:20} + 72°C^{0:30}) x 55 cycles.

**Table 19. Discriminatory forward primers utilized in Example 10.**

| Name | Sequence | Seq ID No. |
|---|---|---|
| GCK-356-1 | UniFor-UniPro-CCCTGGGTCCCTGGGaGAATC-x | 46 |
| GCK-356-2 | UniFor-UniPro-CGAGGAGAACCACATTCTCCcAGGGT-x | 47 |
| ERBB3-33-1 | UniFor-UniPro-GGGCGGCCGTGACuCACC-x | 48 |
| ERBB3-33-2 | UniFor-UniPro-GAGGGAAGGGGGTGAGTcACGGCG-x | 49 |
| TTR-1257-1 | UniFor-UniPro-CCTGGGAGCCATTTGCCTCuGGGTT-x | 50 |
| TTR-1257-2 | UniFor-UniPro-CTTTGGCAACTTACCCAGAGGcAAATC-x | 51 |
| HAMP-253-1 | UniFor-UniPro-GCACTGAGCTCCCAGAuCTGGC-x | 52 |
| HAMP-253-2 | UniFor-UniPro-GCAAGCGGCCCAGATCuGGGAC-x | 53 |
| BIRC5-606-1 | UniFor-UniPro-GACGACCCCATGTAAGTCTTCuCTGGG-x | 54 |
| BIRC5-606-2 | UniFor-UniPro-CGAGGCTGGCCAGAGAaGACTTT-x | 55 |
| SAA 146-1 | UniFor-UniPro- CTTTCCCAACAAGATTATCATTTCCTTTAAaAAAAT-x | 56 |
| SAA 146-2 | UniFor-UniPro-CGCCCCAGGATAACTATTTTTTTTaAAGGT-x | 57 |
| IDO1-97-1 | UniFor-UniPro-AGACACTGAGGGGCACCaGAGGT-x | 58 |
| IDO1-97-2 | UniFor-UniPro-CTTGTAGTCTGCTCCTCTGGuGCCCG-x | 59 |
| IDO1-176-1 | UniFor-UniPro- AGTAAAGAGTACCATATTGATGAAGAAgTGGGA-x | 60 |
| IDO1-176-2 | UniFor-UniPro-GCAGAGCAAAGCCCACTTcTTCAA-x | 61 |
| CYP27A-31016-1 | UniFor-UniPro-CCTTTGGTGAGGACTCCCAgATGGC-x | 62 |
| CYP27A-31016-2 | UniFor-UniPro-CCTGGGCCCCATCTGgGAGTG-x | 63 |
| SAA 226-1 | UniFor-UniPro-TCTCCTCTGATCTAGAGAGGTAAGcAGGGA-x | 64 |
| SAA 226-2 | UniFor-UniPro-ACCAGGCCCGACCCTGCTuACCTG-x | 65 |
| KIF11-369-1 | UniFor-UniPro-GAGAAGGGGAAGAACATCCAgGTGGA-x | 66 |
| KIF11-369-2 | UniFor-UniPro-GCATCTCACCACCACCTGgATGTA-x | 67 |
| C3-1394-1 | UniFor-UniPro-CTGGACAGCACTAGTTTTTTGCcTGGGT-x | 68 |
| C3-1394-2 | UniFor-UniPro-CCACGACTTCCCAGGCaAAAAT-x | 69 |
| HOGA-505-1 | UniFor-UniPro-CACTGCAGAGGTGGACTaTGGGT-x | 70 |
| HOGA-505-2 | UniFor-UniPro-GATTCTCCTCCAGTTTCCCATAGuCCACG-x | 71 |
| EGFR-123344-1 | UniFor-UniPro-CCAGAGGATGTTCAATAACTGTGAgGTGGA-x | 72 |
| EGFR-123344-2 | UniFor-UniPro-CAAATTCCCAAGGACCACCuCACAC-x | 73 |
| ALDH2-15144-1 | UniFor-UniPro-TGAAGGGGACAAGGTGAGAaCTGGA-x | 74 |
| ALDH2-15144-2 | UniFor-UniPro-CCCAAGGTAAGTCACCAGTTCuCACCA-x | 75 |
| AGXT-140-1 | UniFor-UniPro-CCATGGCCTCTCACAAGCTgCTGGA-x | 76 |
| AGXT-140-2 | UniFor-UniPro-GGGGGTCACCAGCAGcTTGTC-x | 77 |
| APOC-2929-1 | UniFor-UniPro-CCGTTAAGGACAAGTTCTCTGAGTuCTGGC-x | 78 |
| APOC-2929-2 | UniFor-UniPro-TCAGGGTCCAAATCCCAGAACuCAGAC-x | 79 |
| Met 27554-1 | UniFor-UniPro- AATTTTATTTACTTCTTGACGGTCCAAAGGgAAACA-x | 80 |
| Met 27554-2 | UniFor-UniPro-GTCTGAGCATCTAGAGTTTCCCuTTGGT-x | 81 |
| SAA 88-1 | UniFor-UniPro-AGGTGAGGAGCACACCAAGGAgTGATA-x | 82 |
| SAA 88-2 | UniFor-UniPro- GAAAACAGAGTAAGTTTTAAAAATCACTCcTTGGA-x | 83 |
| HIF1A-293-1 | UniFor-UniPro-TCGCACCCCCACCTcTGGAG-x | 84 |
| HIF1A-293-2 | UniFor-UniPro-GAAGGAAAGGCAAGTCCAGAGgTGGGC-x | 85 |
| Met 27475-1 | UniFor-UniPro- AATTTTATTTACTTCTTGACGGTCCAAAGGgAAACA-x | 86 |
| Met 27475-2 | UniFor-UniPro-GTCTGAGCATCTAGAGTTTCCCuTTGGT-x | 87 |
| HAMP-295-1 | UniFor-UniPro-CTCGCCAGCCTGACCaGTGGG-x | 88 |
| HAMP-295-2 | UniFor-UniPro-GGGAAAACAGAGCCACTGGuCAGGG-x | 89 |
| GRHPR-2234-1 | UniFor-UniPro-GCCTCCTCTCCGACCAcGTGGT-x | 90 |
| GRHPR-2234-2 | UniFor-UniPro-GGATCCTCTTGTCCACGTGgTCGGAC-x | 91 |
| HAMP-88-1 | UniFor-UniPro-GGCGCCACCACCTTcTTGGT-x | 92 |
| HAMP-88-2 | UniFor-UniPro-GCTCTGTCTCATTTCCAAGAAgGTGGA-x | 93 |
| Met 27254-1 | UniFor-UniPro-GAGCCAAAGTCCTTTCATCTGTaAAGGT-x | 94 |
| Met 27254-2 | UniFor-UniPro-GAAGTTGATGAACCGGTCCTTTACaGATGT-x | 95 |
| GRHPR-2264-1 | UniFor-UniPro-ACAAGAGGATCCTGGATGCTgCAGGA-x | 96 |
| GRHPR-2264-2 | UniFor-UniPro-CGCTCTAGCTCCTTGGCaGGGAA-x | 97 |
| Serpina 279-1 | UniFor-UniPro-ACTCAGTTCCACAGGTGGGAGgGAGGC-x | 98 |
| Serpina 279-2 | UniFor-UniPro-CACTCTAAGCCCTGCTGTCCCaCCTGA-x | 99 |
| Myc 459-1 | UniFor-UniPro-CGGGAGGCTATTCTGCCCATTuGGGAT-x | 100 |
| Myc 459-2 | UniFor-UniPro-CGGGGAAGTGTCCCCAAAuGGGCT-x | 101 |
| Serpina 130-1 | UniFor-UniPro-GCTGCTGCTGCCAGGAAuTCCAC-x | 102 |
| Serpina 130-2 | UniFor-UniPro-CCCCTCCAACCTGGAATTcCTGGG-x | 103 |
| Myc 490-1 | UniFor-UniPro-CTGCCAGGACCCGCTTCuCTGAT-x | 104 |
| Myc 490-2 | UniFor-UniPro-CAAGGAGAGCCTTTCAGAGAaGCGGC-x | 105 |
| GRHPR-2179-1 | UniFor-UniPro-GATGAGCCCATCCCTGCcAAGGT-x | 106 |
| GRHPR-2179-2 | UniFor-UniPro-CGCTCTAGCTCCTTGGCaGGGAA-x | 107 |
| GYG-2851-1 | UniFor-UniPro-TCGCCACCCCTCAGGuCTCAC-x | 108 |
| GYG-2851-2 | UniFor-UniPro-ACCTCATGGAGTCTGAGACCuGAGGC-x | 109 |
| GYG-2793-1 | UniFor-UniPro-GCCCTGGTCCTGGGAuCATCA-x | 110 |
| GYG-2793-2 | UniFor-UniPro-CTGTGCTGTTTCAGAGATGATCcCAGGT-x | 111 |
| Serpina 79-1 | UniFor-UniPro-CAAGAGTCCTGAGCTGAACCAAgAAGGT-x | 112 |
| Serpina 79-2 | UniFor-UniPro-CGACCCCCTCCTCCTTCTTgGTTCT-x | 113 |
| Myc 538-1 | UniFor-UniPro-CTGCTTAGACGCTGGATTTTTTuCGGGA-x | 114 |
| Myc 538-2 | UniFor-UniPro-CTGGTTTTCCACTACCCGAAArAAAAA-x | 115 |
| GYG-2744-1 | UniFor-UniPro-CTTTGTATTAAGATCAGGCCTTTGTgACACA-x | 116 |
| GYG-2744-2 | UniFor-UniPro-CATCGTTTGTGGTTAGTGTCACaAAGGG-x | 117 |
| RNase P For | GCGGAGGGAAGCTCATCAG | 118 |
| RNase P Rev | CCCTAGTCTCAGACCTTCCCAA | 119 |
| RNase P probe | FAM-CCACGAGCTGAGTGCGTCCTGTCA-IBFQ | 120 |

DNA is uppercase, RNA is lowercase. FAM = 6-Fluorescein fluorescent dye (IDT, Coralville, IA). IBFQ=Iowa Black^{™} fluorescent quencher (IDT, Coralville, IA). UniFor-UniPro = universal forward primer, and universal probe binding site. X = propanediol (C3) spacer blocking group.

**Table 20. Non-discriminatory reverse primers utilized in Example 10.**

| Name | Sequence | Seq ID No. |
|---|---|---|
| GCK-356-1 | GAGGAAACTGTGACTGAACCTC | 121 |
| GCK-356-2 | CCAAGGCTTCTCCGCC | 122 |
| ERBB3-33-1 | GAGTCCGGGGAGGGATG | 123 |
| ERBB3-33-2 | CAATCCCTACTCCAGCCTC | 124 |
| TTR-1257-1 | ATGTGAGCCTCTCTCTACCAA | 125 |
| TTR-1257-2 | GTCCTCTGATGGTCAAAGTTCTA | 126 |
| HAMP-253-1 | CACTGGTCAGGCTGGC | 127 |
| HAMP-253-2 | CAAGCTCAAGACCCAGCA | 128 |
| BIRC5-606-1 | CAACTCAAATCTTTTGACAACTCAG | 129 |
| BIRC5-606-2 | GGAGCTGGAAGGCTGG | 130 |
| SAA 146-1 | TTCAGAATGGTATGGCTGTATGC | 131 |
| SAA 146-2 | CACAGATCAGGTGAGGAGCA | 132 |
| IDO1-97-1 | GTTTTCCATAGCGTGTGCC | 133 |
| IDO1-97-2 | GTGGTCACTGGCTGTGG | 134 |
| IDO1-176-1 | TTCCCACATTTTACTGCCTTCTC | 135 |
| IDO1-176-2 | CGCTATGGAAAACTCCTGGA | 136 |
| CYP27A-31016-1 | CAGGTCTGTGCATCAGCG | 137 |
| CYP27A-31016-2 | CTTTCTGGAAGCGATACCTG | 138 |
| SAA 226-1 | CGCACAGAACTCAACATGGG | 139 |
| SAA 226-2 | AATAGTTATCCTGGGGCATACAGC | 140 |
| KIF11-369-1 | GCTCGGAATCCTGTCAGC | 141 |
| KIF11-369-2 | CAGCCAAATTCGTCTGCG | 142 |
| C3-1394-1 | GGGATGTTCCAGTCACTGTTAC | 143 |
| C3-1394-2 | GGTTGGTGGCAGGGG | 144 |
| HOGA-505-1 | AGGGGAAGGTGCCCAG | 145 |
| HOGA-505-2 | AAGGTGGACATTGCGGG | 146 |
| EGFR-123344-1 | TCATAATTCCTCTGCACATAGGT | 147 |
| EGFR-123344-2 | GCCAAGGCACGAGTAACA | 148 |
| ALDH2-15144-1 | CGTATAAAATAGAAGACGAATCCATCCC | 149 |
| ALDH2-15144-2 | ATGGCACGATGCCGT | 150 |
| AGXT-140-1 | GGCTTGAGCAGGGCC | 151 |
| AGXT-140-2 | TGGCCAAGGCCAGTG | 152 |
| APOC-2929-1 | TCAGGCAGCCACGGC | 153 |
| APOC-2929-2 | GTGACCGATGGCTTCAGT | 154 |
| Met 27554-1 | CATACGCAGCCTGAAGTATATTAAACA | 155 |
| Met 27554-2 | TAGATGCTCAGACTTTTCACACAAGA | 156 |
| SAA 88-1 | TTCAGAATGGTATGGCTGTATGC | 157 |
| SAA 88-2 | AGCAGGGAAGGCTCAGTATAAATAG | 158 |
| HIF1A-293-1 | TAAGCGCTGGCTCCCT | 159 |
| HIF1A-293-2 | CTCTAGTCTCACGAGGGGTT | 160 |
| Met 27475-1 | CTCTTTTCTGTGAGAATACACTCCAG | 161 |
| Met 27475-2 | TACCCCATTAAGTATGTCCATGCC | 162 |
| HAMP-295-1 | TCTCCCATCCCTGCTGC | 163 |
| HAMP-295-2 | CCGCTTGCCTCCTGC | 164 |
| GRHPR-2234-1 | CACCCAGTGTGCACCT | 165 |
| GRHPR-2234-2 | GCCAAGGAGCTAGAGCGA | 166 |
| HAMP-88-1 | GAGGCGGTGGTCTGAG | 167 |
| HAMP-88-2 | TGTTCCCTGTCGCTCTG | 168 |
| Met 27254-1 | CTTTAGCCTTCTCACTGATATCGAATG | 169 |
| Met 27254-2 | GCATATTCTCCCCACAGATAGAAGA | 170 |
| GRHPR-2264-1 | CCTGCCCACCCAGTG | 171 |
| GRHPR-2264-2 | CTGTGAGGTGGAGCAGTG | 172 |
| Serpina 279-1 | TAGCTCCTGGGCATTTCTTCC | 173 |
| Serpina 279-2 | AGCTTGAGGAGAGCAGGAAAG | 174 |
| Myc 459-1 | CCTGGTTTTCCACTACCCGA | 175 |
| Myc 459-2 | CACTGGAACTTACAACACCCG | 176 |
| Serpina 130-1 | TTCCTGCTCTCCTCAAGCTCT | 177 |
| Serpina 130-2 | GAGCTGAACCAAGAAGGAGGA | 178 |
| Myc 490-1 | AGGCATTCGACTCATCTCAGC | 179 |
| Myc 490-2 | TGCACTGGAACTTACAACACC | 180 |
| GRHPR-2179-1 | TCGGAGAGGAGGCAGAG | 181 |
| GRHPR-2179-2 | TTCTCCTGAGGGCCTCC | 182 |
| GYG-2851-1 | ACAGGGAGAAGGATGTCAGAG | 183 |
| GYG-2851-2 | GTCCTGGGATCATCTCTGAAAC | 184 |
| GYG-2793-1 | ACAGGGAGAAGGATGTCAGAG | 185 |
| GYG-2793-2 | CACTAACCACAAACGATGCCT | 186 |
| Serpina 79-1 | GAATTCCTGGCAGCAGCA | 187 |
| Serpina 79-2 | CTACTGCCTCCACCCGAA | 188 |
| Myc 538-1 | TAGGCATTCGACTCATCTCAGC | 189 |
| Myc 538-2 | TGCACTGGAACTTACAACACC | 190 |
| GYG-2744-1 | GGACCAGGGCACCTTTG | 191 |
| GYG-2744-2 | GGCTTTCTCCAGATAAGATACTG | 192 |

| | | |
|---|---|---|
| DNA is uppercase. | | |

Although the reverse primers were not cleaved by RNase H2 in these assays, the results suggest that blocked-cleavable reverse primers could also be utilized in these assays.

Analysis of the amplification data was performed using a ΔΔCq method. Briefly, the ΔCq was calculated between each of the target Cqs and the corresponding reference (RNase P) Cqs. A conversion was then done with the calculated ΔCq, where ΔCq experimental was calculated as being equal to 2^-ΔCq. ΔΔCq was then calculated by normalization against the ΔCq calculated from the WT (un-mutated) control.

Experimental results are shown in Figures 13A and 13B. Figure 13A shows the clear difference between the NGS and T7 endonuclease cleavage data. Of the 72 assays tested, 64 of the T7EI results were >25% discordant in their quantification of the amount of mutant template present compared to the NGS gold standard. The error in T7EI quantification is expected as EMCA assays usually underestimate genome editing rates, as discussed above.

Figure 13B shows the comparison between the NGS data and the method of the present invention. A total of 74 assays were tested in the new assay format. Of these, 13 failed to amplify (17%), of which 10/13 (76%) of the failed assays showed sequence features that impair primer function and could easily be removed from future testing by a design algorithm (G-quadraplexes, hairpins, etc.). Of the 61/74 assays that amplified, only 2/61 (3.3%) showed more than 25% divergence from the results obtained with the NGS experiment. These data, combined with the data from Figure 13A, demonstrate the utility of the assays of the disclosure in providing a rapid, inexpensive PCR-based method to detect CRISPR genome mutation events and how the accuracy of this method is much superior to EMCA assays.

### EXAMPLE 11

This example demonstrates the effect of certain mutations on the activity of *P.a.* RNase H2 enzyme. In particular, the mutation G12A has limited effect on mismatch discrimination when the mismatch is located opposite the RNA, while the mutation P13T has no effect.

Towards identifying mutant RNase H2 enzymes with the desired properties, a collection of *P.a.* RNase H2 mutants were developed. Among these, mutations G12A and P13T were tested and found to confer increased specificity. Similar mutations in RNase H2 enzymes from other species that support rhPCR may likewise show similar improved properties relative to their WT forms.

6x-His-tagged-mutant and WT *P.a.* RNase H2 proteins were generated by standard site-directed mutagenesis (SDM) techniques (see, e.g., Weiner M. et al., Gene, 151:119-123 (1994)). The primers used for SDM of the *P.a.* RNase H2 enzyme are shown in Table 21. The mutagenized proteins were sequence verified and expressed in *E. coli* using standard methods. Purification was done by affinity purification over a charged Ni²⁺ column, as described in Dobosy et al. (2011) and US Patent No. 8,911,948 B2). Final amino acid sequences for the mutagenized proteins are shown in Table 22. After expression and purification, specific activity and unit definitions were determined using previously described techniques (US patent 8,911,948 B2).

**Table 21. SDM primers for mutagenesis of the RNase H2 enzymes used in Example 11.**

| **Primer name** | **SEQ ID NO:** | **Specific AA changes** | **Sequence** |
|---|---|---|---|
| G12A Forward Oligo | 193 | G12A | TGCAGGTGCAGATGAAGCTGGTCGTGCCC CAGTTATTGGTCCGCTGGTTATTG |
| G12A Reverse Oligo | 194 | G12A | CAATAACCAGCGGACCAATAACTGGGGC ACGACCAGCTTCATCTGCACCTGCA |
| P13T Forward Oligo | 195 | P13T | AGGTGCAGATGAAGCTGGTCGTGGTACG GTTATTGGTCCGCTGGTTATTGTTG |
| P13T Reverse Oligo | 196 | P13T | CAACAATAACCAGCGGACCAATAACCGT ACCACGACCAGCTTCATCTGCACCT |

All bases are DNA. Characters shown in bold are the mutagenic nucleotides.

**Table 22. Amino acid sequences for the RNase H2 proteins in Example 11.**

| **Mut ID #** | **SEQ ID NO:** | **Specific AA changes** | **Sequence** |
|---|---|---|---|
| N/A | 197 | WT RNase H2 | MKVAGADEAGRGPVIGPLVIVAAVVEEDKI RSLTKLGVKDSKQLTPAQREKLFDEIVKVL DDYSVVIVSPQDIDGRKGSMNELEVENFVK ALNSLKVKPEVIYIDSADVKAERFAENIRSR LAYEAKVVAEHKADAKYEIVSAASILAKVI |
| | | | RDREIEKLKAEYGDFGSGYPSDPRTKKWLE EWYSKHGNFPPIVRRTWDTAKKIEEKFKRA QLTLDNFLKRFRN |
| 1 | 198 | G12A | MKVAGADEAGRAPVIGPLVIVAAVVEEDKI RSLTKLGVKDSKQLTPAQREKLFDEIVKVL DDYSVVIVSPQDIDGRKGSMNELEVENFVK ALNSLKVKPEVIYIDSADVKAERFAENIRSR LAYEAKVVAEHKADAKYEIVSAASILAKVI RDREIEKLKAEYGDFGSGYPSDPRTKKWLE EWYSKHGNFPPIVRRTWDTAKKIEEKFKRA QLTLDNFLKRFRN |
| 2 | 199 | P13T | MKVAGADEAGRGTVIGPLVIVAAVVEEDKI RSLTKLGVKDSKQLTPAQREKLFDEIVKVL DDYSVVIVSPQDIDGRKGSMNELEVENFVK ALNSLKVKPEVIYIDSADVKAERFAENIRSR LAYEAKVVAEHKADAKYEIVSAASILAKVI RDREIEKLKAEYGDFGSGYPSDPRTKKWLE EWYSKHGNFPPIVRRTWDTAKKIEEKFKRA QLTLDNFLKRFRN |

Location of mutations are shown in bold and underlined typeface.

To determine whether these mutant RNase H2 enzymes increase mismatch discrimination when the mismatch is opposite the RNA, 1^{st} generation quantitative rhPCR assays were utilized against the rs4939827 SNP present in SMAD7. This SNP has been utilized in the past (Dobosy et al, 2011, and US patent 8,911,948 B2) to characterize rhPCR efficiency and specificity, and its response under differing conditions is well understood. The primers utilized in these assays are shown in Table 23, SEQ ID NOs: 200-203. Assays were done in 10 µL reaction volumes. Thermal cycling and data collection was performed with a CFX384^{®} Real Time System (Bio-Rad^{®}, Hercules, CA). Briefly, 1x of the iQ^{™} SYBR^{®} Green Supermix^{®} was utilized in combination with 200 nM (2 pmol) of either the blocked forward primer (SEQ ID NOs: 202 or 203) and 200 nM (2 pmol) of the unblocked reverse primer (SEQ ID NO: 201), or 200 nM (2 pmol) of unblocked control forward primer (SEQ ID NO: 200) and 200 nM (2 pmol) of unblocked reverse primer (SEQ ID NO: 201). 5 mU of either WT (SEQ ID NO: 197) or each mutant RNase H2 enzyme (SEQ ID NOs: 198 or 199) was added to each reaction. 10 ng of genomic cell line DNA (cell lines NA1852 and NA18537, Coriell Institute for Medical Research, Camden, NJ), representing the two homozygous genotypes at the rs4939827 SNP, was included in each reaction. Reactions were cycled under the following conditions 95 °C^{3:00} → (95 °C^{0:10} → 60 °C^{0:30}) x 85. Fluorescence data was collected after each extension time point. After the reaction was completed, data was analyzed, and the average Cq values for each point was calculated. The results are presented in Table 24.

**Table 23. Sequences and SEQ IDs for the primers used in the experiment described in Example 11.**

| **SEQ ID NO:** | **Name** | **Sequence** |
|---|---|---|
| 200 | rs4939827 | CAGCCTCATCCAAAAGAGGAAA |
| | unblocked | |
| 201 | rs4939827 Rev | CTCACTCTAAACCCCAGCATT |
| | unblocked | |
| 202 | rs4939827 rC | CAGCCTCATCCAAAAGAGGAAAcAGGA-x |
| | 4dmx | |
| 203 | rs4939827 rU | CAGCCTCATCCAAAAGAGGAAAuAGGA-x |
| | 4dmx | |

DNA is uppercase, RNA is lowercase. x = C3 spacer (propanediol) blocker group.

**Table 24. Cq and ΔCq values for the experiment described in Example 11.**

| **RNase H2 enzyme** | **Primer name** | **Forward primer SEQ ID NO** | **CC template** | **TT template** | **ΔCq** |
|---|---|---|---|---|---|
| WT | Unblocked | 200 | 23.2 | 23.1 | |
| | rC 4dmx | 202 | 24.6 | 34.6 | 10.0 |
| | rU 4dmx | 203 | 36.3 | 23.5 | 12.8 |
| P13T (Mut ID 1) | Unblocked | 200 | 23.8 | 23.0 | |
| | rC 4dmx | 202 | 25.2 | 35.2 | 10.0 |
| | rU 4dmx | 203 | 39.0 | 26.0 | 13.0 |
| G12A (Mut ID 2) | Unblocked | 200 | 21.6 | 21.7 | |
| | rC 4dmx | 202 | 64.8 | 81.6 | 16.9 |
| | rU 4dmx | 203 | 54.6 | 41.5 | 13.1 |

These data show that these mutations do not significantly increase mismatch discrimination when the SNP is located immediately opposite the RNA residue in the rhPCR primer. The possible exception of the G12A mutation is noted, but insufficient amount of RNase H2 was utilized in this experiment resulting in inefficient cleavage of primers and a severely delayed amplification compared to the match template.

### EXAMPLE 12

This example demonstrates the effect of certain mutations on the activity of *P.a.* RNase H2 enzyme. In particular, mutations G12A and P13T increase mismatch discrimination when the mismatch is placed 5' of the RNA nucleotide.

To determine whether mismatch discrimination can be increased with the G12A and P13T mutant RNase H2 enzymes when the mismatch is located 5' of the RNA nucleotide, assays targeting rs113488022, the V600E SNP in the human BRAF gene were designed with the mismatch located 5' of the RNA. The primers utilized in these assays are shown in Table 25, SEQ ID NOs: 204-207. Assays were performed in 10 µL reaction volumes. Thermal cycling and data collection were carried out with a CFX384^{®} Real Time System (Bio-Rad^{®}, Hercules, CA). Briefly, 1x of the iQ^{™} SYBR^{®} Green Supermix^{®} was utilized in combination with 200 nM (2 pmol) of either the blocked forward primer (SEQ ID NOs: 206 or 207) and 200 nM (2 pmol) of the unblocked reverse primer (SEQ ID NO: 205), or 200 nM (2 pmol) of unblocked control forward primer (SEQ ID NO: 204) and 200 nM (2 pmol) of unblocked reverse primer (SEQ ID NO: 205). 10 mU or 25 mU of either WT (SEQ ID NO: 197) or each mutant RNase H2 enzyme (SEQ ID NOs: 198 or 199) was added to each reaction. The 2,000 copies of synthetic double-stranded gBlock^{®} (IDT, Coralville, IA) template, representing the two homozygous genotypes at the rs113488022 SNP (SEQ ID NOs: 208 or 209), were also added to each reaction. Samples were cycled under the following conditions 95 °C^{3:00} → (95 °C^{0:10} → 60 °C^{0:30}) x 85. Fluorescence intensity was collected after each 60 °C extension step. The Cq values were averaged from 3 replicates. The results are shown in Table 26.

**Table 25. Sequences and SEQ ID NOs for the primers and templates used in the experiment described in Example 12.**

| **SEQ ID NO:** | **Name** | **Sequence** |
|---|---|---|
| 204 | rs113488022 | GTGATTTTGGTCTAGCTACAGT |
| | unblocked | |
| 205 | rs113488022 | CCTCAATTCTTACCATCCACAAA |
| | Rev unblocked | |
| 206 | rs113488022 | GTGATTTTGGTCTAGCTACAGTgAAATG-x |
| | TrG 4dmx | |
| 207 | rs113488022 | GTGATTTTGGTCTAGCTACAGAgAAATG-x |
| | ArG 4dmx | |
| 208 | gBlock^{®} T template | TAAGAGGAAAGATGAAGTACTATGTTTTAAA GAATATTATATTACAGAATTATAGAAATTAG ATCTCTTACCTAAACTCTTCATAATGCTTGCT CTGATAGGAAAATGAGATCTACTGTTTTCCTT TACTTACTACACCTCAGATATATTTCTTCATG AAGACCTCACAGTAAAAATAGGTGATTTTGG TCTAGCTACAGTGAAATCTCGATGGAGTGGG TCCCATCAGTTTGAACAGTTGTCTGGATCCAT TTTGTGGATGGTAAGAATTGAGGCTATTTTTC CACTGATTAAATTTTTGGCCCTGAGATGCTGC TGAGTTACTAGAAAGTCATTGAAGGTCTCAA CTATAGTATTTTCATAGTTCCCAGTATTCACA AAAATCAGTGTTCTTATTTTTT |
| 209 | gBlock^{®} A template | TAAGAGGAAAGATGAAGTACTATGTTTTAAA GAATATTATATTACAGAATTATAGAAATTAG ATCTCTTACCTAAACTCTTCATAATGCTTGCT CTGATAGGAAAATGAGATCTACTGTTTTCCTT TACTTACTACACCTCAGATATATTTCTTCATG AAGACCTCACAGTAAAAATAGGTGATTTTGG TCTAGCTACAGAGAAATCTCGATGGAGTGGG TCCCATCAGTTTGAACAGTTGTCTGGATCCAT TTTGTGGATGGTAAGAATTGAGGCTATTTTTC CACTGATTAAATTTTTGGCCCTGAGATGCTGC TGAGTTACTAGAAAGTCATTGAAGGTCTCAA |
| | | CTATAGTATTTCATAGTTCCCAGTATTCACA AAAATCAGTGTTCTTATTTTTT |

DNA is uppercase, RNA is lowercase. X = C3 spacer (propanediol) blocker group. Location of the mismatch is shown in bold and underlined in the gBlocks^{®}.

**Table 26. Cq and ΔCq values for the experiments in Example 12.**

| **RNase H2** | **Forward Primer** | **10 mU RNase H2** | | | **25 mU RNase H2** | | |
|---|---|---|---|---|---|---|---|
| | | **T template** | **A template** | **ΔCq** | **T template** | **A template** | **ΔCq** |
| WT | Unblocked | 26.2 | 25.6 | | 26.0 | 26.0 | |
| | TrG | 26.8 | 29.1 | 2.3 | 26.9 | 29.0 | 2.1 |
| | ArG | 37.2 | 26.8 | 10.4 | 36.1 | 26.8 | 9.3 |
| P13T | Unblocked | 26.2 | 25.8 | | 25.8 | 25.8 | |
| | TrG | 27.2 | 31.4 | 4.2 | 26.5 | 30.0 | 3.4 |
| | ArG | 39.0 | 27.0 | 12.0 | 38.8 | 26.9 | 11.9 |
| G12A | Unblocked | 26.1 | 26.0 | | 26.0 | 26.0 | |
| | TrG | 28.6 | 36.5 | 7.9 | 27.1 | 33.2 | 6.2 |
| | ArG | 42.1 | 30.7 | 11.4 | 38.8 | 27.7 | 11.1 |

These data show that the P13T RNase H2 mutant improved the ΔCq quantification of the mismatch discrimination with the TrG primer from 2.3 cycles with WT to 4.3 cycles (with 10 mU RNase H2). The P13T RNase H2 also improved mismatch discrimination with the ArG primer, increasing the ΔCq from 10.4 to 12.0 cycles. While modest, these changes can be significant in sensitive assays. The data also shows that the G12A RNase H2 mutant improved the ΔCq quantification of the mismatch discrimination with the TrG primer from 2.3 cycles with WT (with 10 mU RNase H2) to 6.2 cycles (with 25 mU RNase H2). The G12A RNase H2 also improved mismatch discrimination with the ArG primer, increasing the ΔCq from 10.4 to 11.4 cycles. The change with the ArG primer is less important here, as it already was effective. Both of these mutations therefore improve mismatch discrimination when the mismatch is located 5' of the RNA nucleotide.

### EXAMPLE 13

This example demonstrates the effect of certain mutations on the activity of *P.a.* RNase H2 enzyme. In particular, mutations G12A and P13T increase mismatch discrimination when the mismatch is placed 3' of the RNA.

To determine whether mismatch discrimination can be increased with the G12A and P13T mutant RNase H2 enzymes when the mismatch is located 3' of the RNA, assays targeting rs113488022, the V600E SNP in the human BRAF gene were designed with the mismatch located 3' of the RNA. The primers utilized in these assays are shown in Table 27, SEQ ID NOs: 204, 205, 210, and 211. Assays were conducted in 10 µL reaction volumes. Thermal cycling and data collection was performed with a CFX384^{®} Real Time System (Bio-Rad^{®}, Hercules, CA). Briefly, 1x of the iQ^{™} SYBR^{®} Green Supermix^{®} was utilized in combination with 200 nM (2 pmol) of either the blocked forward primer (SEQ ID NOs: 210 or 211) and 200 nM (2 pmol) of the unblocked reverse primer (SEQ ID NO: 205), or 200 nM (2 pmol) of unblocked control forward primer (SEQ ID NO: 204) and 200 nM (2 pmol) of unblocked reverse primer (SEQ ID NO: 205). 5 mU of either WT (SEQ ID NO: 197) or each mutant RNase H2 enzyme (SEQ ID NOs: 198 or 199) was added to each reaction. The 2,000 copies of synthetic double-stranded gBlock^{®} (IDT, Coralville, IA) template, representing the two homozygous genotypes at the rs113488022 SNP (SEQ ID NOs: 208 or 209), were also added to each reaction. Reactions were cycled under the following conditions 95 °C^{3:00} → (95 °C^{0:10} → 60 °C^{0:30}) x 85. Fluorescence intensity was collected after each extension step. The average Cq values were averaged from 3 replicates. The results are shown in Table 28.

**Table 27. Sequences and SEQ IDs for the primers and templates used in the experiment described in Example 13.**

| **SEQ ID NO:** | **Name** | **Sequence** |
|---|---|---|
| 204 | rs113488022 | GTGATTTTGGTCTAGCTACAGT |
| | unblocked | |
| 205 | rs113488022 | CCTCAATTCTTACCATCCACAAA |
| | Rev unblocked | |
| 210 | rs113488022 | GTGATTTTGGTCTAGCTACArGTAAAA-x |
| | rGT 4dmx | |
| 211 | rs113488022 | GTGATTTTGGTCTAGCTACArGTAAAA-x |
| | rGA 4dmx | |

DNA is uppercase, RNA is lowercase. X = C3 spacer (propanediol) blocker group.

**Table 28. Cq and ΔCq values for the experiments in Example 13.**

| **RNase H2** | **Forward Primer** | **10 mU RNase H2** | | | **25 mU RNase H2** | | |
|---|---|---|---|---|---|---|---|
| | | **T template** | **A template** | **ΔCq** | **T template** | **A template** | **ΔCq** |
| WT | Unblocked | 26.4 | 26.0 | | 25.9 | 26.0 | |
| | rGT 4dmx | 26.4 | 26.5 | 0.2 | 25.7 | 26.3 | 0.6 |
| | rGA 4dmx | 33.3 | 26.0 | 7.3 | 31.3 | 25.9 | 5.3 |
| P13T | Unblocked | 25.8 | 25.7 | | 25.5 | 25.8 | |
| | rGT 4dmx | 31.7 | 37.3 | 5.6 | 26.1 | 30.6 | 4.5 |
| | rGA 4dmx | 42.0 | 27.0 | 15.0 | 41.4 | 26.4 | 15.0 |
| G12A | Unblocked | 25.8 | 25.8 | | 25.7 | 25.9 | |
| | rGT 4dmx | 55.7 | 66.8 | 11.1 | 41.7 | 50.3 | 8.6 |
| | rGA 4dmx | 75.6 | 48.2 | 27.4 | 58.0 | 35.4 | 22.5 |

These data show that the P13T RNase H2 mutant improved the ΔCq discrimination with the rGT primer from 0.2 cycles (seen for WT) to 4.5 cycles (seen for mutant at 10 mU RNase H2). The P13T RNase H2 also improved mismatch discrimination with the rGA primer, increasing the ΔCq from 7.3 to 15.0 cycles. These improvements turned a failed assay into successful one, in particular when the rGT primer was employed. Furthermore, the G12A RNase H2 mutant improved the ΔCq of the mismatch discrimination with the rGT primer from 0.2 cycles to 8.6 cycles. The G12A RNase H2 also improved mismatch discrimination with the rGA primer, increasing the ΔCq from 7.3 to 22.5 cycles. This change came at the cost of some amplification efficiency, but this would likely be overcome with the addition of more G12A mutant RNase H2 enzyme. In conclusion, both of these mutations improve mismatch discrimination when the mismatch is located 3' of the RNA nucleotide.

### EXAMPLE 14

This example demonstrates the effect of certain mutations on the activity of *P.a.* RNase H2 enzyme. In particular, mutation G169A of *P.a.* RNase H2 has no effect on mismatch discrimination when the mismatch is located opposite the RNA.

Towards identifying mutant RNase H2 enzymes with the desired properties, an additional *P.a.* RNase H2 mutant was developed with by substituting glycine with alanine at position 169 (G169A). Similar mutations in RNase H2 enzymes from other species that support rhPCR may likewise show similar improved properties relative to their WT forms.

6x-His-tagged-mutant and WT *P.a.* RNase H2 proteins were generated by standard site-directed mutagenesis (SDM) techniques (see, e.g., Weiner M. et al., Gene, 151:119-123 (1994)). The primers used for SDM of the *P.a.* RNase H2 enzyme are shown in Table 29. The mutagenized proteins were sequence verified and expressed in *E. coli* using standard methods. Purification was done by affinity purification over a charged Ni²⁺ column, as described in Dobosy et al. (2011) and US Patent No. 8,911,948 B2). Final amino acid sequences for the mutagenized proteins are shown in Table 30. After expression and purification, specific activity and unit definitions were determined using previously described techniques (US patent 8,911,948 B2).

**Table 29. SDM primers for mutagenesis of the RNase H2 enzymes used in Example 14.**

| **Primer name** | **SEQ ID NO:** | **Specific AA changes** | **Sequence** |
|---|---|---|---|
| G169A Forward Oligo | 212 | G169A | AGCCGAATACGGTGATTTTGGTTCCGCAT ACCCGTCTGATCCGCGTACTAAGA |
| G169A Reverse Oligo | 213 | G169A | TCTTAGTACGCGGATCAGACGGGTATGCG GAACCAAAATCACCGTATTCGGCT |

All bases are DNA. Characters shown in bold are the mutagenic nucleotides.

**Table 30. Amino acid sequences for the RNase H2 proteins in Example 14.**

| **Mut ID #** | **SEQ ID NO:** | **Specific AA changes** | **Sequence** |
|---|---|---|---|
| N/A | 197 | WT RNase H2 | MKVAGADEAGRGPVIGPLVIVAAVVEEDKI RSLTKLGVKDSKQLTPAQREKLFDEIVKVL DDYSVVIVSPQDIDGRKGSMNELEVENFVK ALNSLKVKPEVIYIDSADVKAERFAENIRSR LAYEAKVVAEHKADAKYEIVSAASILAKVI RDREIEKLKAEYGDFGSGYPSDPRTKKWLE EWYSKHGNFPPIVRRTWDTAKKIEEKFKRA QLTLDNFLKRFRN |
| 3 | 214 | G169A | MKVAGADEAGRGPVIGPLVIVAAVVEEDKI RSLTKLGVKDSKQLTPAQREKLFDEIVKVL DDYSVVIVSPQDIDGRKGSMNELEVENFVK ALNSLKVKPEVIYIDSADVKAERFAENIRSR LAYEAKVVAEHKADAKYEIVSAASILAKVI RDREIEKLKAEYGDFGSAYPSDPRTKKWLE EWYSKHGNFPPIVRRTWDTAKKIEEKFKRA QLTLDNFLKRFRN |

Location of mutations are shown in bold and underlined typeface.

To determine whether this mutant RNase H2 enzyme increases mismatch discrimination when the mismatch is opposite the RNA, quantitative rhPCR assays were utilized against the rs4939827 SNP present in SMAD7. This SNP has been utilized in the past (Dobosy et al, 2011, and US patent 8,911,948 B2) to characterize rhPCR efficiency and specificity, and its response under differing conditions is well understood. The primers utilized in these assays are shown in Table 31, SEQ ID NOs: 215-218. Assays were done in 10 µL reaction volumes. Thermal cycling and data collection was performed with a CFX384^{®} Real Time System (Bio-Rad^{®}, Hercules, CA). Briefly, 1x of the iQ^{™} SYBR^{®} Green Supermix^{®} was utilized in combination with 200 nM (2 pmol) of either the blocked forward primer (SEQ ID NOs: 217 or 218) and 200 nM (2 pmol) of the unblocked reverse primer (SEQ ID NO: 216), or 200 nM (2 pmol) of unblocked control forward primer (SEQ ID NO: 215) and 200 nM (2 pmol) of unblocked reverse primer (SEQ ID NO: 216). 5 mU of either WT (SEQ ID NO: 197) or G169 mutant RNase H2 enzyme (SEQ ID NO: 214) was added to each reaction. 10 ng of genomic cell line DNA (cell lines NA1852 and NA1853 7, Coriell Institute for Medical Research, Camden, NJ), representing the two homozygous genotypes at the rs4939827 SNP, was included in each reaction. Reactions were cycled under the following conditions 95 °C^{3:00} → (95 °C^{0:10} → 60 °C^{0:30}) x 85. Fluorescence data was collected after each extension time point. After the reaction was completed, data was analyzed, and the average Cq values for each point was calculated. The results are presented in Table 24.

**Table 31. Sequences and SEQ IDs for the primers used in the experiment described in Example 14.**

| **SEQ ID NO:** | **Name** | **Sequence** |
|---|---|---|
| 215 | rs4939827 unblocked | CAGCCTCATCCAAAAGAGGAAA |
| 216 | rs4939827 Rev unblocked | CTCACTCTAAACCCCAGCATT |
| 217 | rs4939827 rC 4dmx | CAGCCTCATCCAAAAGAGGAAAcAGGA-x |
| 218 | rs4939827 rU 4dmx | CAGCCTCATCCAAAAGAGGAAAuAGGA-x |

DNA is uppercase, RNA is lowercase. x = C3 spacer (propanediol) blocker group.

**Table 32. Cq and ΔCq values for the experiment described in Example 14.**

| **RNase H2 enzyme** | **Primer name** | **Forward primer SEQ ID NO** | **CC template** | **TT template** | **ΔCq** |
|---|---|---|---|---|---|
| WT | Unblocked | 7 | 22.4 | 23.0 | |
| | rC 4dmx | 9 | 22.9 | 34.3 | 11.5 |
| | rU 4dmx | 10 | 32.6 | 23.3 | 9.3 |
| G169A (Mut ID 3) | Unblocked | 7 | 22.2 | 22.0 | |
| | rC 4dmx | 9 | 23.2 | 31.1 | 8.0 |
| | rU 4dmx | 10 | 39.2 | 27.5 | 11.8 |

These data show that these mutations do not significantly increase mismatch discrimination when the SNP is located immediately opposite the RNA residue in the rhPCR primer.

### EXAMPLE 15

This example demonstrates the effect of certain mutations on the activity of *P.a.* RNase H2 enzyme. In particular, mutation of *P.a.* RNase H2 at G169A increases mismatch discrimination when the mismatch is placed 5' of the RNA nucleotide.

To determine whether mismatch discrimination can be increased with the G169A mutant RNase H2 enzyme when the mismatch is located 5' of the RNA nucleotide, assays targeting rs113488022, the V600E SNP in the human BRAF gene were designed with the mismatch located 5' of the RNA. The primers utilized in these assays are shown in Table 27, SEQ ID NOs: 204-207. Assays were performed in 10 µL reaction volumes. Thermal cycling and data collection were carried out with a CFX384^{®} Real Time System (Bio-Rad^{®}, Hercules, CA). Briefly, 1x of the iQ^{™} SYBR^{®} Green Supermix^{®} was utilized in combination with 200 nM (2 pmol) of either the blocked forward primer (SEQ ID NOs: 206 or 207) and 200 nM (2 pmol) of the unblocked reverse primer (SEQ ID NO: 205), or 200 nM (2 pmol) of unblocked control forward primer (SEQ ID NO: 204) and 200 nM (2 pmol) of unblocked reverse primer (SEQ ID NO: 205). 25 mU of either WT (SEQ ID NO: 197) or G169 mutant RNase H2 enzyme (SEQ ID NO: 214) was added to each reaction. The 2,000 copies of synthetic double-stranded gBlock^{®} (IDT, Coralville, IA) template, representing the two homozygous genotypes at the rs113488022 SNP (SEQ ID NOs: 208 or 209), were also added to each reaction. Reactions were cycled under the following conditions 95 °C^{3:00} → (95 °C^{0:10} → 60 °C^{0:30}) x 85. Fluorescence intensity was collected after each extension step. The average Cq values were averaged from 3 replicates. The results are shown in Table 33.

**Table 33. Cq and ΔCq values for the experiments in Example 15.**

| **RNase H2** | **Forward Primer** | **10 mU RNase H2** | | | **25 mU RNase H2** | | |
|---|---|---|---|---|---|---|---|
| | | **T template** | **A template** | **ΔCq** | **T template** | **A template** | **ΔCq** |
| WT | Unblocked | 26.8 | 25.9 | | 26.1 | 25.8 | |
| | rGT 4dmx | 28.1 | 28.1 | 0.0 | 25.6 | 25.9 | 0.3 |
| | rGA 4dmx | 40.5 | 26.3 | 14.2 | 28.1 | 25.6 | 2.5 |
| G169A | Unblocked | 26.2 | 26.0 | | 26.9 | 26.7 | |
| | rGT 4dmx | 29.2 | 39.3 | 10.1 | 26.1 | 27.3 | 1.2 |
| | rGA 4dmx | 43.2 | 26.4 | 16.8 | 31.7 | 25.9 | 5.9 |

These data show that the G169A RNase H2 mutant improved the ΔCq discrimination with the rGT primer from 0.0 cycles (seen for WT) to 10.1 cycles (seen for mutant at 5 mU RNase H2). The G169A RNase H2 also slightly improved mismatch discrimination with the rGA primer, increasing the ΔCq from 14.2 to 16.8 cycles. These improvements turned failed assay into successful one, in particular when the rGT primer was employed. In conclusion, this mutation improves mismatch discrimination when the mismatch is located 3' of the RNA nucleotide.

The RNase H2 mutations examined in Examples 11-15 enhanced mismatch discrimination more when the mutation was located 5' or 3' of the RNA than when located opposite of the RNA nucleotide. Together, these results suggest that limitations of mutation discrimination of rhPCR are based not only on the ability of the RNase H2 to recognize the mismatch and the ability of a polymerase to extend the cleaved primer, but also from the conversion of the mismatched template to the primer sequence. This conversion masks the ability of the RNase H2 enzyme to recognize the mismatch when the mismatch is located at the RNA nucleotide. This could happen in several ways. Most (if not all) conversions spring from a cleavage occurring on the 3' side of the mismatch. Once this type of cleavage occurs, it can be extended by the DNA polymerase, after which the newly generated template fully matches the primers. The converted template then contaminates the sample because it is expected to be amplified in subsequent cycles.

RNase H2 usually cleaves strand at 5' side of RNA nucleotide. Experiments (not shown) have indicated that the rare cleavage on the 3' side of RNA nucleotide occurs at a rate of approximately 1 per 1,000 cleavage reactions. If the mismatch is located at the RNA site, such rare cleavage will cause template conversion. Introducing the mismatch 5' of the RNA nucleotide makes conversion of the template to the primer sequence occur in the first cycle of replication. In this case, mismatch discrimination depends solely on the ability of the RNase H2 to recognize the mismatch and the ability of polymerase to extend the mismatched primer, since the polymerase is expected to be inhibited by a mismatch at the 3' end of a primer.

On the other hand, placing the mismatch 3' of the RNA nucleotide reduces (or completely eliminates) the template conversion. Again, in this position, mismatch discrimination depends solely on the ability of RNase H2 to recognize the mismatch and the ability of the polymerase to extend the mismatched primer. However, the polymerase is not expected to be inhibited in this case since the mismatch site is cleaved out of the primer. The results may also have arisen because the mutations revealed here are more susceptible to mutations present 5' and 3' of the RNA than they are opposite the RNA. These hypotheses are not intended to limit the scope of the invention.

### EXAMPLE 16

This example demonstrates the effect of certain mutations on the activity of *P.a.* RNase H2 enzyme. In particular, mutations G12A and P13T increase mismatch discrimination when the mismatch is placed 3' of the RNA with multiple SNPs.

To determine whether mismatch discrimination can be increased with the G12A and P13T mutant RNase H2 enzymes when the mismatch is located 3' of the RNA, assays targeting rs12744607, rs60118785, rs60607502, rs7992897, rs3117947, rs7583169, rs4853850, rs28520334, rs13316719, rs61514082, rs12908737, rs6572257, rs2242527, rs1076939, rs35593667, and rs6046375 were designed with the mismatch located 3' of the RNA. Assays were conducted in 10 µL reaction volumes. Thermal cycling and data collection was performed with a CFX384^{™} Real-Time System (Bio-Rad^{®}, Hercules, CA). Briefly, 1x of the iQ^{™} SYBR^{®} Green Supermix^{®} was utilized in combination with 200 nM (2 pmol) of either the blocked forward primer and 200 nM (2 pmol) of the unblocked reverse primer. 10 mU of either WT (SEQ ID NO: 197) or 20 mU P13T mutant RNase H2 enzyme (SEQ ID NOs: 198 or 199) or 50 mU G12A mutant RNase H2 enzyme (SEQ ID NO: 199) was added to each reaction. The 10 ng of NA12878 or NA24385 genomic cell line DNA, representing the two homozygous genotypes at each of these SNPs, were also added to each reaction. Reactions were cycled under the following conditions 95 °C^{3:00} → (95 °C^{0:10} → 60 °C^{0:30}) x 85 cycles. Fluorescence intensity was collected after each extension step. The average Cq values were averaged from 3 replicates. The results are shown in Table 34.

**Table 34. Cq and ΔCq values for the experiments in Example 16.**

| | WT RN2 | | | P13T | | | G12A | | |
|---|---|---|---|---|---|---|---|---|---|
| | NA12878 | NA24385 | ΔC 9 | NA12878 | NA24385 | ΔC 9 | NA12878 | NA24385 | ΔCq |
| rs12744607 | >85 | >85 | N/A | 73.0 | >85 | 12. 0 | 38.2 | 61.5 | 23.3 |
| rs13316719 | 27.5 | 25.3 | 2.2 | 26.4 | 25.5 | 1.0 | 26.4 | 27.0 | -0.7 |
| rs60118785 | 25.6 | 37.8 | 12.3 | 40.8 | 63.7 | 22. 9 | 26.7 | 37.6 | 11.0 |
| rs61514082 | 24.7 | 27.2 | 2.5 | 25.8 | 52.5 | 26. 7 | 26.0 | 36.5 | 10.5 |
| rs60607502 | 25.4 | 25.5 | 0.0 | 25.9 | 34.8 | 8.9 | 29.8 | 44.0 | 14.1 |
| rs12908737 | 34.7 | 70.6 | 35.9 | 75.3 | >85 | 9.7 | 35.7 | 52.9 | 17.2 |
| rs7992897 | 25.1 | 27.9 | 2.8 | 24.5 | 24.4 | 0.1 | 25.9 | 30.7 | 4.8 |
| rs6572257 | >85 | 71.5 | 13.5 | 71.3 | 40.9 | 30. 4 | 49.2 | 27.7 | 21.5 |
| rs3117947 | 47.3 | 62.4 | 15.1 | 33.3 | 43.5 | 10. 2 | 25.6 | 26.3 | 0.6 |
| rs2242527 | 29.4 | 47.9 | 18.5 | 39.2 | 67.7 | 28. 5 | 32.6 | 50.5 | 17.9 |
| rs7583169 | 27.6 | 26.0 | 1.6 | 31.6 | 35.7 | 4.0 | 29.1 | 41.8 | 12.7 |
| rs1076939 | 33.7 | 40.2 | 6.5 | 34.1 | 59.6 | 25. 5 | 36.3 | 49.9 | 13.6 |
| rs4853850 | 30.2 | 31.9 | 1.7 | 36.6 | 56.1 | 19. 5 | 29.3 | 34.0 | 4.7 |
| rs35593667 | 31.7 | 34.5 | 2.7 | 42.3 | 64.9 | 22. 7 | 38.3 | 73.2 | 34.9 |
| rs28520334 | 26.1 | 25.1 | 1.0 | 24.9 | 24.3 | 0.6 | 24.7 | 25.0 | 0.3 |
| rs6046375 | >85 | 61.1 | 24.0 | 80.4 | 46.1 | 34. 3 | 53.1 | 28.2 | 24.9 |

These data show that the P13T RNase H2 mutant improved the ΔCq discrimination at multiple SNPs. An excellent example is rs61514082, where the ΔCq increased from 2.5 cycles (seen for WT) to 26.7 cycles (seen for mutant at 20 mU RNase H2). The P13T RNase H2 also improved mismatch discrimination at the rs60607502 SNP, increasing the ΔCq from 0.0 to 8.9 cycles. Other SNPs also improved their specificity. The two SNPs that did not improve ΔCq (rs13316719 and rs28520334) show that this mutation can be further improved, and does not solve all issues with all assays, despite a wide-ranging improvement being observed. These improvements turned many failed assays into successful ones, showing the broad improvements in ΔCq that this mutation confers.

The G12A RNase H2 mutant also improved the ΔCq of the mismatch discrimination at multiple SNPs. The rs61514082 SNP increased in ΔCq from 2.5 cycles to 10.5 cycles. The G12A RNase H2 also improved mismatch discrimination with the rs60607502 SNP, increasing the ΔCq from 0.0 to 14.1 cycles. In conclusion, both mutations improve mismatch discrimination when the mismatch is located 3' of the RNA nucleotide.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method for detecting on-target and predicted off-target genome editing events, the method comprising:
(a) providing a multiplex RNase H2-dependent PCR (rhPCR) reaction mixture comprising:
(i) an on-target oligonucleotide primer having a 5' tail sequence that comprises a universal primer sequence and optionally a universal probe sequence, wherein the tail is non-complementary to the target DNA sequence; a cleavage domain positioned 5' of a blocking group, the blocking group linked at or near the end of the 3'-end of the oligonucleotide primer and a complementary region flanking the on-target genome edited locus, wherein the blocking group prevents primer extension and/or inhibits the oligonucleotide primer from serving as a template for DNA synthesis;
(ii) one or more off-target oligonucleotide primers having a cleavage domain positioned 5' of a blocking group linked at or near the end of the 3'-end of the oligonucleotide primer and a complementary region flanking one or more predicted off-target genome edited loci, wherein the blocking group prevents primer extension and/or inhibits the oligonucleotide primer from serving as a template for DNA synthesis;
(iii) a sample nucleic acid comprising a target DNA sequence, wherein the target DNA sequence has been altered with a gene editing enzyme;
(iv) a cleaving enzyme, wherein the cleaving enzyme is an RNase H2 enzyme; and
(v) a polymerase, wherein the polymerase is a high-discrimination polymerase;
(b) hybridizing the on-target oligonucleotide primer to the on-target genome edited locus to form an on-target double-stranded substrate and hybridizing the one or more off-target oligonucleotide primers to the one or more predicted off-target genome edited loci to form one or more off-target double-stranded substrates;
(c) cleaving the on-target double stranded substrate with the cleaving enzyme at a point within or adjacent to the cleavage domain to remove the blocking group from the on-target oligonucleotide primer and cleaving the off-target double stranded substrate with the cleaving enzyme at a point within or adjacent to the cleavage domain to remove the blocking group from the off-target oligonucleotide primer; and
(d) extending the on-target oligonucleotide primer and the off-target oligonucleotide primer with the polymerase.

2. The method of claim 1, wherein the target DNA sequence has been altered with a CRISPR enzyme.

3. The method of claim 1, wherein the target DNA sequence has been altered with a Cas9 or Cpf1 enzyme.

4. The method of claim 1, wherein the cleaving enzyme is a hot start cleaving enzyme which is thermostable and has reduced activity at lower temperatures.

5. The method of claim 1, wherein the cleaving enzyme is *Pyrococcus abyssi* RNase H2 enzyme.

6. The method of claim 1, wherein the cleaving enzyme is a chemically modified hot start cleaving enzyme which is thermostable and has reduced activity at lower temperatures.

7. The method of claim 6, wherein the hot start cleaving enzyme is a chemically modified *Pyrococcus abyssi* RNase H2.

8. The method of claim 1, wherein the cleaving enzyme is a hot start cleaving enzyme that is reversibly inactivated through interaction with an antibody at lower temperatures.

9. The method of claim 1, wherein the cleavage domain comprises at least one RNA base, and the cleaving enzyme cleaves between the position complementary to the variation and the RNA base.

10. The method of claim 9, wherein the cleavage domain comprises:
(a) at least one RNA base located 3' of a position of variation; and
(b) one DNA base between the position of variation and the RNA base.

11. The method of claim 1, wherein the cleavage domain comprises one or more 2'-modified nucleosides, and the cleaving enzyme cleaves between the position complementary to a variation and the one or more 2'-modified nucleosides.

12. The method of claim 11, wherein the one or more modified nucleosides are 2'-fluoronucleosides.

13. The method of claim 1, wherein the polymerase is a mutant H784Q Taq polymerase.

14. The method of claim 13, wherein the mutant H784Q Taq polymerase is reversibly inactivated via chemical, aptamer, or antibody modification.

15. The method of claim 1, wherein the one or more off-target oligonucleotide primers contains a 5' tail sequence that comprises a universal primer sequence and optionally a universal probe sequence, wherein the tail is non-complementary to the target DNA sequence.

16. The method of claim 1, wherein the one or more off-target oligonucleotide primers are capable of detecting greater than 100 predicted off-target genome editing events.

## Patentansprüche

1. Verfahren zum Nachweisen von On-Target- und vorhergesagten Off-Target-Genomeditierungsereignissen, wobei das Verfahren umfasst:
(a) Bereitstellen einer Multiplex-RNase-H2-abhängigen PCR-(rhPCR-)Reaktionsmischung, umfassend:
(i) einen On-Target-Oligonukleotidprimer mit einer 5*'*-Schwanzsequenz, die eine universelle Primersequenz und optional eine universelle Sondensequenz umfasst, wobei der Schwanz nicht komplementär zur Ziel-DNA-Sequenz ist; eine Spaltungsdomäne, die 5*'* von einer Blockierungsgruppe angeordnet ist,
wobei die Blockierungsgruppe an oder nahe dem Ende des 3'-Endes des Oligonukleotidprimers gebunden ist und ein komplementärer Bereich den On-Target-genomeditieren Locus flankiert, wobei die Blockierungsgruppe eine Primerverlängerung verhindert und/oder den Oligonukleotidprimer daran hindert, als Matrize für eine DNA-Synthese zu dienen;
(ii) einen oder mehrere Off-Target-Oligonukleotidprimer mit einer Spaltungsdomäne, die 5*'*-seitig einer Blockierungsgruppe positioniert ist, die an oder nahe dem 3'-Ende des Oligonukleotidprimers gebunden ist, und einem komplementären Bereich, der einen oder mehrere vorhergesagte, genomeditierte Off-Target-Loci flankiert, wobei die Blockierungsgruppe eine Primerverlängerung verhindert und/oder den Oligonukleotidprimer daran hindert, als Matrize für eine DNA-Synthese zu dienen;
(iii) eine Probenukleinsäure, umfassend eine Ziel-DNA-Sequenz, wobei die Ziel-DNA-Sequenz durch ein Geneditierungsenzym verändert wurde;
(iv) ein Spaltungsenzym, wobei das Spaltungsenzym ein RNase-H2-Enzym ist; und
(v) eine Polymerase, wobei die Polymerase eine hochselektive Polymerase ist;
(b) Hybridisieren des On-Target-Oligonukleotidprimers an den genomeditieren On-Target-Locus, um ein doppelsträngiges On-Target-Substrat zu bilden, und Hybridisieren des einen oder der mehreren Off-Target-Oligonukleotidprimer an den einen oder die mehreren vorhergesagten genomeditieren Off-Target-Loci, um ein oder mehrere doppelsträngige Off-Target-Substrate zu bilden;
(c) Spalten des doppelsträngigen On-Target-Substrats mit dem Spaltungsenzym an einer Stelle innerhalb oder angrenzend an die Spaltungsdomäne, um die Blockierungsgruppe von dem On-Target-Oligonukleotidprimer zu entfernen, und Spalten des doppelsträngigen Off-Target-Substrats mit dem Spaltungsenzym an einer Stelle innerhalb oder angrenzend an die Spaltungsdomäne, um die Blockierungsgruppe von dem Off-Target-Oligonukleotidprimer zu entfernen; und
(d) Verlängern des On-Target-Oligonukleotidprimers und des Off-Target-Oligonukleotidprimers mit der Polymerase.

2. Verfahren nach Anspruch 1, wobei die Ziel-DNA-Sequenz mit einem CRISPR-Enzym verändert wurde.

3. Verfahren nach Anspruch 1, wobei die Ziel-DNA-Sequenz mit einem Cas9- oder Cpf1-Enzym verändert wurde.

4. Verfahren nach Anspruch 1, wobei das Spaltungsenzym ein Hot-Start-Spaltungsenzym ist, das thermostabil ist und bei niedrigeren Temperaturen eine verminderte Aktivität aufweist.

5. Verfahren nach Anspruch 1, wobei das Spaltungsenzym ein *Pyrococcus abyssi-*RNase H2-Enzym ist.

6. Verfahren nach Anspruch 1, wobei das Spaltungsenzym ein chemisch modifiziertes Hot-Start-Spaltungsenzym ist, das thermostabil ist und bei niedrigeren Temperaturen eine verminderte Aktivität aufweist.

7. Verfahren nach Anspruch 6, wobei das Hot-Start-Spaltungsenzym eine chemisch modifizierte *Pyrococcus abyssi*-RNase H2 ist.

8. Verfahren nach Anspruch 1, wobei das Spaltungsenzym ein Hot-Start-Spaltungsenzym ist, das durch Interaktion mit einem Antikörper bei niedrigeren Temperaturen reversibel inaktiviert wird.

9. Verfahren nach Anspruch 1, wobei die Spaltungsdomäne mindestens eine RNA-Base umfasst und das Spaltungsenzym zwischen der zur Variation komplementären Position und der RNA-Base spaltet.

10. Verfahren nach Anspruch 9, wobei die Spaltungsdomäne umfasst:
(a) mindestens eine RNA-Base, die 3' von einer Variationsposition angeordnet ist; und
(b) eine DNA-Base zwischen der Variationsposition und der RNA-Base.

11. Verfahren nach Anspruch 1, wobei die Spaltungsdomäne ein oder mehrere 2*'-*modifizierte Nukleoside umfasst, und das Spaltungsenzym zwischen der Position, die zu einer Variation komplementär ist, und dem einen oder den mehreren 2*'*-modifizierten Nukleosiden spaltet.

12. Verfahren nach Anspruch 11, wobei das eine oder die mehreren modifizierten Nukleoside 2*'*-Fluornukleoside sind.

13. Verfahren nach Anspruch 1, wobei die Polymerase eine mutierte H784Q-Taq-Polymerase ist.

14. Verfahren nach Anspruch 13, wobei die mutierte H784Q-Taq-Polymerase durch chemische, Aptamer- oder Antikörpermodifikation reversibel inaktiviert ist.

15. Verfahren nach Anspruch 1, wobei der eine oder die mehreren Off-Target-Oligonukleotidprimer eine 5*'*-Schwanzsequenz enthalten, die eine universelle Primersequenz und optional eine universelle Sondensequenz umfasst, wobei der Schwanz nicht komplementär zur Ziel-DNA-Sequenz ist.

16. Verfahren nach Anspruch 1, wobei der eine oder die mehreren Off-Target-Oligonukleotidprimer in der Lage sind, mehr als 100 vorhergesagte Off-Target-Genomeditierungsereignisse nachzuweisen.

## Revendications

1. Procédé de détection d'événements d'édition génomique sur cible et prédits hors cible, le procédé comprenant :
(a) la fourniture d'un mélange de réaction multiplex pour PCR dépendant de la RNase H2 (rhPCR) comprenant :
(i) une amorce d'oligonucléotide sur cible présentant une séquence de queue en 5*'* qui comprend une séquence d'amorce universelle et facultativement une séquence de sonde universelle, dans lequel la queue est non complémentaire de la séquence d'ADN cible ; un domaine de clivage positionné en 5*'* d'un groupe de blocage, le groupe de blocage étant lié au niveau ou à proximité de l'extrémité de l'extrémité 3' de l'amorce d'oligonucléotide et d'une région complémentaire flanquant le locus ayant fait l'objet d'une édition du génome cible ayant fait l'objet d'une édition du génome, dans lequel le groupe de blocage empêche une extension d'amorce et/ou interdit que l'amorce d'oligonucléotide serve de matrice pour une synthèse d'ADN ;
(ii) une ou plusieurs amorces d'oligonucléotide hors cible présentant un domaine de clivage positionné en 5*'* d'un groupe de blocage lié au niveau ou à proximité de l'extrémité de l'extrémité 3' de l'amorce d'oligonucléotide et d'une région complémentaire flanquant un ou plusieurs loci ayant fait l'objet d'une édition du génome hors cible prédits, dans lequel le groupe de blocage empêche une extension d'amorce et/ou interdit que l'amorce d'oligonucléotide serve de matrice pour une synthèse d'ADN ;
(iii) un échantillon d'acide nucléique comprenant une séquence d'ADN cible, dans lequel la séquence d'ADN cible a été altérée par une enzyme d'édition de gène ;
(iv) une enzyme de clivage, dans lequel l'enzyme de clivage est une enzyme RNase H2 ; et
(v) une polymérase, dans lequel la polymérase est une polymérase à haute discrimination ;
(b) l'hybridation de l'amorce d'oligonucléotide sur cible au locus ayant fait l'objet d'une édition du génome sur la cible pour former un substrat double brin sur cible et l'hybridation des une ou plusieurs amorces d'oligonucléotide hors cible aux un ou plusieurs loci ayant fait l'objet d'une édition du génome hors cible prédits pour former un ou plusieurs substrats double brin hors cible ;
(c) le clivage du substrat double brin sur cible avec l'enzyme de clivage à un point à l'intérieur du domaine de clivage, ou adjacent à celui-ci, pour retirer le groupe de blocage de l'amorce d'oligonucléotide sur cible et le clivage du substrat double brin hors cible avec l'enzyme de clivage à un point à l'intérieur d u domaine de clivage, ou adjacent à celui-ci, pour retirer le groupe de blocage de l'amorce d'oligonucléotide hors cible ; et
(d) l'extension de l'amorce d'oligonucléotide sur cible et de l'amorce d'oligonucléotide hors cible avec la polymérase.

2. Procédé selon la revendication 1, dans lequel la séquence d'ADN cible a été altérée par une enzyme CRISPR.

3. Procédé selon la revendication 1, dans lequel la séquence d'ADN cible a été altérée par une enzyme Cas9 ou Cpf1.

4. Procédé selon la revendication 1, dans lequel l'enzyme de clivage est une enzyme de clivage à démarrage à chaud qui est thermostable et présente une activité réduite à des températures inférieures.

5. Procédé selon la revendication 1, dans lequel l'enzyme de clivage est une enzyme RNase H2 de *Pyrococcus abyssi*.

6. Procédé selon la revendication 1, dans lequel l'enzyme de clivage est une enzyme de clivage à démarrage à chaud chimiquement modifiée qui est thermostable et présente une activité réduite à des températures inférieures.

7. Procédé selon la revendication 6, dans lequel l'enzyme de clivage à démarrage à chaud est une RNase H2 de *Pyrococcus abyssi* chimiquement modifiée.

8. Procédé selon la revendication 1, dans lequel l'enzyme de clivage est une enzyme de clivage à démarrage à chaud qui est inactivée de manière réversible par interaction avec un anticorps à des températures inférieures.

9. Procédé selon la revendication 1, dans lequel le domaine de clivage comprend au moins une base d'ARN, et l'enzyme de clivage clive entre la position complémentaire de la variation et la base d'ARN.

10. Procédé selon la revendication 9, dans lequel le domaine de clivage comprend :
(a) au moins une base d'ARN située en 3' d'une position de variation ; et
(b) une base d'ADN entre la position de variation et la base d'ARN.

11. Procédé selon la revendication 1, dans lequel le domaine de clivage comprend un ou plusieurs nucléosides modifiés en 2*'*, et l'enzyme de clivage clive entre la position complémentaire d'une variation et les un ou plusieurs nucléosides modifiés en 2*'*.

12. Procédé selon la revendication 11, dans lequel les un ou plusieurs nucléosides modifiés sont des 2*'*-fluoronucléosides.

13. Procédé selon la revendication 1, dans lequel la polymérase est une polymérase Taq H784Q mutante.

14. Procédé selon la revendication 13, dans lequel la polymérase Taq H784Q mutante est inactivée de manière réversible par modification chimique, par aptamère ou anticorps.

15. Procédé selon la revendication 1, dans lequel les une ou plusieurs amorces d'oligonucléotide hors cible contiennent une séquence de queue en 5*'* qui comprend une séquence d'amorce universelle et facultativement une séquence de sonde universelle, dans lequel la queue est non complémentaire de la séquence d'ADN cible.

16. Procédé selon la revendication 1, dans lequel les une ou plusieurs amorces d'oligonucléotide hors cible sont aptes à détecter plus de 100 événements d'édition génomique hors cible prédits.
